(19) Europäisches Patentamt
European Patent Office
Office européen des brevets

(11) Veröffentlichungsnummer: **0 656 357 A1**

# (12) EUROPÄISCHE PATENTANMELDUNG

(21) Anmeldenummer: **94116757.9**

(22) Anmeldetag: **24.10.94**

(51) Int. Cl.6: **C07D 401/12**, A01N 43/54, C07D 407/14, A01N 43/66, C07D 405/04, //(C07D401/12, 239:00,213:00),(C07D401/12, 215:00,213:00)

(30) Priorität: **02.11.93 DE 4337322**

(43) Veröffentlichungstag der Anmeldung: **07.06.95 Patentblatt 95/23**

(84) Benannte Vertragsstaaten: **BE CH DE FR GB IT LI NL**

(71) Anmelder: **BASF Aktiengesellschaft**
**Carl-Bosch-Strasse 38**
**D-67063 Ludwigshafen (DE)**

(72) Erfinder: **Rheinheimer, Joachim, Dr.**
**Merziger Strasse 24**
**D-67063 Ludwigshafen (DE)**
Erfinder: **Vogelbacher, Uwe Josef, Dr.**
**Niedererdstrasse 56**
**D-67071 Ludwigshafen (DE)**
Erfinder: **Baumann, Ernst, Dr.**
**Falkenstrasse 6a**
**D-67373 Dudenhofen (DE)**
Erfinder: **Gerber, Matthias, Dr.**
**Brandenburger Strasse 24**
**D-67117 Limburgerhof (DE)**
Erfinder: **Westphalen, Karl-Otto, Dr.**
**Mausbergweg 58**
**D-67346 Speyer (DE)**
Erfinder: **Walter, Helmut, Dr.**
**Grünstadter Strasse 82**
**D-67283 Obrigheim (DE)**

(54) **Pyridin-N-oxid substituierte Salicylaldehyd- bzw. Salicylsäurederivate, Verfahren zu ihrer Herstellung und ihre Verwendung als Herbizide.**

(57) Pyridin-N-oxid substituierte Salicylaldehyd- und Salicylsäurederivate der allgemeinen Formel I,

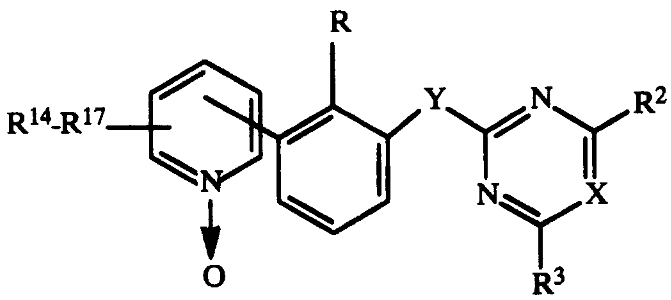

I

in der R eine Formylgruppe, eine Gruppe $CO_2H$ oder einen zu $CO_2H$ hydrolysierbaren Rest bedeutet und die übrigen Substituenten folgende Bedeutung haben:

- $R^2$ Halogen, Alkyl, Halogenalkyl, Alkoxy, Halogenalkoxy oder Alkylthio;
- X Stickstoff oder $CR^{13}$, wobei $R^{13}$ Wasserstoff oder Halogen bedeutet oder zusammen mit $R^3$ eine 3- bis 4-gliedrige Alkylen- oder Alkenylenkette bildet, in der jeweils eine Methylengruppe durch Sauerstoff ersetzt ist;
- $R^3$ Halogen, Alkyl, Halogenalkyl, Alkoxy, Halogenalkoxy, Alkylthio, oder $R^3$ ist mit $R^{13}$ wie oben

EP 0 656 357 A1

angegeben zu einem 5- oder 6-gliedrigen Ring verknüpft;

Y Sauerstoff oder Schwefel;

$R^{14}$-$R^{17}$

eine ggf. alkylsubstituierte Cycloalkylgruppe; eine ggf. substituierte Alkylgruppe; eine ggf. substituierte Alkoxy- oder Alkylthiogruppe; eine Dialkylamino- oder eine Dialkylaminoxygruppe; eine ggf. substituierte Alkenyl- oder Alkinylgruppe, Wasserstoff, Halogen, Nitro oder Cyano mit herbizider Wirkung.

Die vorliegende Erfindung betrifft Pyridin-N-oxid-substituierte Salicylaldehyd- und Salicylsäurederivate der allgemeinen Formel I,

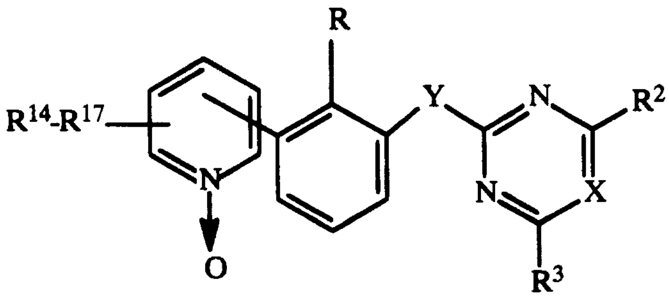

I

in der R eine Formylgruppe, eine Gruppe $CO_2H$ oder einen zu $CO_2H$ hydrolysierbaren Rest bedeutet und die übrigen Substituenten folgende Bedeutung haben:

$R^2$ Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;

X Stickstoff oder $CR^{13}$, wobei $R^{13}$ Wasserstoff oder Halogen bedeutet oder zusammen mit $R^3$ eine 3- bis 4-gliedrige Alkylen- oder Alkenylenkette bildet, in der jeweils eine Methylengruppe durch Sauerstoff ersetzt ist;

$R^3$ Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, oder $R^3$ ist mit $R^{13}$ wie oben angegeben zu einem 5- oder 6-gliedrigen Ring verknüpft;

Y Sauerstoff oder Schwefel;

$R^{14}$-$R^{17}$

a) eine $C_3$-$C_8$-Cycloalkylgruppe, welche ein bis drei $C_1$-$C_4$-Alkylreste tragen kann;

b) eine $C_1$-$C_8$-Alkylgruppe, eine $C_1$-$C_8$-Alkoxygruppe, eine $C_1$-$C_4$-Alkylthiogruppe, wobei die genannten Gruppen jeweils ein bis fünf Halogenatome und/oder einen der folgenden Reste tragen können:
$C_1$-$C_4$-Alkoxy, $C_1$-$C_4$-Halogenalkoxy, $C_1$-$C_4$-Alkylthio, Cyano, $C_3$-$C_8$-Cycloalkyl oder Di-$C_1$-$C_4$-Alkylamino;

c) eine Di-$C_1$-$C_4$-Alkylamino- oder eine Di-$C_1$-$C_4$-Alkylaminoxygruppe.

d) eine $C_2$-$C_6$-Alkenyl- oder eine $C_2$-$C_6$-Alkinylgruppe, welche ein bis fünf Halogenatome und/oder einen der folgenden Reste tragen kann:
$C_1$-$C_4$-Alkoxy, $C_1$-$C_4$-Halogenalkoxy oder $C_1$-$C_4$-Alkylthio;

e) Wasserstoff, Halogen, Nitro oder Cyano.

Gemäß dem Stand der Technik, z.B. WO 91/13065 und DE-A 39 19 435 weisen Salicylaldehyd- bzw. Salicylsäurederivate mit einem unsubstituierten Pyridylrest eine herbizide Wirkung auf. Die Wirkung der literaturbekannten Verbindungen ist im Hinblick auf herbizide Wirkung, die bioregulatorische Wirkung und/oder Selektivität nicht immer befriedigend, daher bestand die Aufgabe, pyridylsubstituierte Salicylaldehyd- bzw. Salicylsäurederivate mit verbesserter Wirkung zur Verfügung zu stellen.

Demgemäß wurden die eingangs definierten Pyridin-N-oxid substituierten Derivate I gefunden. Die neuen Verbindungen I zeigen eine ausgezeichnete herbizide Wirkung mit zum Teil verbesserter Selektivität gegenüber Kulturpflanzen.

Ferner wurden Verfahren zur Herstellung der Verbindungen I sowie ihre Verwendung als Herbizide und Wachstumsregulatoren gefunden.

Weiterhin sind Zwischenprodukte der Formel IV Gegenstand der Erfindung:

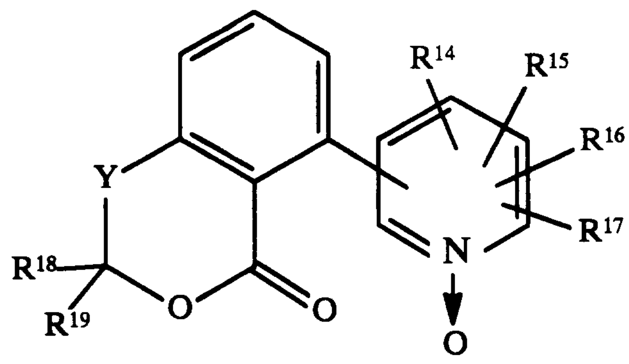

IV

in der die Reste $R^{14}$-$R^{17}$ die eingangs genannten Bedeutungen haben und Y, $R^{18}$ bzw. $R^{19}$ bedeuten:
Wasserstoff;
$C_1$-$C_4$-Alkyl, wobei dieser Rest jeweils ein bis fünf Halogenatome und/oder ein bis zwei $C_1$-$C_4$-Alkoxy-Gruppen tragen kann;
Phenyl, wobei dieser Rest jeweils ein bis fünf Halogenatome und/oder ein bis zwei der folgenden Gruppen tragen kann: $C_1$-$C_4$-Alkyl, $C_1$-$C_4$-Alkoxy, $C_1$-$C_4$-Halogenalkyl oder Nitro;
ferner die beiden Reste gemeinsam eine $C_2$-$C_6$-Alkylenkette, die durch ein bis fünf Halogenatome und/oder $C_1$-$C_4$-Alkylreste substituiert sein kann;
Y ein Sauerstoff- oder Schwefelatom.

Gegenstand der Erfindung sind auch Zwischenprodukte der Formel III

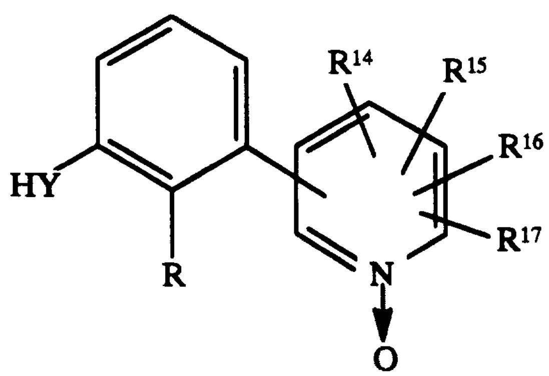

III

in der die Reste die vorstehend angegebenen Bedeutungen haben sowie solche Zwischenprodukte der Formel III, bei denen R für $COR^1$ steht und $R^1$ die vorstehend angegebenen Bedeutungen hat.

In der Beschreibung haben die unten genannten Substituenten bevorzugt folgende Bedeutung:
$C_1$-$C_4$-Alkyl: Methyl, Ethyl, 1-Propyl, 2-Propyl, 2-Methyl-2 propyl, 2-Methyl-1-propyl, 1-Butyl, 2-Butyl;
$C_1$-$C_8$-Alkyl: $C_1$-$C_4$-Alkyl sowie Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl;
$C_1$-$C_2$-Halogenalkyl: Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor 2-fluorethyl, 2,2,2-Trichlorethyl und Pentafluorethyl;
$C_1$-$C_2$-Halogenalkoxy: Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy;
$C_1$-$C_4$-Alkoxy: Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
$C_1$-$C_4$-Alkylthio: Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
$C_3$-$C_6$-Alkenyl: 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
$C_3$-$C_6$-Alkinyl: 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-Pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
Der Rest R ist breit variabel bezüglich der Reste, die sich zur Carboxylgruppe hydrolysieren lassen. Beispielsweise steht R für eine Gruppe

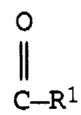

in der $R^1$ die folgende Bedeutung hat:

$R^1$

a) Wasserstoff;

b) eine Succinylimidoxygruppe;

c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder einen bis zwei der folgenden Reste tragen kann:
$C_1$-$C_4$-Alkyl; $C_1$-$C_4$-Halogenalkyl, insbesondere $C_1$-$C_2$-Halogenalkyl; $C_1$-$C_4$-Halogenalkoxy, insbesondere $C_1$-$C_2$-Halogenalkoxy; $C_1$-$C_4$-Alkoxy; $C_1$-$C_4$-Alkylthio;

d) $R^1$ ferner ein Rest -(O)m-$NR^6R^7$ in dem m für 0 oder 1 steht und $R^6$ und $R^7$, die gleich oder unterschiedlich sein können, die folgende Bedeutung haben:

Wasserstoff
$C_1$-$C_8$-Alkyl, insbesondere $C_1$-$C_4$-Alkyl;
$C_3$-$C_6$-Alkenyl, insbesondere 2-Propenyl, 2-Butenyl, 3-Methyl-2-butenyl und 3-Methyl-2-pentenyl;
$C_3$-$C_6$-Alkinyl, vorzugsweise 2-Propinyl, 2-Butinyl, 1-Methyl-2-propinyl und 1-Methyl-2-butinyl, insbesondere 2-Propinyl;
$C_3$-$C_8$-Cycloalkyl, wie Cyclopropyl, Cyclobutyl, Cyclopentyl, Cyclohexyl, Cycloheptyl und Cyclooctyl, wobei diese Alkyl-, Cycloalkyl-, Alkenyl- und Alkinylgruppen jeweils ein bis fünf, insbesondere ein bis drei Halogenatome, bevorzugt Fluor oder Chlor und/oder ein bis zwei der folgenden Gruppen tragen können:
$C_1$-$C_4$-Alkyl, $C_1$-$C_4$-Alkoxy, $C_1$-$C_4$-Alkylthio, $C_1$-$C_4$-Halogenalkoxy, $C_3$-$C_6$-Alkenyloxy, $C_3$-$C_6$-Alkenylthio, $C_3$-$C_6$-Alkinyloxy, $C_3$-$C_6$-Alkinylthio, wobei die in diesen Resten vorliegenden Alkenyl- und Alkinylbestandteile vorzugsweise den oben genannten Bedeutungen entsprechen;
$C_1$-$C_4$-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
$C_1$-$C_4$-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
$C_3$-$C_6$-Alkenylcarbonyl, $C_3$-$C_6$-Alkinylcarbonyl, $C_3$-$C_6$-Alkenyloxycarbonyl und $C_3$-$C_6$-Alkinyloxycarbonyl, wobei die Alkenyl- bzw. Alkinylreste vorzugsweise wie vorstehend definiert sind;
Phenyl, gegebenenfalls ein oder mehrfach substituiert durch Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
Di-($C_1$-$C_4$-alkyl)amino wie insbesondere Dimethylamino, Diethylamino, Dipropylamino, N-Propyl-N-methylamino, N-Propyl-N-ethylamino, Diisopropylamino, N-Isopropyl-N-methylamino, N-Isopropyl-N-ethylamino, N-

Isopropyl-N-propylamino;
$R^6$ und $R^7$ ferner Phenyl, das durch einen oder mehrere der folgenden Reste substituiert sein kann: Halogen, Nitro, Cyano, $C_1$-$C_4$-Alkyl, $C_1$-$C_4$-Halogenalkyl, insbesondere $C_1$-$C_2$-Halogenalkyl, $C_1$-$C_4$-Alkoxy, $C_1$-$C_4$-Halogenalkoxy, insbesondere $C_1$-$C_2$-Halogenalkoxy oder $C_1$-$C_4$-Alkylthio;
oder $R^6$ und $R^7$ bilden gemeinsam eine zu einem Ring geschlossene, gegebenenfalls substituierte $C_4$-$C_7$-Alkylenkette, die ein Heteroatum, ausgewählt aus der Gruppe Sauerstoff, Schwefel oder Stickstoff, enthalten kann wie $-(CH_2)_4-$, $-(CH_2)_5-$, $-(CH_2)_6-$, $-(CH_2)_7-$,$-(CH_2)_2-O-(CH_2)_2-$, $-CH_2-S-(CH_2)_3-$, $-(CH_2)_2-O-(CH_3)_3-$, $-NH-(CH_2)_3-$, $-CH_2-NH-(CH_2)_2-$, $-CH_2-CH=CH-CH_2-$, $-CH=CH-(CH_2)_3-$, wobei als Substituenten insbesondere $C_1$-$C_4$-Alkylreste in Betracht kommen;

e) $R^1$ ferner eine Gruppe

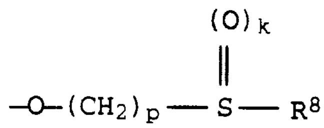

in der k die Werte 0, 1 und 2, p die Werte 1, 2, 3 und 4 annehmen und $R^8$ für $C_1$-$C_4$-Alkyl, $C_1$-$C_4$-Halogenalkyl, $C_3$-$C_6$-Alkenyl, $C_3$-$C_6$-Alkinyl oder gegebenenfalls substituiertes Phenyl steht, wie insbesondere für $R^6$ und $R^7$ genannt;

f) $R^1$ ferner ein Rest $OR^9$, worin $R^9$ bedeutet:

i) Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion wie tert.-$C_1$-$C_4$-Alkylammonium oder Ammonium [$NH_4^+$];

ii) $C_3$-$C_8$-Cycloalkyl wie vorstehend genannt, welches ein bis drei $C_1$-$C_4$-Alkylgruppen tragen kann, insbesondere Cyclopropyl, Cyclopentyl, Cyclohexyl, Metylcyclohexyl;

iii) $C_1$-$C_8$-Alkyl, welches ein bis fünf Halogenatome, insbesondere Fluor und Chlor und/oder einen der folgenden Reste tragen kann:
$C_1$-$C_4$-Alkoxy, $C_1$-$C_4$-Alkylthio, Cyano, $C_1$-$C_4$-Alkylcarbonyl, $C_3$-$C_8$-Cycloalkyl, $C_1$-$C_4$-Alkoxycarbonyl, Phenyl, ein- oder mehrfach durch Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiertes Phenyl oder Phenoxy, wie insbesondere oben genannt;

iv) eine $C_1$-$C_8$-Alkylgruppe, welche ein bis fünf, vorzugsweise ein bis drei Halogenatome, insbesondere Fluor und/oder Chlor tragen kann und einen der folgenden Reste trägt: ein 5-gliedriger Heteroaromat, enthaltend ein bis drei Stickstoffatome oder ein 5-gliedriger Heteroaromat, enthaltend ein Stickstoffatom und ein Sauerstoff- oder Schwefelatom wie Pyrazolyl, Imidazolyl, Benzimidazolyl, Triazolyl, Benztriazolyl, Isooxazolyl, Oxazolyl, Thiazolyl, gebunden über ein C-Atom oder falls möglich N-Atom, wobei der Heteroaromat ein bis vier Halogenatome und/oder ein bis zwei der folgenden Reste tragen kann:
Nitro, Cyano, $C_1$-$C_4$-Alkyl, $C_1$-$C_4$-Halogenalkyl, $C_1$-$C_4$-Alkoxy, Phenyl, $C_1$-$C_4$-Halogenalkoxy und/oder $C_1$-$C_4$-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;

v) eine $C_2$-$C_6$-Alkylgrupe, welche in der 2-Position einen der folgenden Reste trägt: $C_1$-$C_4$-Alkoxyimino, $C_3$-$C_6$-Alkinyloxyimino, $C_3$-$C_6$-Halogenalkenyloxyimino, $C_3$-$C_6$-Alkenyloxyimino oder Benzyloxyimino;

vi) eine $C_3$-$C_6$-Alkenyl- oder eine $C_3$-$C_6$-Alkinylgruppe, wobei diese Gruppen ihrerseits ein bis fünf Halogenatome tragen können;

vii) $R^9$ ferner ein Phenylrest, welcher ein bis fünf Halogenatome und/oder ein bis drei der folgenden Reste tragen kann: Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, wie insbesondere oben genannt;

viii) ein über ein Stickstoffatom verknüpfter 5-gliedriger Heteroaromat, enthaltend ein bis drei Stickstoffatome wie Pyrazolyl, Imidazolyl, Benzimidazolyl, Triazolyl, Benztriazolyl, vorzugsweise gebunden über die 1-Position, wobei der Heteroaromat ein bis zwei Halogenatome und/oder ein bis zwei der folgenden Reste tragen kann:
$C_1$-$C_4$-Alkyl, $C_1$-$C_4$-Halogenalkyl, $C_1$-$C_4$-Alkoxy, Phenyl, $C_1$-$C_4$-Halogenalkoxy und/oder $C_1$-$C_4$-Alkylt-

hio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;

ix) $R^9$ ferner ein Gruppe $-N=CR^{10}R^{11}$, worin $R^{10}$ und $R^{11}$, die gleich oder verschieden sein können, bedeuten:

$C_1$-$C_{12}$-Alkyl, insbesondere $C_1$-$C_8$-Alkyl, $C_3$-$C_6$-Alkenyl, $C_3$-$C_6$-Alkinyl, $C_3$-$C_8$-Cycloalkyl, wobei diese Reste einen $C_1$-$C_4$-Alkoxy, $C_1$-$C_4$-Alkylthio und/oder einen gegebenenfalls substituierten Phenylrest, wie insbesondere vorstehend genannt, tragen können;

Phenyl, das durch einen oder mehrere der folgenden Reste substituiert sein kann: Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, wobei diese Reste insbesondere den oben für $R^1$ genannten entsprechen;

oder $R^{10}$ und $R^{11}$ bilden gemeinsam eine $C_3$-$C_{12}$ Alkylenkette, welche ein bis drei $C_1$-$C_4$-Alkylgruppen tragen und ein Heteroatom aus der Gruppe Sauerstoff, Schwefel und Stickstoff enthalten kann, wie insbesondere bei $R^6$ und $R^7$ genannt;

g) $R^1$ ferner ein Rest $-NH-SO_2-R^{12}$ worin $R^{12}$ bedeutet:

$C_1$-$C_4$-Alkyl, $C_3$-$C_6$-Alkenyl, $C_3$-$C_6$-Alkinyl, $C_3$-$C_8$-Cycloalkyl wie insbesondere vorstehend für $R^1$ genannt, wobei diese Reste einen $C_1$-$C_4$-Alkoxy-, $C_1$-$C_4$-Alkylthio- und/oder einen Phenylrest wie oben genannt tragen können;

Phenyl, gegebenenfalls substituiert, insbesondere wie vorstehend genannt.

Besonders bevorzugt steht $R^1$ für eine Gruppe $OR^9$.

Im Hinblick auf die biologische Wirkung sind Wirkstoffe der Formel I bevorzugt, in der die übrigen Substituenten die folgende Bedeutung haben:

$R^2$ die bei $R^1$ im einzelnen genannten $C_1$-$C_4$-Alkyl-, $C_1$-$C_4$-Halogenalkyl-, $C_1$-$C_4$-Alkoxy-, $C_1$-$C_4$-Halogenalkoxy-, $C_1$-$C_4$-Alkylthiogruppen und Halogenatome bedeutet, insbesondere Chlor, Methyl, Methoxy, Ethoxy, Difluormethoxy, Trifluormethoxy, besonders bevorzugt Methoxy;

X Stickstoff oder $CR^{13}$, worin

$R^{13}$ bevorzugt Wasserstoff oder Halogen wie Fluor oder Chlor, oder zusammen mit $R^3$ eine 4-bis 5-gliedrige Alkylen- oder Alkenylenkette, in der jeweils eine Methylengruppe durch Sauerstoff ersetzt ist, wie $-CH_2-CH_2-O-$, $-CH=CH-O-$, $-CH_2-CH_2-CH_2-O-$, $-CH=CH-CH_2O-$, insbesondere Wasserstoff, Fluor und $-CH_2-CH_2-O-$;

$R^3$ $C_1$-$C_4$-Alkyl, $C_1$-$C_4$-Halogenalkyl, insbesondere $C_1$-$C_2$-Halogenalkyl, $C_1$-$C_4$-Alkoxy, $C_1$-$C_4$-Halogenalkoxy, insbesondere $C_1$-$C_2$-Halogenalkoxy, $C_1$-$C_4$-Alkylthiogruppen, insbesondere Chlor, Methyl, Methoxy, Ethoxy, Difluormethoxy, Trifluormethoxy, besonders bevorzugt Methoxy, oder mit $R^{13}$ wie oben genannt zu einem 5-oder 6-gliedrigen Ring verknüpft ist;

$R^{14}$-$R^{17}$

a) eine $C_3$-$C_8$-Cycloalkylgruppe, welche ein bis drei $C_1$-$C_4$-Alkylreste tragen kann, insbesondere Cyclopropyl, Cyclopentyl, Cyclohexyl, Methylcyclohexyl;

$b_1$) eine $C_1$-$C_8$-Alkylgruppe, welche ein bis fünf Halogenatome und/oder einen der folgenden Reste tragen kann:

$C_1$-$C_4$-Alkoxy, $C_1$-$C_4$-Halogenalkoxy, besonders $C_1$-$C_2$-Halogenalkoxy, $C_1$-$C_4$-Alkylthio, Cyano, Halogen, z.B. Fluor oder Chlor, $C_3$-$C_8$-Cycloalkyl oder Di-$C_1$-$C_4$-Alkylamino;

besonders bevorzugt sind 2-Methoxyethyl, 2-Ethoxyethyl, 2-Dimethylaminoethyl, 2-Diethylaminoethyl, Methoxymethyl, Ethoxymethyl, Dimethylaminomethyl, Diethylaminomethyl, Ethyl, Isopropyl, n-Propyl und Trifluormethyl, Methyl;

$b_2$) eine $C_1$-$C_8$-Alkoxygruppe, welche ein bis fünf Halogenatome und/oder einen der folgenden Reste tragen kann:

$C_1$-$C_4$-Alkoxy, $C_1$-$C_4$-Halogenalkoxy, besonders $C_1$-$C_2$-Halogenalkoxy, $C_1$-$C_4$-Alkylthio, Cyano, Halogen, z.B. Fluor oder Chlor, $C_3$-$C_8$-Cycloalkyl oder Di-$C_1$-$C_4$-Alkylamino;

besonders bevorzugt sind 2-Methoxyethoxy, 2-Ethoxyethoxy, 2-Dimethylaminoethoxy, 2-Diethylaminoethoxy, Methoxymethoxy, Ethoxymethoxy, Dimethylaminomethoxy, Diethylaminomethoxy, 2-Methylthioethoxy, 2,2,2-Trifluorethoxy, Methoxy, Ethoxy, Propoxy, 2-Propoxy;

$b_3$) eine $C_1$-$C_4$-Alkylthiogruppe, welche ein bis fünf Halogenatome und/oder einen der folgenden Reste tragen kann: $C_1$-$C_4$-Alkoxy, $C_1$-$C_4$-Halogenalkoxy, $C_1$-$C_4$-Alkylthio, Cyano, $C_3$-$C_8$-Cycloalkyl oder Di-$C_1$-$C_4$-Alkylamino, besonders bevorzugt Methylthio, Ethylthio, Propylthio, 2-Propylthio, Methoxymethylthio;

c) eine Di-($C_1$-$C_4$-Alkyl)amino- oder eine Di-($C_1$-$C_4$-Alkyl)aminoxygruppe, besonders bevorzugt Dimethylamino, Diethylamino, N-Methyl-N-Ethylamino, Diisopropylamino, Dipropylamino, Dimethylaminoxy, Diethylaminoxy;

d) eine $C_2$-$C_6$-Alkenyl- oder eine $C_2$-$C_6$-Alkinylgruppe, welche ein bis fünf Halogenatome und/oder einen der folgenden Reste tragen kann:

$C_1$-$C_4$-Alkoxy, $C_1$-$C_4$-Halogenalkoxy oder $C_1$-$C_4$-Alkylthio, besonders bevorzugt Vinyl, 2-Propenyl, Ethinyl, 1-Propinyl, 3-Propinyl, 3-Methoxy-1-propinyl, Chlorallyl;

e) Wasserstoff, Halogen wie Fluor, Chlor, Brom oder Iod, Nitro; besonders bevorzugt Wasserstoff und Halogen wie insbesondere Fluor oder Chlor.

Besonders bevorzugt sind Pyridin-N-oxid substituierte Salicylaldehyd- und Salicylsäurederivate der Formel I gemäß Anspruch 1, in der mindestens zwei der Reste $R^{14}$ bis $R^{17}$ Wasserstoff bedeuten.

Ferner sind bevorzugt Verbindungen der Formel I, in der $R^3$ für Methoxy und X für CH oder CF stehen oder in der $R^3$ zusammen mit X eine $OCH_2CH_2$-Kette bilden.

Im Hinblick auf die bestimmungsgemäße Verwendung der Endprodukte I' sind Zwischenprodukte der Formel IV bevorzugt, bei denen $R^{14}$-$R^{17}$ und Y die vorstehend bei den Wirkstoffen als bevorzugt angegebenen Bedeutungen haben und $R^{18}$ und $R^{19}$ bedeuten:

Wasserstoff;

$C_1$-$C_4$-Alkyl, wobei dieser Rest jeweils ein bis fünf Halogenatome und/oder ein bis zwei $C_1$-$C_4$-Alkoxy-Gruppen tragen kann, besonders Methyl und Ethyl;

Phenyl, wobei dieser Rest jeweils ein bis fünf Halogenatome und/oder ein bis zwei der folgenden Gruppen tragen kann: $C_1$-$C_4$-Alkyl, $C_1$-$C_4$-Alkoxy, $C_1$-$C_4$-Halogenalkyl oder Nitro, besonders Phenyl;

ferner können die beiden Reste gemeinsam eine $C_2$-$C_6$-Alkylenkette sein, die durch ein bis fünf Halogenatome und/oder $C_1$-$C_4$-Alkylreste substituiert sein kann, besonders Butylen oder Pentylen.

Bei den Zwischenprodukten der Formel III sind diejenigen bevorzugt, bei denen R bzw. $R^1$, $R^{14}$-$R^{17}$ und Y die bei den Wirkstoffen bevorzugten Bedeutungen haben.

Die Zwischenprodukte der Formel IV erhält man beispielsweise auf dem im folgenden Reaktionsschema dargestellten Weg:

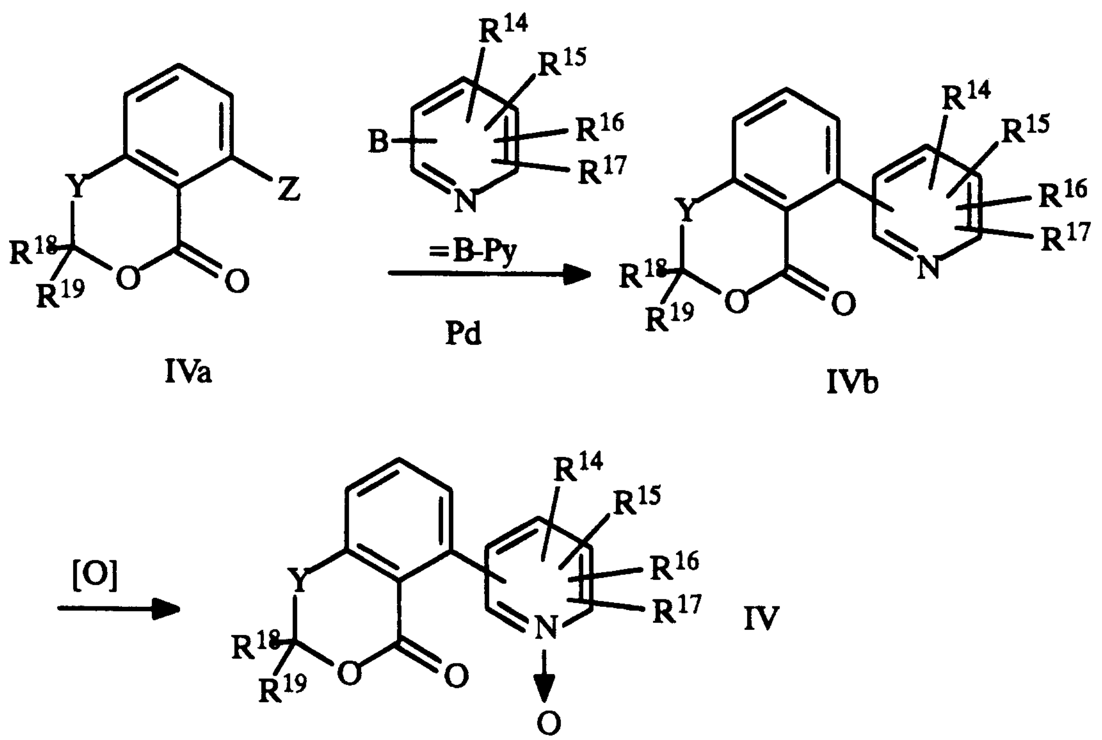

IVa
IVb
IV

Dabei stehen

B für Trialkylstannyl wie Trimethylstannyl, Triethylstannyl, Tributylstannyl, Tripentylstannyl oder Trihexylstannyl, Dihydroxyboranyl, Dialkoxyboranyl wie Dimethoxyboranyl, Diethoxyboranyl, Dipropoxyboranyl, Diisopropoxyboranyl oder Dibutoxyboranyl oder Isomere oder Alkylendioxyboranyl wie Ethylendioxyboranyl oder 1,3-Prpylendioxyboranyl;

Z für Chlor, Brom, Iod, $C_1$-$C_4$-Halogenalkylsulfonyloxy, $C_1$-$C_4$-Alkylsulfonyloxy wie Trifluormethylsulfonyloxy oder Fluorsulfonyloxy
und die übrigen Raste für die vorstehend beschriebenen Gruppen.

Die Herstellung der cyclischen Acetale IVa kann ausgehend von den zugrunde liegenden 6-Hydroxybenzoesäuren (für Y = O) bzw. 6-Mercaptobenzoesäuren(für Y = S) durch Umsetzung mit einer Verbindung $O = CR^{18}R^{19}$ in Gegenwart eines sauren Katalysators erfolgen. Man kann zur Bindung des bei der Reaktion entstehenden Wassers ein wasserentziehendes Mittel zusetzen wie z. B. Molekularsieb, Säureanhydride wie z. B. Trifluoracetanhydrid oder Acetanhydrid, Natriumsulfat, Calciumchlorid, wenn das den Umsatz fördert. Geeignet ist auch ein reaktionsfähiges Acetal der Verbindung $O = CR^{18}R^{19}$ wie z. B. ein Dimethylacetal, Diethylacetal, Ethylenacetal oder Propylenacetal. Als Katalysatoren eignen sich besonders saure Ionenaustauscher, Protonensäuren wie z. B. Toluolsulfonsäure, Schwefelsäure, Phosphorsäure u. s. w. oder Lewis-Säuren wie z. B. Aluminiumchlorid, Titantetrachlorid, Zinkchlorid, Calciumchlorid, Magnesiumchlorid, Zinnchloride u. s. w.. Oftmals lassen sich das gebildete Wasser oder der aus dem Acetal gebildete Alkohol direkt aus der Reaktionsmischung abdestillieren.

Wenn Z für $C_1$-$C_4$-Halogenalkylsulfonyloxy, $C_1$-$C_4$-Alkylsulfonyloxy oder Fluorsulfonyloxy steht, so kann man auch zuerst die Hydroxyverbindung (Z = OH) wie vorstehend beschrieben herstellen und diese dann beispielsweise durch Umsetzung mit Sulfonsäureanhydriden oder -Halogeniden in die gewünschten Vorstufen IVa überführen (s. Herstellbeispiele 1a, 1b).

Die Vorstufen IVb erhält man besonders vorteilhaft durch Umsetzung von Verbindungen IVa mit Verbindungen B-Py in Gegenwart einer katalytisch wirksamen Palladiumverbindung. Die verwendeten Bor- oder Zinnverbindungen sind entweder bekannt oder analog zu bekannten Verbindungen herstellbar.

Bei diesem Verfahren wird eine katalytisch wirksame Palladiumverbindung eingesetzt. Dabei sind beliebige Palladiumsalze oder - Komplexe geeignet, die in der Reaktionsmischung zumindest teilweise löslich sind. Die Oxidationsstufe des Palladiums kann 0 oder 2 betragen. Bei den Palladiumsalzen kommen u. a. folgende Gegenionen in Betracht: Chlorid, Bromid, Iodid, Sulfat, Acetat, Trifluoracetat, Acetylacetonat oder Hexafluoro-2,4-pentadionat. Es können viele verschiedene Palladiumkomplexe verwendet werden. Voraussetzung ist lediglich, daß die Liganden am Palladium unter den Reaktionsbedingungen vom Substrat verdrängt werden können. Besonders geeignet sind Phosphinliganden wie z. B. Aryl-Alkylphosphine wie u. a. Methyldiphenylphosphin, Isopropyldiphenylphosphin, Triarylphosphine wie u. a. Triphenylphosphin, Tritolylphosphin, Trixylylphosphin, Trihetarylphosphine wie Trifurylphosphin oder dimere Phosphine. Gut geeignet sind auch olefinische Liganden wie u. a. Dibenzylidenaceton oder seine Salze, Cycloocta-1,5-dien oder Amine wie Trialkylamine (z. B. Triethylamin, Tetramethylethylendiamin, N-Methylmorpholin) oder Pyridin.

Man kann den verwendeten Komplex direkt bei der Reaktion einsetzen. So kann man z. B. mit Tetrakistriphenylphosphinpalladium(0), Bistriphenylphosphinpalladiumdichlorid, Bistriphenylphosphinpalladiumdiacetat, einem Dibenzylidenaceton-Palladium(0)-Komplex, Tetrakismethyldiphenylphosphinpalladium(0) oder Bis(1,2-diphenylphosphinoethan)palladiumdichlorid verfahren. Man kann auch ein Palladiumsalz und zusätzlich einen geeigneten Liganden verwenden, die dann erst in situ den katalytisch aktiven Komplex bilden. Diese Vorgehensweise bietet sich z. B. bei den oben genannten Salzen und Phosphinliganden wie z. B. Trifurylphosphin oder Tritolylphosphin an. Auch können Palladiumkomplexe wie z. B. Tris(dibenzylidenaceton)dipalladium, Bis(dibenzylidenaceton)palladium oder 1,5-Cyclooctadienpalladiumdichlorid durch die Zugabe von Liganden wie z. B. Trifurylphosphin oder Tritolylphosphin weiter aktiviert werden.

Üblicherweise werden 0,001 bis 10 mol-%, insbesondere 0.005 bis 5 mol-% der Palladiumverbindung (Salz oder Komplex), bezogen auf die Verbindung B-Py verwendet. Höhere Mengen sind möglich aber eher unwirtschaftlich.

Die Menge von B-Py, bezogen auf den Ausgangsstoff IVa liegt im allgemeinen bei 0,8 bis 3, bevorzugt bei 0,95 bis 1,5 Moläquivalenten.

Für die Reaktion sind alle Lösungsmittel geeignet, die nicht selbst mit den verwendeten Substraten reagieren. Polare Lösungsmittel beschleunigen die Reaktion. Besonders geeignet sind Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Amine wie Triethylamin. Vorteilhaft ist oftmals die Verwendung von Mischungen z. B. von Ethern mit Amiden. Auch Alkylalkohole und Wasser können als Mischungspartner in Frage kommen, besonders, wenn der Rest B ein Boratom enthält. Die Zugabe von Tetraalkylammoniumhalogeniden oder Alkalimetallhalogeniden wie z. B. Lithiumchlorid ist oft hilfreich und insbesondere anzuraten, wenn Z für einen Sulfonyloxyrest steht. Besonders wenn der Rest B ein Boratom enthält, ist es oft nützlich, eine organische oder anorganische Base wie Kaliumcarbonat, Natriumcarbonat, Calciumcarbonat, Calciumhydroxid, Natriumhydroxid, Kaliumhydroxid, Kaliumphosphat, Natriumphosphat, Pyridin oder ein Amin wie Triethylamin zuzusetzen.

Die Reaktionstemperatur liegt zwischen -20 und 200 °C, bevorzugterweise zwischen 50 und 160 °C. Die Reaktionszeiten betragen üblicherweise zwischen einigen Minuten und 50 Stunden, meist 0,5-10 Stunden. Bei der Verwendung niedrig siedender Lösungsmittel ist es manchmal nützlich, die Umsetzung unter Eigendruck im Autoklaven durchzuführen.

Gegebenenfalls können die so erhaltenen Produkte der Formel IVb durch weitere Reaktionen in andere Zwischenprodukte der Formel IVb überführt werden. So kann es erforderlich sein, Schutzgruppen abzuspalten.

Für die Oxidation von IVb zu IV kommen die verschiedensten Peroxide wie Wasserstoffperoxid, tert.-Butylperoxid, organische oder anorganische Peroxide oder deren Salze wie 3-Chlorperbenzoesäure, Peressigsäure, Perameisensäure, Dibenzoylperoxid usw. in Frage. Man kann das Peroxid auch in situ aus einem anderen Peroxid, beispielsweise Peressigsäure aus Essigsäure und Wasserstoffperoxid herstellen.

Das Peroxid wird vorzugsweise in gleichen Äquivalenten wie das Ausgangsmaterial IVb eingesetzt. Man kann auch einen Überschuß verwenden, um die Reaktion zu vervollständigen. Geeignet sind alle Lösungsmittel, die selbst nicht mit den Oxidationsmitteln störende Nebenreaktionen eingehen, wie z. B. Wasser, Alkylalkohole, Carbonsäuren wie Essigsäure oder Ameisensäure oder halogenierte Kohlenwasserstoffe wie u. a. Methylenchlorid. Die Reaktionstemperaturen liegen zwischen -30 °C und 130 °C vorzugsweise zwischen -10 °C und 80 °C.

Aromatische Carbonsäurederivate der Formel I erhält man beispielsweise, indem man ein entsprechendes aromatisches Carbonsäurederivat der Formel III,

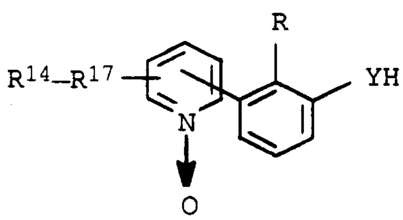

III

das nach den weiter unten angeführten Beispielen zugänglich ist, mit einer entsprechenden Verbindung der Formel II

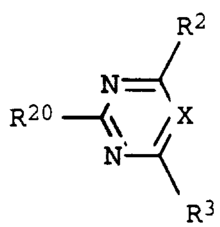

II

in Gegenwart einer Base umsetzt.

$R^{20}$ bedeutet Chlor, Brom, Iod, Aryl- oder Alkylsulfonyl wie z. B. Toluolsulfonyl oder Methylsulfonyl oder eine andere äquivalente Abgangsgruppe. Verbindungen der Formel II mit einem reaktionsfähigen Substituenten $R^{20}$ sind in den meisten Fällen bekannt oder mit dem allgemeinen Fachwissen leicht zu erhalten. Als Base können Alkali- oder Erdalkalimetallhydride wie NaH oder $CaH_2$, Alkalimetallhydroxyde wie NaOH oder KOH, Alkalimetallalkoholate wie Kalium-tert.-butylat, Alkalimetallcarbonate wie $Na_2CO_3$ oder $K_2CO_3$, Alkalimetallamide wie $NaNH_2$ oder Lithiumdiisopropylamid oder tertiäre Amine wie Triethylamin oder Pyridin Verwendung finden. Bei Einsatz einer anorganischen Base kann man einen Phasentransferkatalysator zusetzen, wenn dies den Umsatz fördert. Dafür kommen beispielsweise Kronenether oder organische Ammoniumverbindungen in Frage.

Es können viele der üblichen Lösungsmittel verwendet werden wie zum Beispiel Dimethylsulfoxid, Dimethylformamid, Tetrahydrofuran, Methyl-t.-butylether, Diethylether, Aceton, Methylethylketon, Toluol, Benzol, Dimethoxyethan, Dioxan, Pyridin oder t.-Butanol. Die Reaktionstemperaturen liegen im allgemeinen zwischen -80 und 150 °C, vorzugsweise zwischen -10 und 130 °C.

Soweit es sich bei den in der beschriebenen Weise hergestellten Verbindungen der Formel I um Carbonsäuren handelt (wenn also $R^1$ Hydroxyl bedeutet), können hieraus die im Anspruch 2 aufgeführten Carbonsäurederivate z. B. auch dadurch hergestellt werden, daß man die Carbonsäure zunächst auf übliche Weise in eine aktivierte Form wie ein Halogenid oder Imidazolid überführt und dieses dann mit der

entsprechenden Hydroxylverbindung der Formel $R^1$-H umsetzt. Diese beiden Schritte lassen sich beispielsweise auch dadurch vereinfachen, daß man die Carbonsäure in Gegenwart eines wasserabspaltenden Mittels wie z. B. eines Carbodiimids oder eines geeigneten Phosphonsäureanhydrids auf die Hydroxylverbindung einwirken läßt.

Ein anderes Verfahren zur Herstellung von Pflanzenschutzwirkstoffen der Formel I' geht von Zwischenprodukten der Formel IV aus, die mit einem Salz $R^1$-M und einem Heterocyclus V umgesetzt werden:

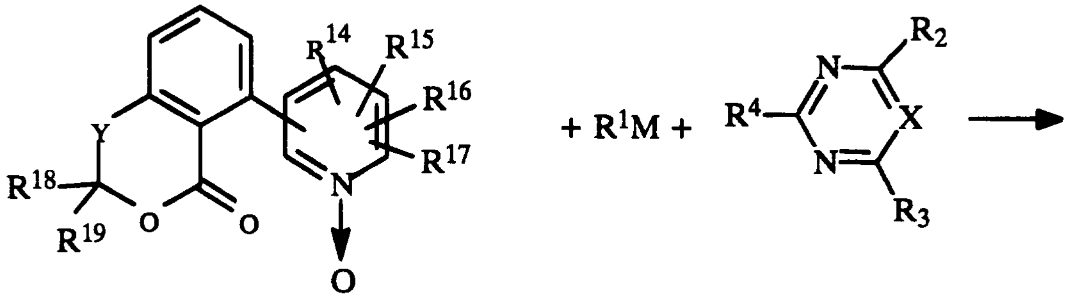

IV V

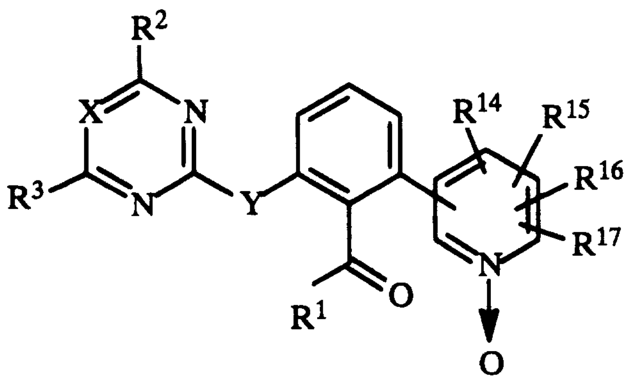

I'

Dabei haben $R^2$, $R^3$, $R^{14}$-$R^{17}$, Y, $R^{18}$ und $R^{19}$ die oben beschriebenen Bedeutungen und die übrigen Reste bedeuten:

M ein Alkalimetallkation wie Lithium, Natrium, Kalium oder ein Äquivalent eines Erdalkalimetallkations wie Magnesium, Calcium, Barium;

$R^4$ Halogen wie Fluor, Chlor, Brom, Iod, Alkylsulfonyl, besonders Methylsulfonyl oder Halogenalkylsulfonyl, besonders Trifluormethylsulfonyl;

$R^1$ Reste wie vorstehend beschrieben mit Ausnahme von Wasserstoff, der Succinylimidoxygruppe sowie denjenigen Resten $OR^9$, bei denen $R^9$ für Wasserstoff, ein Alkalimetallkation, das Äquivalent eines Erdalkalimetallkations, das Ammoniumkation oder ein organisches Ammoniumion steht.

Die Umsetzung der Zwischenprodukte der Formel IV mit dem Salz $R^1M$ und die weitere Reaktion mit dem Heterocyclus der Formel V kann in einem Reaktionsgefäß direkt bis zum Wirkstoff I' durchgeführt werden. In diesem Fall gibt man zuerst das Salz $R^1M$ zu, das man auch in situ aus einer Verbindung $R^1H$ und einer Base nach altbekannten Verfahren herstellen kann. Anschließend fügt man dann den Heterocyclus V hinzu. Die Zugabe von V sollte vorteilhaft erst erfolgen, wenn der erste Schritt der Reaktion, die Addition des Salzes $R^1M$ an das Zwischenprodukt IV, weitgehend abgeschlossen ist. Das kann zwischen wenigen Minuten und etlichen Stunden dauern, wobei die Reaktionstemperatur zwischen -40 °C und 200 °C, meist zwischen 0 °C und 130 °C liegt.

Es ist auch möglich, die Reaktion nach der ersten Stufe abzubrechen und das Zwischenprodukt III,

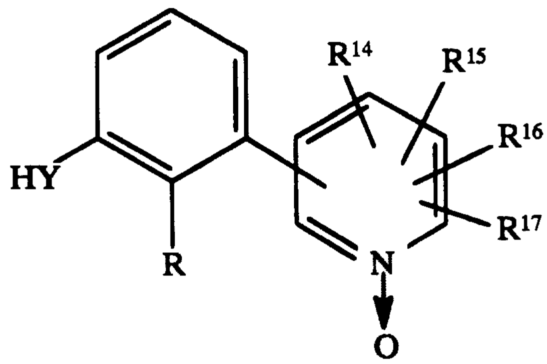

III

bei dem R für -$COR^1$ steht, zu isolieren. III läßt sich dann nach dem weiter oben beschriebenen Verfahren weiter umsetzen.

In beiden Fällen sind die üblichen Lösungsmittel geeignet, vorausgesetzt, sie können von der verwendeten Base oder dem Salz $R^1M$ nicht selbst deprotoniert werden und an der Reaktion teilnehmen. Besonders gut geeignet sind polare Lösungsmittel, z. B. Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Dimethylsulfoxid. Man kann einen Phasentransfer-Katalysator wie einen Kronenether oder ein quartäres Ammoniumsalz zusetzen, wenn das den Umsatz fördert.

Die Reaktionstemperatur liegt zwischen -40 und 200 °C, bevorzugterweise zwischen 0 und 160 °C. Die Reaktionszeiten betragen üblicherweise zwischen einigen Minuten und 50 Stunden, meist 0,5-10 Stunden.

Pro Mol Ausgangsstoff IV werden im allgemeinen 0,8 bis 3, insbesondere 0,9 bis 1,5 Moläquivalente der Verbindung $R^1M$ eingesetzt. Die Menge an Heterocyclus V liegt ebenfalls zweckmäßigerweise bei 0,8 bis 3, insbesondere 0,9 bis 1,5 Moläquivalenten bezogen auf IV.

Die Umsetzung kann kontinuierlich oder diskontinuierlich; bei Atmosphärendruck, Über- oder Unterdruck vorgenommen werden.

Die Aufarbeitung erfolgt in an sich bekannter Weise. Die Möglichkeiten sind vielfältig und hängen im Einzelfall von der Löslichkeit des Produktes in den verwendeten Lösungsmitteln sowie von der Mischbarkeit der Lösungsmittel mit Wasser und den Siedepunkten der Lösungsmittel ab. Sowohl wäßrige als auch nichtwäßrige Aufarbeitungen sind möglich. Eine geeignete Aufarbeitungsmethode besteht darin, die Reaktionsmischung, aus der man vorher das Lösungsmittel auch teilweise oder ganz verdampfen kann, mit Wasser zu vermischen und das Produkt abzufiltrieren oder einem organischen Lösungsmittel zu extrahieren.

Man kann nach diesem Verfahren auch die Zwischenstufen IVb zunächst wie vorstehend beschrieben zu den Verbindungen VI umsetzen, die teilweise bekannt sind (DE-A-39 19 435) und diese dann in die Endprodukte überführen wie in folgendem Schema illustriert:

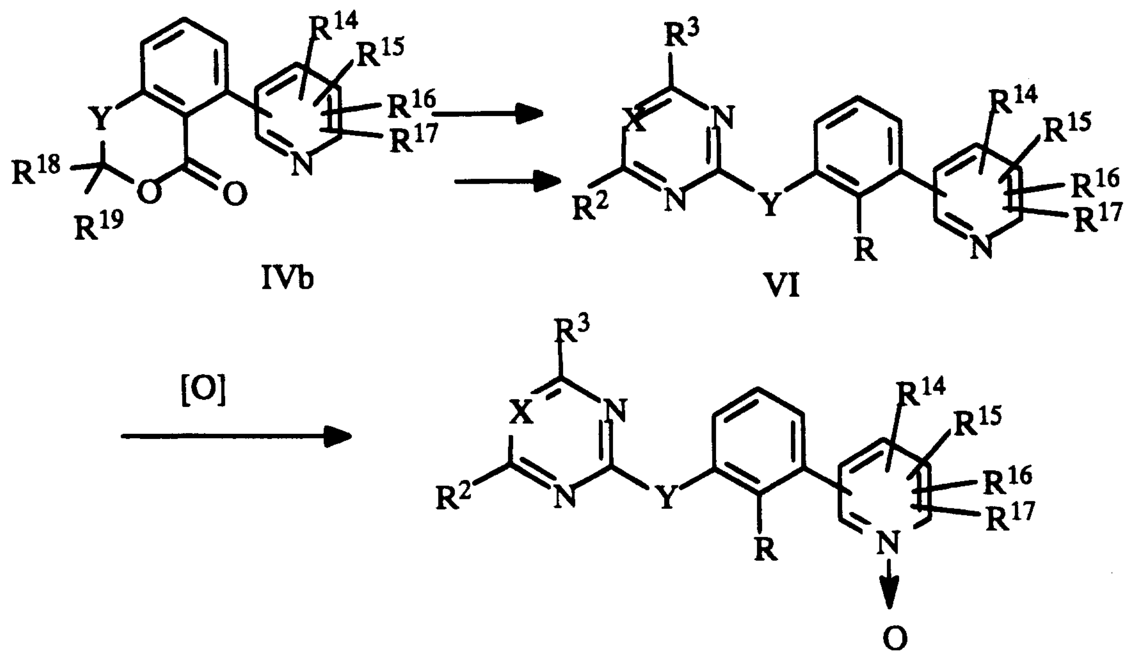


IVb

VI

Dabei steht R vorzugsweise für -$COR^1$, wobei $R^1$ nicht für Wasserstoff, die Succinylimidoxygruppe sowie diejenigen Reste $OR^9$ steht, bei denen $R^9$ Wasserstoff oder eines der genannten Kationen bedeutet.

Die Reaktionsbedingungen bei der Oxidation entsprechen den zuvor bei der Oxidation von IVb zu IV beschriebenen.

Weiterhin lassen sich Carbonsäuren der Formel I (wobei also $R^1$ Hydroxyl bedeutet) auch wie folgt zu vielen der beschriebenen Ester umsetzen: Man überführt die Carbonsäure dazu zunächst in ein Salz - besonders ein Alkalimetallsalz - und bringt dieses dann - gegebenenfalls in Gegenwart einer der weiter oben genannten Basen - mit einem Alkylierungsmittel zur Reaktion. Das Alkylierungsmittel hat die allgemeine Formel $R^1$-$R^{21}$, wobei für $R^{21}$ die üblichen Abgangsgruppen, wie beispielsweise Chlor, Brom, Iod, Aryl- oder Alkylsulfonyl wie z. B. Toluolsulfonyl oder Methylsulfonyl in Betracht kommen. Die verwendeten Alkylierungsmittel sind im allgemeinen bekannt oder sie lassen sich analog zu bekannten Verbindungen herstellen.

Die Verbindungen I bzw. die sie enthaltenden Mittel sowie deren umweltverträgliche Salze z.B. von Alkalimetallen und Erdalkalimetallen können als Herbizide in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser

geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirksfoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. 20 Gew.-Teile der Verbindung Nr. 2.261 werden in einer Mischung gelost, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

II. 20 Gew.-Teile der Verbindung Nr. 2.261 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile des Wirkstoffs Nr. 2.261 werden in einer Mischung gelöst, die aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 2.261 werden mit 3 Gew.-Teilen des Natriumsalzes der Diisobutylnaphthalin-$\alpha$-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.

V. 3 Gew.-Teile des Wirkstoffs Nr. 2.261 werden mit 97 Gew.-Teilen feinteiligem Kaolin vermischt. Man erhält auf diese Weise ein Stäubemittel, das 3 Gew.-% des Wirkstoffs enthält.

VI. 20 Gew.-Teile des Wirkstoffs Nr. 2.261 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 2,0, vorzugsweise 0,01 bis 1,0 kg/ha aktive Substanz (a.S.).

In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius , Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays,

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig von der Pflanzenart und -sorte; von dem Zeitpunkt der Applikation, bezogen auf das Entwicklungsstadium der Pflanze, und von der Jahreszeit; von dem Applikationsort und -verfahren (z.B. Samenbeize, Bodenbehandlung, Blattapplikation oder Stamminjektion bei Bäumen); von klimatischen Faktoren (z.B. Temperatur, Niederschlagsmenge, außerdem Tageslänge und Lichtintensität); von der Bodenbeschaffenheit (einschließlich Düngung); von der Formulierung bzw. Anwendungsform des Wirkstoffs und von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:

A.

Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.

Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Langenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B.

Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B.

Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zukkerrohr sowie Zitrusfrüchte zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C.

Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D.

Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es nämlich zu einer besseren Ausnutzung des vorhandenen Wassers, weil u.a. die Öffnungsweite der Stomata reduziert wird, eine dickere Epidermis und Cuticula ausgebildet werden, die Durchwurzelung des Bodens verbessert wird und das Mikroklima im Pflanzenbestand durch einen kompakteren Wuchs günstig beeinflußt wird.

Besonders gut eignen sich die Verbindungen I zur Halmverkürzung von Kulturpflanzen wie Gerste, Raps und Weizen.

Die erfindungsgemäß zu verwendenden Wachstumsregulatoren der Formel I können den Kulturpflanzen sowohl von Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).

Die Aufwandmenge an Wirkstoff ist infolge der hohen Pflanzenverträglichkeit nicht kritisch. Die optimale Aufwandmenge variiert ja nach Bekämpfungsziel, Jahreszeit, Zielpflanzen und Wachstumsstadien.

Zur Verbreiterung des Wirkungsspektrums und zur Erzielung synergistischer Effekte können die Pyridin-N-oxid substituierten Salicylsäurederivate I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht. Bei Wachstumsregulatoren kommen insbesondere Chlormequatchlorid, Ethylen und Mepiquatchlorid in Frage.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Beispiel 1

a)

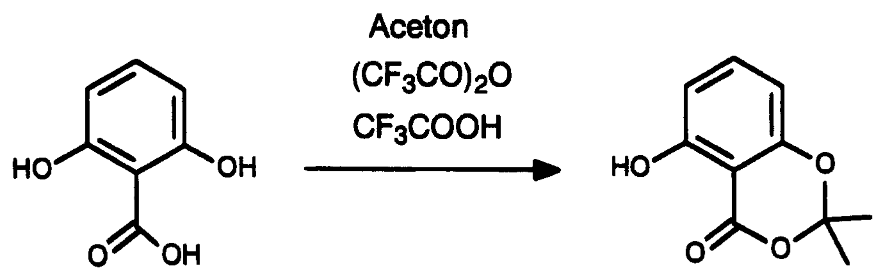


5-Hydroxy-2,2-dimethyl-4H-(1, 3)benzodioxin-4-on

Man legt 100 g (0,66 Mol) 2,6-Dihydroxybenzoesäure in 800 ml Trifluoressigsäure vor und gibt 100 ml Aceton sowie 216 g (1,98 Mol) Trifluoracetanhydrid zu. Man kocht ca. 2 h unter Rückfluß, und tropft anschließend unter Rückfluß 50 ml Aceton pro Stunde zu (gesamte Reaktionszeit 7,5 h, gesamte Acetonzugabe 375 ml). Die Reaktionsmischung wird im Vakuum bei ca. 55 °C eingeengt, dreimal mit Toluol aufgefüllt und wieder eingeengt und schließlich bei 45 °C an der Ölpumpe getrocknet.

Das ölige Produkt wird in 2 l Methyl-tert.-butylether aufgenommen, mit 2 l Wasser und 2 l gesättigter $NaHCO_3$-Lösung versetzt und 1,5 h gerührt. Man trennt ab, extrahiert mit Methyl-tert.-butylether, wäscht die vereinigten organischen Phasen mit Kochsalzlösung, trocknet über Natriumsulfat und engt ein. Der Rückstand wird mit 200 ml n-Pentan verrührt, abgesaugt und getrocknet. Ausbeute: 115 g (90%). Schmp. 60-62 °C.

b)

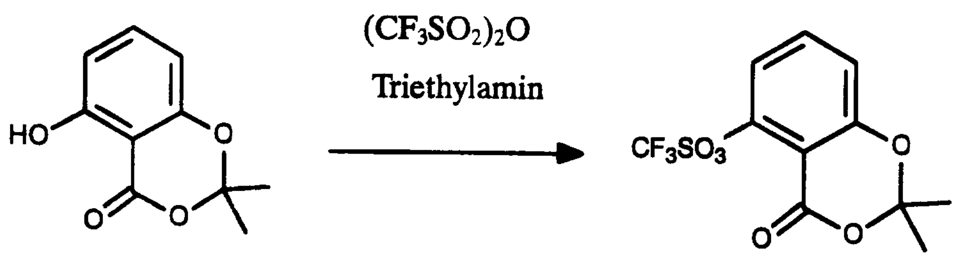


2,2-Dimethyl-5-trifluormethylsulfonyloxy-4H-(1, 3)benzodioxin-4-on:

Man löst 80 g (0,41 Mol) 5-Hydroxy-2,2-dimethyl-4H-(1, 3)benzodioxin-4-on in 1,5 l Methylenchlorid, gibt bei 0 °C 129 g (1,28 Mol) Triethylamin zu und tropft dann innerhalb von 2 h 314 g (1,11 Mol) Trifluormethylsulfonsäureanhydrid zu. Man läßt auf 10 °C erwärmen, rührt bei dieser Temperatur 10 min nach und gibt die Mischung dann unter Rühren in 1,5 l Wasser bei 0 °C. Man trennt die organische Phase ab, extrahiert mit Methylenchlorid, wäscht die vereinigten organischen Phasen mit Wasser und einmal mit gesättigter Kochsalzlösung, trocknet über Natriumsulfat und engt ein. Der Rückstand wird mit 200 ml n-Pentan verrührt, abfiltriert, mit n-Pentan nachgewaschen und bei 40 °C an der Ölpumpe getrocknet. Ausbeute: 118 g (88 %). Schmelzpunkt: 115 °C.

c)

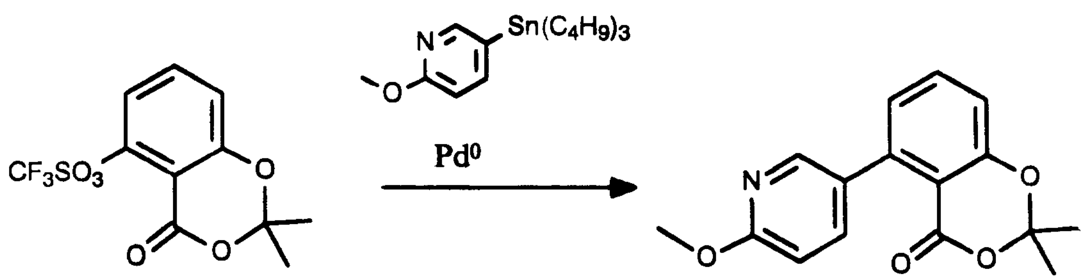


2,2-Dimethyl-5-(2-methoxypyridin-5-yl)-4H-(1, 3)benzodioxin-4-on

Man löst 8,7 g 2,2-Dimethyl-5-trifluormethylsulfonyloxy-4H-(1, 3)benzodioxin-4-on, 23,2 g 2-Methoxy-5-tributylstannylpyridin, 3,4 g Lithiumchlorid, 0,6 g Tetrakistriphenylphosphinpalladium$^0$ und 70 mg 2,6-Di-t.-butyl-4-methylphenol in 150 ml Dioxan und rührt 3 h bei 140 ° C im Autoklaven. Man engt im Vakuum ein, verrührt mit 200 ml n-Pentan, filtriert über wenig Kieselgel-60, wäscht mit n-Pentan nach und eluiert das Produkt mit Essigsäureethylester. Nach dem Einengen verbleiben 10,3 g vom Schmp. 160 ° C.

d)

2-(4,6-Dimethoxypyrimidin-2-yloxy)-6-(2-methoxypyridin-5-yl)-benzoesäureacetonoximester

0,67 g Acetonoxim, gelöst in 30 ml Toluol werden mit 1,66 g einer 30 %igen Natriummethylat-Lösung in Methanol versetzt und im Vakuum eingeengt. Man gibt 35 ml Dimethylformamid und dann 2,50 g 2,2-Dimethy1-5-(2-methoxyridin-5-yl)-4H-(1, 3)benzodioxin-4-on zu, rührt 15 min bei Raumtemperatur und fügt schließlich 1,90 g 4,6-Dimethoxy-2-methylsulfonylpyrimidin hinzu. Man rührt mehrere Stunden nach, gießt das Reaktionsgemisch in 300 ml Wasser, extrahiert mit Methyl-t.-butylether, wäscht gründlich mit Wasser und gesättigter Kochsalzlösung, trocknet über Natriumsulfat und engt im Vakuum ein. Das Rohprodukt wird durch Chromatographie an Kieselgel-60 mit n-Hexan/Essigsäureethylester gereinigt. $^1$H-NMR ($CDCl_3$) $\delta$ = 1,65(s); 1,95(s); 3,85(s); 3,98(s); 5,78(s); 6,77(d); 7,3-7,7(m); 8,25(d).

Beispiel 2

a)

2,2-Dimethyl-5-(6-methoxypyridin-2-yl)-4H-(1, 3)benzodioxin-4-on

Man löst 10,9 g 2,2-Dimethyl-5-trifluormethylsulfonyloxy-4H-(1, 3)benzodioxin-4-on, 16,0 g 6-Methoxy-2-tributylstannylpyridin, 4,25 g Lithiumchlorid, 0,78 g Tetrakistriphenylphosphinpalladium$^0$ und 40 mg 2,6-Di-t.-butyl-4-methylphenol in 120 ml Dioxan und rührt 4 h bei 140 ° C im Autoklaven. Man engt im Vakuum ein und chromatographiert das Produkt an Kieselgel-60 mit Essigsäureethylester/Toluol. Man erhält 9,1 g (95 %) eines Feststoffes vom Schmp. 130-132 ° C.

b)

2-(4,6-Dimethoxypyrimidin-2-yloxy)-6-(6-methoxypyridin-2-yl)-benzoesäurebenzylester

1,36 g Benzylalkohol gelöst in 50 ml Dimethylformamid werden mit 380 mg Natriumhydrid (80 %ig in Paraffinöl) versetzt und ca. 1 h bei Raumtemperatur nachgerührt. Man gibt 3,0 g 2,2-Dimethyl-5-(6-methoxypyridin-2-yl)-4H-(1,3)benzodioxin-4-onzu, rührt 3 h bei Raumtemperatur und fügt schließlich 2,65 g 4,6-Dimethoxy-2-methylsulfonylpyrimidin hinzu. Man rührt 2 h bei Raumtemperatur, 1,5 h bei 80 ° C und 3 h bei 115 ° C, gießt in phosphorsaures Eiswasser, extrahiert mit Essigsäureethylester, wäscht mit Wasser, trocknet über Natriumsulfat und engt im Vakuum ein. Das Rohprodukt wird durch Chromatographie an Kieselgel-60 mit Cyclohexan/Toluol/Essigsäureethylester gereinigt. $^1$H-NMR ($CDCl_3$): $\delta$ = 3,80(s); 3,89(s); 5,02(s); 5,72(s); 6,67(d = ; 6,7-7,7(m).

Beispiel 3

2,2-Dimethyl-5-(1-oxopyridin-4-yl)-4H-(1, 3)benzodioxin-4-on:

Man legt 300 mg 2,2-Dimethyl-5-(pyridin-4-yl)-4H-(1, 3)benzodioxin-4-on in 20 ml Methylenchlorid vor, gibt bei Raumtemperatur 260 mg 3-Chlorperbenzoesäure zu und rührt über Nacht. Die Reaktionsmischung wird direkt durch Chromatographie an Kieselgel-60 mit Methylenchlorid/Methanol aufgearbeitet. Ausbeute: 200 mg. $^1$H-NMR ($CDCl_3$): $\delta$: 1,80 (s); 6,95 (d); 7,07 (d); 7,25 (d); 7,60 (t); 8,25(d).

Beispiel 4

2,2-Dimethyl-5-(2-methoxy-1-oxopyridin-5-yl)-4H-(1,3)benzodioxin-4-on:
Man legt 4,17 g 2,2-Dimethyl-5-(2-methoxypvridin-5-yl)-4H-(1, 3)benzodioxin-4-on in 200 ml Methylenchlorid vor, gibt bei Raumtemperatur 6,0 g 3-Chlorperbenzoesäure zu und rührt über Nacht. Nach Zugabe von etwas Natriumsulfat wird die Reaktionsmischung direkt durch Chromatographie an Kieselgel-60 mit Methylenchlorid / Ethylacetat / Methanol aufgearbeitet. Ausbeute: 2,3 g. Fp.: 82 ° C.

Beispiel 5

2-(4,6-Dimethoxypyrimidin-2-yloxy)-6-(1-oxopyridin-3-yl)-benzoesäurebenzylester:
Man legt 3,0 g 2-(4,6-Dimethoxypyrimidin-2-yloxy)-6-(pyridin-3-yl)-benzoesäurebenzylester in 100 ml Methylenchlorid vor, gibt bei Raumtemperatur 2,8 g 3-Chlorperbenzoesäure zu und rührt über Nacht. Die Reaktionsmischung wird direkt durch Chromatographie an Kieselgel-60 mit Methylenchlorid / Ethylacetat / Methanol aufgearbeitet. Ausbeute: 3,0 g. $^1$H-NMR ($CDCl_3$): δ: 3,78 (s); 5,03 (s); 5,73 (s); 7,00-7,40 (m); 7,55 (t); 8,10 (d); 8,27(s).

Beispiel 6

2-(4,6-Dimethoxypyrimidin-2-yloxy)-6-(6-methyl-1-oxopyridin-2-yl)-benzoesäure:
Man legt 200 mg 2-(4,6-Dimethoxypyrimidin-2-yloxy)-6-(6-methylpyridin-2-yl)-benzoesäure in 10 ml Essigsäure vor, gibt bei Raumtemperatur 68 mg 30%iges Wasserstoffperoxid zu und rührt über Nacht. Die Reaktionsmischung wird direkt im Vakuum eingeengt, in Methylenchlorid aufgenommen, mit Wasser gewaschen, über Natriumsulfat getrocknet und wieder im Vakuum eingeengt. Ausbeute: 190 mg. $^1$H-NMR ($d_6$-DMSO): δ: 2,50 (s); 3,82 (s); 5,50 (s); 6,95 (d); 7,02 (d); 7,20-7,35 (m); 7,43 (d); 7,80 (t).

Beispiel 7

2-(4,6-Dimethoxypyrimidin-2-yloxy)-6-(1-oxopyridin-3-yl)-benzoesäureacetonoximester:
Man legt 370 mg 2-(4,6-Dimethoxypyrimidin-2-yloxy)-6-(pyridin-3-yl)-benzoesäureacetonoximester in 25 ml Methylenchlorid vor, gibt bei Raumtemperatur 370 mg 3-Chlorperbenzoesäure zu und rührt über Nacht. Die Reaktionsmischung wird direkt durch Chromatographie an Kieselgel-60 mit Methylenchlorid / Ethylacetat / Methanol aufgearbeitet. Ausbeute: quantitativ. $^1$H-NMR ($CDCl_3$): δ: 1,74 (s); 1,95 (s); 3,83 (s); 5,77 (s); 7,25-7,45 (m); 7,60 (t); 8,23 (d); 8,34 (s).

Besonders bevorzugte Verbindungen der Formel I, die gemäß den voranstehenden Beispielen herstellbar sind, sind in den nachfolgenden Tabellen 1 bis 6 zusammengestellt.

Tabelle 1

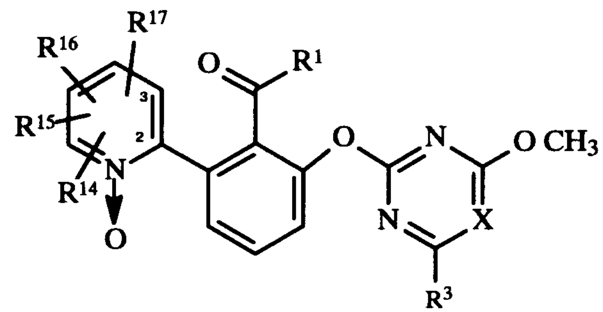

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.001 | OH | $OCH_3$ | CH | 3-$OCH_3$ | H | H | H | |
| 1.002 | OH | $OCH_3$ | CH | 4-$OCH_3$ | H | H | H | |
| 1.003 | OH | $OCH_3$ | CH | 5-$OCH_3$ | H | H | H | |
| 1.004 | OH | $OCH_3$ | CH | 6-$OCH_3$ | H | H | H | |
| 1.005 | 2-Propaniminoxy | $OCH_3$ | CH | 3-$OCH_3$ | H | H | H | |
| 1.006 | 2-Propaniminoxy | $OCH_3$ | CH | 4-$OCH_3$ | H | H | H | |
| 1.007 | 2-Propaniminoxy | $OCH_3$ | CH | 5-$OCH_3$ | H | H | H | |
| 1.008 | 2-Propaniminoxy | $OCH_3$ | CH | 6-$OCH_3$ | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.009 | Benzyloxy | $OCH_3$ | CH | 3-$OCH_3$ | H | H | H | |
| 1.010 | Benzyloxy | $OCH_3$ | CH | 4-$OCH_3$ | H | H | H | |
| 1.011 | Benzyloxy | $OCH_3$ | CH | 5-$OCH_3$ | H | H | H | |
| 1.012 | Benzyloxy | $OCH_3$ | CH | 6-$OCH_3$ | H | H | H | |
| 1.013 | OH | $OCH_3$ | CH | 3-$OC_2H_5$ | H | H | H | |
| 1.014 | OH | $OCH_3$ | CH | 4-$OC_2H_5$ | H | H | H | |
| 1.015 | OH | $OCH_3$ | CH | 5-$OC_2H_5$ | H | H | H | |
| 1.016 | OH | $OCH_3$ | CH | 6-$OC_2H_5$ | H | H | H | |
| 1.017 | 2-Propaniminoxy | $OCH_3$ | CH | 3-$OC_2H_5$ | H | H | H | |
| 1.018 | 2-Propaniminoxy | $OCH_3$ | CH | 4-$OC_2H_5$ | H | H | H | |
| 1.019 | 2-Propaniminoxy | $OCH_3$ | CH | 5-$OC_2H_5$ | H | H | H | |
| 1.020 | 2-Propaniminoxy | $OCH_3$ | CH | 6-$OC_2H_5$ | H | H | H | |
| 1.021 | Benzyloxy | $OCH_3$ | CH | 3-$OC_2H_5$ | H | H | H | |
| 1.022 | Benzyloxy | $OCH_3$ | CH | 4-$OC_2H_5$ | H | H | H | |
| 1.023 | Benzyloxy | $OCH_3$ | CH | 5-$OC_2H_5$ | H | H | H | |
| 1.024 | Benzyloxy | $OCH_3$ | CH | 6-$OC_2H_5$ | H | H | H | |
| 1.025 | OH | $OCH_3$ | CH | 3-n-$C_3H_7$ | H | H | H | |
| 1.026 | OH | $OCH_3$ | CH | 4-n-$C_3H_7$ | H | H | H | |
| 1.027 | OH | $OCH_3$ | CH | 5-n-$C_3H_7$ | H | H | H | |
| 1.028 | OH | $OCH_3$ | CH | 6-n-$C_3H_7$ | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.029 | 2-Propaniminoxy | $OCH_3$ | CH | 3-n-$C_3H_7$ | H | H | H | |
| 1.030 | 2-Propaniminoxy | $OCH_3$ | CH | 4-n-$C_3H_7$ | H | H | H | |
| 1.031 | 2-Propaniminoxy | $OCH_3$ | CH | 5-n-$C_3H_7$ | H | H | H | |
| 1.032 | 2-Propaniminoxy | $OCH_3$ | CH | 6-n-$C_3H_7$ | H | H | H | |
| 1.033 | Benzyloxy | $OCH_3$ | CH | 3-n-$C_3H_7$ | H | H | H | |
| 1.034 | Benzyloxy | $OCH_3$ | CH | 4-n-$C_3H_7$ | H | H | H | |
| 1.035 | Benzyloxy | $OCH_3$ | CH | 5-n-$C_3H_7$ | H | H | H | |
| 1.036 | Benzyloxy | $OCH_3$ | CH | 6-n-$C_3H_7$ | H | H | H | |
| 1.037 | OH | $OCH_3$ | CH | 3-i-$C_3H_7$ | H | H | H | |
| 1.038 | OH | $OCH_3$ | CH | 4-i-$C_3H_7$ | H | H | H | |
| 1.039 | OH | $OCH_3$ | CH | 5-i-$C_3H_7$ | H | H | H | |
| 1.040 | OH | $OCH_3$ | CH | 6-i-$C_3H_7$ | H | H | H | |
| 1.041 | 2-Propaniminoxy | $OCH_3$ | CH | 3-i-$C_3H_7$ | H | H | H | |
| 1.042 | 2-Propaniminoxy | $OCH_3$ | CH | 4-i-$C_3H_7$ | H | H | H | |
| 1.043 | 2-Propaniminoxy | $OCH_3$ | CH | 5-i-$C_3H_7$ | H | H | H | |
| 1.044 | 2-Propaniminoxy | $OCH_3$ | CH | 6-i-$C_3H_7$ | H | H | H | |
| 1.045 | Benzyloxy | $OCH_3$ | CH | 3-i-$C_3H_7$ | H | H | H | |
| 1.046 | Benzyloxy | $OCH_3$ | CH | 4-i-$C_3H_7$ | H | H | H | |
| 1.047 | Benzyloxy | $OCH_3$ | CH | 5-i-$C_3H_7$ | H | H | H | |
| 1.048 | Benzyloxy | $OCH_3$ | CH | 6-i-$C_3H_7$ | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.049 | OH | $OCH_3$ | CH | 3-$SCH_3$ | H | H | H | |
| 1.050 | OH | $OCH_3$ | CH | 4-$SCH_3$ | H | H | H | |
| 1.051 | OH | $OCH_3$ | CH | 5-$SCH_3$ | H | H | H | |
| 1.052 | OH | $OCH_3$ | CH | 6-$SCH_3$ | H | H | H | |
| 1.053 | 2-Propaniminoxy | $OCH_3$ | CH | 3-$SCH_3$ | H | H | H | |
| 1.054 | 2-Propaniminoxy | $OCH_3$ | CH | 4-$SCH_3$ | H | H | H | |
| 1.055 | 2-Propaniminoxy | $OCH_3$ | CH | 5-$SCH_3$ | H | H | H | |
| 1.056 | 2-Propaniminoxy | $OCH_3$ | CH | 6-$SCH_3$ | H | H | H | |
| 1.057 | Benzyloxy | $OCH_3$ | CH | 3-$SCH_3$ | H | H | H | |
| 1.058 | Benzyloxy | $OCH_3$ | CH | 4-$SCH_3$ | H | H | H | |
| 1.059 | Benzyloxy | $OCH_3$ | CH | 5-$SCH_3$ | H | H | H | |
| 1.060 | Benzyloxy | $OCH_3$ | CH | 6-$SCH_3$ | H | H | H | |
| 1.061 | OH | $OCH_3$ | CH | 3-$SC_2H_5$ | H | H | H | |
| 1.062 | OH | $OCH_3$ | CH | 4-$SC_2H_5$ | H | H | H | |
| 1.063 | OH | $OCH_3$ | CH | 5-$SC_2H_5$ | H | H | H | |
| 1.064 | OH | $OCH_3$ | CH | 6-$SC_2H_5$ | H | H | H | |
| 1.065 | 2-Propaniminoxy | $OCH_3$ | CH | 3-$SC_2H_5$ | H | H | H | |
| 1.066 | 2-Propaniminoxy | $OCH_3$ | CH | 4-$SC_2H_5$ | H | H | H | |
| 1.067 | 2-Propaniminoxy | $OCH_3$ | CH | 5-$SC_2H_5$ | H | H | H | |
| 1.068 | 2-Propaniminoxy | $OCH_3$ | CH | 6-$SC_2H_5$ | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.069 | Benzyloxy | $OCH_3$ | CH | 3-$SC_2H_5$ | H | H | H | |
| 1.070 | Benzyloxy | $OCH_3$ | CH | 4-$SC_2H_5$ | H | H | H | |
| 1.071 | Benzyloxy | $OCH_3$ | CH | 5-$SC_2H_5$ | H | H | H | |
| 1.072 | Benzyloxy | $OCH_3$ | CH | 6-$SC_2H_5$ | H | H | H | |
| 1.073 | OH | $OCH_3$ | CH | 3-S-n-$C_3H_7$ | H | H | H | |
| 1.074 | OH | $OCH_3$ | CH | 4-S-n-$C_3H_7$ | H | H | H | |
| 1.075 | OH | $OCH_3$ | CH | 5-S-n-$C_3H_7$ | H | H | H | |
| 1.076 | OH | $OCH_3$ | CH | 6-S-n-$C_3H_7$ | H | H | H | |
| 1.077 | 2-Propaniminoxy | $OCH_3$ | CH | 3-S-n-$C_3H_7$ | H | H | H | |
| 1.078 | 2-Propaniminoxy | $OCH_3$ | CH | 4-S-n-$C_3H_7$ | H | H | H | |
| 1.079 | 2-Propaniminoxy | $OCH_3$ | CH | 5-S-n-$C_3H_7$ | H | H | H | |
| 1.080 | 2-Propaniminoxy | $OCH_3$ | CH | 6-S-n-$C_3H_7$ | H | H | H | |
| 1.081 | Benzyloxy | $OCH_3$ | CH | 3-S-n-$C_3H_7$ | H | H | H | |
| 1.082 | Benzyloxy | $OCH_3$ | CH | 4-S-n-$C_3H_7$ | H | H | H | |
| 1.083 | Benzyloxy | $OCH_3$ | CH | 5-S-n-$C_3H_7$ | H | H | H | |
| 1.084 | Benzyloxy | $OCH_3$ | CH | 6-S-n-$C_3H_7$ | H | H | H | |
| 1.085 | OH | $OCH_3$ | CH | 3-S-i-$C_3H_7$ | H | H | H | |
| 1.086 | OH | $OCH_3$ | CH | 4-S-i-$C_3H_7$ | H | H | H | |
| 1.087 | OH | $OCH_3$ | CH | 5-S-i-$C_3H_7$ | H | H | H | |
| 1.088 | OH | $OCH_3$ | CH | 6-S-i-$C_3H_7$ | H | H | H | |

| Nr. | $R^1$ | $R^3$ | X | $R^{14}$ | $R^{15}$ | $R^{16}$ | $R^{17}$ | Phys. Daten $^1$H-NMR (DMSO) |
|---|---|---|---|---|---|---|---|---|
| 1.089 | 2-Propaniminoxy | $OCH_3$ | CH | 3-S-i-$C_3H_7$ | H | H | H | |
| 1.090 | 2-Propaniminoxy | $OCH_3$ | CH | 4-S-i-$C_3H_7$ | H | H | H | |
| 1.091 | 2-Propaniminoxy | $OCH_3$ | CH | 5-S-i-$C_3H_7$ | H | H | H | |
| 1.092 | 2-Propaniminoxy | $OCH_3$ | CH | 6-S-i-$C_3H_7$ | H | H | H | |
| 1.093 | Benzyloxy | $OCH_3$ | CH | 3-S-i-$C_3H_7$ | H | H | H | |
| 1.094 | Benzyloxy | $OCH_3$ | CH | 4-S-i-$C_3H_7$ | H | H | H | |
| 1.095 | Benzyloxy | $OCH_3$ | CH | 5-S-i-$C_3H_7$ | H | H | H | |
| 1.096 | Benzyloxy | $OCH_3$ | CH | 6-S-i-$C_3H_7$ | H | H | H | |
| 1.097 | OH | $OCH_3$ | CH | 6-$OCH_2CF_3$ | H | H | H | |
| 1.098 | 2-Propaniminoxy | $OCH_3$ | CH | 6-$OCH_2CF_3$ | H | H | H | |
| 1.099 | Benzyloxy | $OCH_3$ | CH | 6-$OCH_2CF_3$ | H | H | H | |
| 1.100 | OH | $OCH_3$ | CH | 6-$OCH_2CH_2OCH_3$ | H | H | H | |
| 1.101 | 2-Propaniminoxy | $OCH_3$ | CH | 6-$OCH_2CH_2OCH_3$ | H | H | H | |
| 1.102 | Benzyloxy | $OCH_3$ | CH | 6-$OCH_2CH_2OCH_3$ | H | H | H | |
| 1.103 | OH | $OCH_3$ | CH | 6-$OCH_2CH_2N(CH_3)_2$ | H | H | H | |
| 1.104 | 2-Propaniminoxy | $OCH_3$ | CH | 6-$OCH_2CH_2N(CH_3)_2$ | H | H | H | |
| 1.105 | Benzyloxy | $OCH_3$ | CH | 6-$OCH_2CH_2N(CH_3)_2$ | H | H | H | |
| 1.106 | OH | $OCH_3$ | CH | 6-$ON(CH_3)_2$ | H | H | H | |
| 1.107 | 2-Propaniminoxy | $OCH_3$ | CH | 6-$ON(CH_3)_2$ | H | H | H | |
| 1.108 | Benzyloxy | $OCH_3$ | CH | 6-$ON(CH_3)_2$ | H | H | H | |

| Nr. | $R^1$ | $R^3$ | X | $R^{14}$ | $R^{15}$ | $R^{16}$ | $R^{17}$ | Phys. Daten $^1$H-NMR (DMSO) |
|---|---|---|---|---|---|---|---|---|
| 1.109 | $OCH_2CF_3$ | $OCH_3$ | CH | $3-OCH_3$ | H | H | H | |
| 1.110 | $OCH_2CF_3$ | $OCH_3$ | CH | $4-OCH_3$ | H | H | H | |
| 1.111 | $OCH_2CF_3$ | $OCH_3$ | CH | $5-OCH_3$ | H | H | H | |
| 1.112 | $OCH_2CF_3$ | $OCH_3$ | CH | $6-OCH_3$ | H | H | H | |
| 1.113 | Propargyloxy | $OCH_3$ | CH | $3-OCH_3$ | H | H | H | |
| 1.114 | Propargyloxy | $OCH_3$ | CH | $4-OCH_3$ | H | H | H | |
| 1.115 | Propargyloxy | $OCH_3$ | CH | $5-OCH_3$ | H | H | H | |
| 1.116 | Propargyloxy | $OCH_3$ | CH | $6-OCH_3$ | H | H | H | |
| 1.117 | Allyloxy | $OCH_3$ | CH | $3-OCH_3$ | H | H | H | |
| 1.118 | Allyloxy | $OCH_3$ | CH | $4-OCH_3$ | H | H | H | |
| 1.119 | Allyloxy | $OCH_3$ | CH | $5-OCH_3$ | H | H | H | |
| 1.120 | Allyloxy | $OCH_3$ | CH | $6-OCH_3$ | H | H | H | |
| 1.121 | OH | $OCH_2CH_2$ | | $3-OCH_3$ | H | H | H | |
| 1.122 | OH | $OCH_2CH_2$ | | $4-OCH_3$ | H | H | H | |
| 1.123 | OH | $OCH_2CH_2$ | | $5-OCH_3$ | H | H | H | |
| 1.124 | OH | $OCH_2CH_2$ | | $6-OCH_3$ | H | H | H | |
| 1.125 | OH | $OCH_3$ | CF | $3-OCH_3$ | H | H | H | |
| 1.126 | OH | $OCH_3$ | CF | $4-OCH_3$ | H | H | H | |
| 1.127 | OH | $OCH_3$ | CF | $5-OCH_3$ | H | H | H | |
| 1.128 | OH | $OCH_3$ | CF | $6-OCH_3$ | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.129 | OH | $OCH_3$ | CH | 3-$OCH_3$ | 4-$CH_3$ | H | H | |
| 1.130 | OH | $OCH_3$ | CH | 3-$OCH_3$ | 5-$CH_3$ | H | H | |
| 1.131 | OH | $OCH_3$ | CH | 3-$OCH_3$ | 6-$CH_3$ | H | H | |
| 1.132 | OH | $OCH_3$ | CH | 4-$OCH_3$ | 3-$CH_3$ | H | H | |
| 1.133 | OH | $OCH_3$ | CH | 4-$OCH_3$ | 5-$CH_3$ | H | H | |
| 1.134 | OH | $OCH_3$ | CH | 4-$OCH_3$ | 6-$CH_3$ | H | H | |
| 1.135 | OH | $OCH_3$ | CH | 5-$OCH_3$ | 4-$CH_3$ | H | H | |
| 1.136 | OH | $OCH_3$ | CH | 5-$OCH_3$ | 3-$CH_3$ | H | H | |
| 1.137 | OH | $OCH_3$ | CH | 5-$OCH_3$ | 6-$CH_3$ | H | H | |
| 1.138 | OH | $OCH_3$ | CH | 6-$OCH_3$ | 4-$CH_3$ | H | H | |
| 1.139 | OH | $OCH_3$ | CH | 6-$OCH_3$ | 5-$CH_3$ | H | H | |
| 1.140 | OH | $OCH_3$ | CH | 6-$OCH_3$ | 3-$CH_3$ | H | H | |
| 1.141 | OH | $OCH_3$ | CH | 3-$SCH_3$ | 4-$CH_3$ | H | H | |
| 1.142 | OH | $OCH_3$ | CH | 3-$SCH_3$ | 5-$CH_3$ | H | H | |
| 1.143 | OH | $OCH_3$ | CH | 3-$SCH_3$ | 6-$CH_3$ | H | H | |
| 1.144 | OH | $OCH_3$ | CH | 4-$SCH_3$ | 3-$CH_3$ | H | H | |
| 1.145 | OH | $OCH_3$ | CH | 4-$SCH_3$ | 5-$CH_3$ | H | H | |
| 1.146 | OH | $OCH_3$ | CH | 4-$SCH_3$ | 6-$CH_3$ | H | H | |
| 1.147 | OH | $OCH_3$ | CH | 5-$SCH_3$ | 4-$CH_3$ | H | H | |
| 1.148 | OH | $OCH_3$ | CH | 5-$SCH_3$ | 3-($CH_3$) | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.149 | OH | $OCH_3$ | CH | 5-$SCH_3$ | 6-($CH_3$) | H | H | |
| 1.150 | OH | $OCH_3$ | CH | 6-$SCH_3$ | 4-($CH_3$) | H | H | |
| 1.151 | OH | $OCH_3$ | CH | 6-$SCH_3$ | 5-($CH_3$) | H | H | |
| 1.152 | OH | $OCH_3$ | CH | 6-$SCH_3$ | 3-($CH_3$) | H | H | |
| 1.153 | OH | $OCH_3$ | CH | 3-Methyl | H | H | H | |
| 1.154 | OH | $OCH_3$ | CH | 4-Methyl | H | H | H | |
| 1.155 | OH | $OCH_3$ | CH | 5-Methyl | H | H | H | |
| 1.156 | OH | $OCH_3$ | CH | 6-Methyl | H | H | H | $\delta$ [ppm]: 2,50(s); 3,80(s); 5,50(s); 6,95(d); 7,03(d); 7,30(m); 7,45(d); 7,80(t) |
| 1.157 | OH | $OCH_3$ | CH | 3-Ethyl | H | H | H | |
| 1.158 | OH | $OCH_3$ | CH | 4-Ethyl | H | H | H | |
| 1.159 | OH | $OCH_3$ | CH | 5-Ethyl | H | H | H | |
| 1.160 | OH | $OCH_3$ | CH | 6-Ethyl | H | H | H | |
| 1.161 | OH | $OCH_3$ | CH | 3-$CF_3$ | H | H | H | |
| 1.162 | OH | $OCH_3$ | CH | 4-$CF_3$ | H | H | H | |
| 1.163 | OH | $OCH_3$ | CH | 5-$CF_3$ | H | H | H | |
| 1.164 | OH | $OCH_3$ | CH | 6-$CF_3$ | H | H | H | |
| 1.165 | OH | $OCH_3$ | CH | 3-Cl | H | H | H | |
| 1.166 | OH | $OCH_3$ | CH | 4-Cl | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.167 | OH | $OCH_3$ | CH | 5-Cl | H | H | H | |
| 1.168 | OH | $OCH_3$ | CH | 6-Cl | H | H | H | |
| 1.169 | OH | $OCH_3$ | CH | 3-F | H | H | H | |
| 1.170 | OH | $OCH_3$ | CH | 4-F | H | H | H | |
| 1.171 | OH | $OCH_3$ | CH | 5-F | H | H | H | |
| 1.172 | OH | $OCH_3$ | CH | 6-F | H | H | H | |
| 1.173 | OH | $OCH_3$ | CH | 3-O-i-$C_3H_7$ | H | H | H | |
| 1.174 | OH | $OCH_3$ | CH | 4-O-i-$C_3H_7$ | H | H | H | |
| 1.175 | OH | $OCH_3$ | CH | 5-O-i-$C_3H_7$ | H | H | H | |
| 1.176 | OH | $OCH_3$ | CH | 6-O-i-$C_3H_7$ | H | H | H | |
| 1.177 | OH | $OCH_3$ | CH | 3-$CF_3$ | 6-$OCH_3$ | H | H | |
| 1.178 | OH | $OCH_3$ | CH | 4-$CF_3$ | 6-$OCH_3$ | H | H | |
| 1.179 | OH | $OCH_3$ | CH | 5-$CF_3$ | 6-Cl | 4-F | H | |
| 1.180 | OH | $OCH_3$ | CH | 6-$CF_3$ | 4-$CH_3$ | H | H | |
| 1.181 | 2-Propaniminoxy | $OCH_3$ | CH | 3-Methyl | H | H | H | |
| 1.182 | 2-Propaniminoxy | $OCH_3$ | CH | 4-Methyl | H | H | H | |
| 1.183 | 2-Propaniminoxy | $OCH_3$ | CH | 5-Methyl | H | H | H | |
| 1.184 | 2-Propaniminoxy | $OCH_3$ | CH | 6-Methyl | H | H | H | |
| 1.185 | 2-Propaniminoxy | $OCH_3$ | CH | 3-Ethyl | H | H | H | |
| 1.186 | 2-Propaniminoxy | $OCH_3$ | CH | 4-Ethyl | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.187 | 2-Propaniminoxy | $OCH_3$ | CH | 5-Ethyl | H | H | H | |
| 1.188 | 2-Propaniminoxy | $OCH_3$ | CH | 6-Ethyl | H | H | H | |
| 1.189 | 2-Propaniminoxy | $OCH_3$ | CH | 3-$CF_3$ | H | H | H | |
| 1.190 | 2-Propaniminoxy | $OCH_3$ | CH | 4-$CF_3$ | H | H | H | |
| 1.191 | 2-Propaniminoxy | $OCH_3$ | CH | 5-$CF_3$ | H | H | H | |
| 1.192 | 2-Propaniminoxy | $OCH_3$ | CH | 6-$CF_3$ | H | H | H | |
| 1.193 | 2-Propaniminoxy | $OCH_3$ | CH | 3-Cl | H | H | H | |
| 1.194 | 2-Propaniminoxy | $OCH_3$ | CH | 4-Cl | H | H | H | |
| 1.195 | 2-Propaniminoxy | $OCH_3$ | CH | 5-Cl | H | H | H | |
| 1.196 | 2-Propaniminoxy | $OCH_3$ | CH | 6-Cl | H | H | H | |
| 1.197 | 2-Propaniminoxy | $OCH_3$ | CH | 3-F | H | H | H | |
| 1.198 | 2-Propaniminoxy | $OCH_3$ | CH | 4-F | H | H | H | |
| 1.199 | 2-Propaniminoxy | $OCH_3$ | CH | 5-F | H | H | H | |
| 1.200 | 2-Propaniminoxy | $OCH_3$ | CH | 6-F | H | H | H | |
| 1.201 | 2-Propaniminoxy | $OCH_3$ | CH | 3-O-i-$C_3H_7$ | H | H | H | |
| 1.202 | 2-Propaniminoxy | $OCH_3$ | CH | 4-O-i-$C_3H_7$ | H | H | H | |
| 1.203 | 2-Propaniminoxy | $OCH_3$ | CH | 5-O-i-$C_3H_7$ | H | H | H | |
| 1.204 | 2-Propaniminoxy | $OCH_3$ | CH | 6-O-i-$C_3H_7$ | H | H | H | |
| 1.205 | 2-Propaniminoxy | $OCH_3$ | CH | 3-$CF_3$ | 6-$OCH_3$ | H | H | |
| 1.206 | 2-Propaniminoxy | $OCH_3$ | CH | 4-$CF_3$ | 6-$OCH_3$ | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.207 | 2-Propaniminoxy | $OCH_3$ | CH | 5-$CF_3$ | 6-Cl | 4-F | H | |
| 1.208 | 2-Propaniminoxy | $OCH_3$ | CH | 6-$CF_3$ | 4-$CH_3$ | H | H | |
| 1.209 | $OCH_2CF_3$ | $OCH_3$ | CH | 3-Methyl | H | H | H | |
| 1.210 | $OCH_2CF_3$ | $OCH_3$ | CH | 4-Methyl | H | H | H | |
| 1.211 | $OCH_2CF_3$ | $OCH_3$ | CH | 5-Methyl | H | H | H | |
| 1.212 | $OCH_2CF_3$ | $OCH_3$ | CH | 6-Methyl | H | H | H | |
| 1.213 | $OCH_2CF_3$ | $OCH_3$ | CH | 3-Ethyl | H | H | H | |
| 1.214 | $OCH_2CF_3$ | $OCH_3$ | CH | 4-Ethyl | H | H | H | |
| 1.215 | $OCH_2CF_3$ | $OCH_3$ | CH | 5-Ethyl | H | H | H | |
| 1.216 | $OCH_2CF_3$ | $OCH_3$ | CH | 6-Ethyl | H | H | H | |
| 1.217 | $OCH_2CF_3$ | $OCH_3$ | CH | 3-$CF_3$ | H | H | H | |
| 1.218 | $OCH_2CF_3$ | $OCH_3$ | CH | 4-$CF_3$ | H | H | H | |
| 1.219 | $OCH_2CF_3$ | $OCH_3$ | CH | 5-$CF_3$ | H | H | H | |
| 1.220 | $OCH_2CF_3$ | $OCH_3$ | CH | 6-$CF_3$ | H | H | H | |
| 1.221 | $OCH_2CF_3$ | $OCH_3$ | CH | 3-Cl | H | H | H | |
| 1.222 | $OCH_2CF_3$ | $OCH_3$ | CH | 4-Cl | H | H | H | |
| 1.223 | $OCH_2CF_3$ | $OCH_3$ | CH | 5-Cl | H | H | H | |
| 1.224 | $OCH_2CF_3$ | $OCH_3$ | CH | 6-Cl | H | H | H | |
| 1.225 | $OCH_2CF_3$ | $OCH_3$ | CH | 3-F | H | H | H | |
| 1.226 | $OCH_2CF_3$ | $OCH_3$ | CH | 4-F | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.227 | $OCH_2CF_3$ | $OCH_3$ | CH | 5-F | H | H | H | |
| 1.228 | $OCH_2CF_3$ | $OCH_3$ | CH | 6-F | H | H | H | |
| 1.229 | $OCH_2CF_3$ | $OCH_3$ | CH | 3-O-i-$C_3H_7$ | H | H | H | |
| 1.230 | $OCH_2CF_3$ | $OCH_3$ | CH | 4-O-i-$C_3H_7$ | H | H | H | |
| 1.231 | $OCH_2CF_3$ | $OCH_3$ | CH | 5-O-i-$C_3H_7$ | H | H | H | |
| 1.232 | $OCH_2CF_3$ | $OCH_3$ | CH | 6-O-i-$C_3H_7$ | H | H | H | |
| 1.233 | $OCH_2CF_3$ | $OCH_3$ | CH | 3-$CF_3$ | 6-$OCH_3$ | H | H | |
| 1.234 | $OCH_2CF_3$ | $OCH_3$ | CH | 4-$CF_3$ | 6-$OCH_3$ | H | H | |
| 1.235 | $OCH_2CF_3$ | $OCH_3$ | CH | 5-$CF_3$ | 6-Cl | 4-F | H | |
| 1.236 | $OCH_2CF_3$ | $OCH_3$ | CH | 6-$CF_3$ | 4-$CH_3$ | H | H | |
| 1.237 | OH | $OCH_3$ | CH | H | H | H | H | |
| 1.238 | $OCH_2SCH_3$ | $OCH_3$ | CH | H | H | H | H | |
| 1.239 | $OC_2H_4OCH_3$ | $OCH_3$ | CH | H | H | H | H | |
| 1.240 | $OCH(CH_3)COOC_2H_5$ | $OCH_3$ | CH | H | H | H | H | |
| 1.241 | $OCH_2OCH_3$ | $OCH_3$ | CH | H | H | H | H | |
| 1.242 | Cyclohexanimin-oxy | $OCH_3$ | CH | H | H | H | H | |
| 1.243 | Cyclopentanimin-oxy | $OCH_3$ | CH | H | H | H | H | |
| 1.244 | Allyloxy | $OCH_3$ | CH | H | H | H | H | |
| 1.245 | Propargyloxy | $OCH_3$ | CH | H | H | H | H | |

| Nr. | $R^1$ | $R^3$ | X | $R^{14}$ | $R^{15}$ | $R^{16}$ | $R^{17}$ | Phys. Daten $^1H$-NMR (DMSO) |
|---|---|---|---|---|---|---|---|---|
| 1.246 | OH | $OCH_3$ | CH | 3-$OCH_3$ | 6-Cl | H | H | |
| 1.247 | OH | $OCH_3$ | CH | 4-$OCH_3$ | 6-Cl | H | H | |
| 1.248 | OH | $OCH_3$ | CH | 5-$OCH_3$ | 6-Cl | H | H | |
| 1.249 | OH | $OCH_3$ | CH | 3-$OCH_3$ | 4-Cl | H | H | |
| 1.250 | OH | $OCH_3$ | CH | 6-$OCH_3$ | 4-Cl | H | H | |
| 1.251 | OH | $OCH_3$ | CH | 5-OCH3 | 4-Cl | H | H | |
| 1.252 | OH | $OCH_3$ | CH | 3-Methyl | 6-Cl | H | H | |
| 1.253 | OH | $OCH_3$ | CH | 4-Methyl | 6-Cl | H | H | |
| 1.254 | OH | $OCH_3$ | CH | 5-Methyl | 6-Cl | H | H | |
| 1.255 | OH | $OCH_3$ | CH | 3-Methyl | 4-Cl | H | H | |
| 1.256 | OH | $OCH_3$ | CH | 6-Methyl | 4-Cl | H | H | |
| 1.257 | OH | $OCH_3$ | CH | 5-Methyl | 4-Cl | H | H | |
| 1.258 | OH | $OCH_3$ | CH | 3-$OCH_3$ | 6-$OCH_3$ | H | H | |
| 1.259 | OH | $OCH_3$ | CH | 4-$OCH_3$ | 6-$OCH_3$ | H | H | |
| 1.260 | OH | $OCH_3$ | CH | 5-$OCH_3$ | 6-$OCH_3$ | H | H | |
| 1.261 | 2-Propaniminoxy | $OCH_3$ | CH | H | H | H | H | |

Tabelle 2

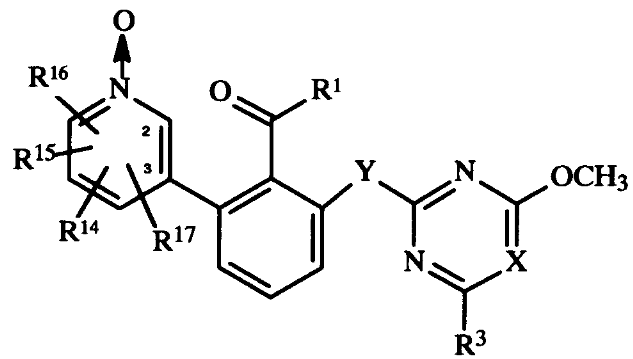

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.001 | OH | O | $OCH_3$ | CH | 2-$OCH_3$ | H | H | H | |
| 2.002 | OH | O | $OCH_3$ | CH | 4-$OCH_3$ | H | H | H | |
| 2.003 | OH | O | $OCH_3$ | CH | 5-$OCH_3$ | H | H | H | |
| 2.004 | OH | O | $OCH_3$ | CH | 6-$OCH_3$ | H | H | H | |
| 2.005 | 2-Propaniminoxy | O | $OCH_3$ | CH | 2-$OCH_3$ | H | H | H | |
| 2.006 | 2-Propaniminoxy | O | $OCH_3$ | CH | 4-$OCH_3$ | H | H | H | |
| 2.007 | 2-Propaniminoxy | O | $OCH_3$ | CH | 5-$OCH_3$ | H | H | H | |
| 2.008 | 2-Propaniminoxy | O | $OCH_3$ | CH | 6-$OCH_3$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.009 | Benzyloxy | O | $OCH_3$ | CH | 2-$OCH_3$ | H | H | H | |
| 2.010 | Benzyloxy | O | $OCH_3$ | CH | 4-$OCH_3$ | H | H | H | |
| 2.011 | Benzyloxy | O | $OCH_3$ | CH | 5-$OCH_3$ | H | H | H | |
| 2.012 | Benzyloxy | O | $OCH_3$ | CH | 6-OCH3 | H | H | H | |
| 2.013 | OH | O | $OCH_3$ | CH | 2-$OC_2H_5$ | H | H | H | |
| 2.014 | OH | O | $OCH_3$ | CH | 4-$OC_2H_5$ | H | H | H | |
| 2.015 | OH | O | $OCH_3$ | CH | 5-$OC_2H_5$ | H | H | H | |
| 2.016 | OH | O | $OCH_3$ | CH | 6-$OC_2H_5$ | H | H | H | |
| 2.017 | 2-Propaniminoxy | O | $OCH_3$ | CH | 2-$OC_2H_5$ | H | H | H | |
| 2.018 | 2-Propaniminoxy | O | $OCH_3$ | CH | 4-$OC_2H_5$ | H | H | H | |
| 2.019 | 2-Propaniminoxy | O | $OCH_3$ | CH | 5-$OC_2H_5$ | H | H | H | |
| 2.020 | 2-Propaniminoxy | O | $OCH_3$ | CH | 6-$OC_2H_5$ | H | H | H | |
| 2.021 | Benzyloxy | O | $OCH_3$ | CH | 2-$OC_2H_5$ | H | H | H | |
| 2.022 | Benzyloxy | O | $OCH_3$ | CH | 4-$OC_2H_5$ | H | H | H | |
| 2.023 | Benzyloxy | O | $OCH_3$ | CH | 5-$OC_2H_5$ | H | H | H | |
| 2.024 | Benzyloxy | O | $OCH_3$ | CH | 6-$OC_2H_5$ | H | H | H | |
| 2.025 | OH | O | $OCH_3$ | CH | 2-n-$C_3H_7$ | H | H | H | |
| 2.026 | OH | O | $OCH_3$ | CH | 4-n-$C_3H_7$ | H | H | H | |
| 2.027 | OH | O | $OCH_3$ | CH | 5-n-$C_3H_7$ | H | H | H | |
| 2.028 | OH | O | $OCH_3$ | CH | 6-n-$C_3H_7$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.029 | 2-Propaniminoxy | O | $OCH_3$ | CH | 2-n-$C_3H_7$ | H | H | H | |
| 2.030 | 2-Propaniminoxy | O | $OCH_3$ | CH | 4-n-$C_3H_7$ | H | H | H | |
| 2.031 | 2-Propaniminoxy | O | $OCH_3$ | CH | 5-n-$C_3H_7$ | H | H | H | |
| 2.032 | 2-Propaniminoxy | O | $OCH_3$ | CH | 6-n-$C_3H_7$ | H | H | H | |
| 2.033 | Benzyloxy | O | $OCH_3$ | CH | 2-n-$C_3H_7$ | H | H | H | |
| 2.034 | Benzyloxy | O | $OCH_3$ | CH | 4-n-$C_3H_7$ | H | H | H | |
| 2.035 | Benzyloxy | O | $OCH_3$ | CH | 5-n-$C_3H_7$ | H | H | H | |
| 2.036 | Benzyloxy | O | $OCH_3$ | CH | 6-n-$C_3H_7$ | H | H | H | |
| 2.037 | OH | O | $OCH_3$ | CH | 2-i-$C_3H_7$ | H | H | H | |
| 2.038 | OH | O | $OCH_3$ | CH | 4-i-$C_3H_7$ | H | H | H | |
| 2.039 | OH | O | $OCH_3$ | CH | 5-i-$C_3H_7$ | H | H | H | |
| 2.040 | OH | O | $OCH_3$ | CH | 6-i-$C_3H_7$ | H | H | H | |
| 2.041 | 2-Propaniminoxy | O | $OCH_3$ | CH | 2-i-$C_3H_7$ | H | H | H | |
| 2.042 | 2-Propaniminoxy | O | $OCH_3$ | CH | 4-i-$C_3H_7$ | H | H | H | |
| 2.043 | 2-Propaniminoxy | O | $OCH_3$ | CH | 5-i-$C_3H_7$ | H | H | H | |
| 2.044 | 2-Propaniminoxy | O | $OCH_3$ | CH | 6-i-$C_3H_7$ | H | H | H | |
| 2.045 | Benzyloxy | O | $OCH_3$ | CH | 2-i-$C_3H_7$ | H | H | H | |
| 2.046 | Benzyloxy | O | $OCH_3$ | CH | 4-i-$C_3H_7$ | H | H | H | |
| 2.047 | Benzyloxy | O | $OCH_3$ | CH | 5-i-$C_3H_7$ | H | H | H | |
| 2.048 | Benzyloxy | O | $OCH_3$ | CH | 6-i-$C_3H_7$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.049 | OH | O | $OCH_3$ | CH | 2-$SCH_3$ | H | H | H | |
| 2.050 | OH | O | $OCH_3$ | CH | 4-$SCH_3$ | H | H | H | |
| 2.051 | OH | O | $OCH_3$ | CH | 5-$SCH_3$ | H | H | H | |
| 2.052 | OH | O | $OCH_3$ | CH | 6-$SCH_3$ | H | H | H | |
| 2.053 | 2-Propaniminoxy | O | $OCH_3$ | CH | 2-$SCH_3$ | H | H | H | |
| 2.054 | 2-Propaniminoxy | O | $OCH_3$ | CH | 4-$SCH_3$ | H | H | H | |
| 2.055 | 2-Propaniminoxy | O | $OCH_3$ | CH | 5-$SCH_3$ | H | H | H | |
| 2.056 | 2-Propaniminoxy | O | $OCH_3$ | CH | 6-$SCH_3$ | H | H | H | |
| 2.057 | Benzyloxy | O | $OCH_3$ | CH | 2-$SCH_3$ | H | H | H | |
| 2.058 | Benzyloxy | O | $OCH_3$ | CH | 4-$SCH_3$ | H | H | H | |
| 2.059 | Benzyloxy | O | $OCH_3$ | CH | 5-$SCH_3$ | H | H | H | |
| 2.060 | Benzyloxy | O | $OCH_3$ | CH | 6-$SCH_3$ | H | H | H | |
| 2.061 | OH | O | $OCH_3$ | CH | 2-$SC_2H_5$ | H | H | H | |
| 2.062 | OH | O | $OCH_3$ | CH | 4-$SC_2H_5$ | H | H | H | |
| 2.063 | OH | O | $OCH_3$ | CH | 5-$SC_2H_5$ | H | H | H | |
| 2.064 | OH | O | $OCH_3$ | CH | 6-$SC_2H_5$ | H | H | H | |
| 2.065 | 2-Propaniminoxy | O | $OCH_3$ | CH | 2-$SC_2H_5$ | H | H | H | |
| 2.066 | 2-Propaniminoxy | O | $OCH_3$ | CH | 4-$SC_2H_5$ | H | H | H | |
| 2.067 | 2-Propaniminoxy | O | $OCH_3$ | CH | 5-$SC_2H_5$ | H | H | H | |
| 2.068 | 2-Propaniminoxy | O | $OCH_3$ | CH | 6-$SC_2H_5$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.069 | Benzyloxy | O | $OCH_3$ | CH | 2-$SC_2H_5$ | H | H | H | |
| 2.070 | Benzyloxy | O | $OCH_3$ | CH | 4-$SC_2H_5$ | H | H | H | |
| 2.071 | Benzyloxy | O | $OCH_3$ | CH | 5-$SC_2H_5$ | H | H | H | |
| 2.072 | Benzyloxy | O | $OCH_3$ | CH | 6-$SC_2H_5$ | H | H | H | |
| 2.073 | OH | O | $OCH_3$ | CH | 2-S-n-$C_3H_7$ | H | H | H | |
| 2.074 | OH | O | $OCH_3$ | CH | 4-S-n-$C_3H_7$ | H | H | H | |
| 2.075 | OH | O | $OCH_3$ | CH | 5-S-n-$C_3H_7$ | H | H | H | |
| 2.076 | OH | O | $OCH_3$ | CH | 6-S-n-$C_3H_7$ | H | H | H | |
| 2.077 | 2-Propaniminoxy | O | $OCH_3$ | CH | 2-S-n-$C_3H_7$ | H | H | H | |
| 2.078 | 2-Propaniminoxy | O | $OCH_3$ | CH | 4-S-n-$C_3H_7$ | H | H | H | |
| 2.079 | 2-Propaniminoxy | O | $OCH_3$ | CH | 5-S-n-$C_3H_7$ | H | H | H | |
| 2.080 | 2-Propaniminoxy | O | $OCH_3$ | CH | 6-S-n-$C_3H_7$ | H | H | H | |
| 2.081 | Benzyloxy | O | $OCH_3$ | CH | 2-S-n-$C_3H_7$ | H | H | H | |
| 2.082 | Benzyloxy | O | $OCH_3$ | CH | 4-S-n-$C_3H_7$ | H | H | H | |
| 2.083 | Benzyloxy | O | $OCH_3$ | CH | 5-S-n-$C_3H_7$ | H | H | H | |
| 2.084 | Benzyloxy | O | $OCH_3$ | CH | 6-S-n-$C_3H_7$ | H | H | H | |
| 2.085 | OH | O | $OCH_3$ | CH | 2-S-i-$C_3H_7$ | H | H | H | |
| 2.086 | OH | O | $OCH_3$ | CH | 4-S-i-$C_3H_7$ | H | H | H | |
| 2.087 | OH | O | $OCH_3$ | CH | 5-S-i-$C_3H_7$ | H | H | H | |
| 2.088 | OH | O | $OCH_3$ | CH | 6-S-i-$C_3H_7$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.089 | 2-Propaniminoxy | O | $OCH_3$ | CH | 2-S-i-$C_3H_7$ | H | H | H | |
| 2.090 | 2-Propaniminoxy | O | $OCH_3$ | CH | 4-S-i-$C_3H_7$ | H | H | H | |
| 2.091 | 2-Propaniminoxy | O | $OCH_3$ | CH | 5-S-i-$C_3H_7$ | H | H | H | |
| 2.092 | 2-Propaniminoxy | O | $OCH_3$ | CH | 6-S-i-$C_3H_7$ | H | H | H | |
| 2.093 | Benzyloxy | O | $OCH_3$ | CH | 2-S-i-$C_3H_7$ | H | H | H | |
| 2.094 | Benzyloxy | O | $OCH_3$ | CH | 4-S-i-$C_3H_7$ | H | H | H | |
| 2.095 | Benzyloxy | O | $OCH_3$ | CH | 5-S-i-$C_3H_7$ | H | H | H | |
| 2.096 | Benzyloxy | O | $OCH_3$ | CH | 6-S-i-$C_3H_7$ | H | H | H | |
| 2.097 | OH | O | $OCH_3$ | CH | 6-$OCH_2CF_3$ | H | H | H | |
| 2.098 | 2-Propaniminoxy | O | $OCH_3$ | CH | 6-$OCH_2CF_3$ | H | H | H | |
| 2.099 | Benzyloxy | O | $OCH_3$ | CH | 6-$OCH_2CF_3$ | H | H | H | |
| 2.100 | OH | O | $OCH_3$ | CH | 6-$OCH_2CH_2OCH_3$ | H | H | H | |
| 2.101 | 2-Propaniminoxy | O | $OCH_3$ | CH | 6-$OCH_2CH_2OCH_3$ | H | H | H | |
| 2.102 | Benzyloxy | O | $OCH_3$ | CH | 6-$OCH_2CH_2OCH_3$ | H | H | H | |
| 2.103 | OH | O | $OCH_3$ | CH | 6-$OCH_2CH_2N(CH_3)_2$ | | H | H | |
| 2.104 | 2-Propaniminoxy | O | $OCH_3$ | CH | 6-$OCH_2CH_2N(CH_3)_2$ | | H | H | |
| 2.105 | Benzyloxy | O | $OCH_3$ | CH | 6-$OCH_2CH_2N(CH_3)_2$ | | H | H | |
| 2.106 | OH | O | $OCH_3$ | CH | 6-$ON(CH_3)_2$ | H | H | H | |
| 2.107 | 2-Propaniminoxy | O | $OCH_3$ | CH | 6-$ON(CH_3)_2$ | H | H | H | |
| 2.108 | Benzyloxy | O | $OCH_3$ | CH | 6-$ON(CH_3)_2$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.109 | $OCH_2CF_3$ | O | $OCH_3$ | CH | 2-$OCH_3$ | H | H | H | |
| 2.110 | $OCH_2CF_3$ | O | $OCH_3$ | CH | 4-$OCH_3$ | H | H | H | |
| 2.111 | $OCH_2CF_3$ | O | $OCH_3$ | CH | 5-$OCH_3$ | H | H | H | |
| 2.112 | $OCH_2CF_3$ | O | $OCH_3$ | CH | 6-$OCH_3$ | H | H | H | |
| 2.113 | Propargyloxy | O | $OCH_3$ | CH | 2-$OCH_3$ | H | H | H | |
| 2.114 | Propargyloxy | O | $OCH_3$ | CH | 4-$OCH_3$ | H | H | H | |
| 2.115 | Propargyloxy | O | $OCH_3$ | CH | 5-$OCH_3$ | H | H | H | |
| 2.116 | Propargyloxy | O | $OCH_3$ | CH | 6-$OCH_3$ | H | H | H | |
| 2.117 | Allyloxy | O | $OCH_3$ | CH | 2-$OCH_3$ | H | H | H | |
| 2.118 | Allyloxy | O | $OCH_3$ | CH | 4-$OCH_3$ | H | H | H | |
| 2.119 | Allyloxy | O | $OCH_3$ | CH | 5-$OCH_3$ | H | H | H | |
| 2.120 | Allyloxy | O | $OCH_3$ | CH | 6-$OCH_3$ | H | H | H | |
| 2.121 | OH | O | $OCH_2CH_2$ | | 2-$OCH_3$ | H | H | H | |
| 2.122 | OH | O | $OCH_2CH_2$ | | 4-$OCH_3$ | H | H | H | |
| 2.123 | OH | O | $OCH_2CH_2$ | | 5-$OCH_3$ | H | H | H | |
| 2.124 | OH | O | $OCH_2CH_2$ | | 6-$OCH_3$ | H | H | H | |
| 2.125 | OH | O | $OCH_3$ | CF | 2-$OCH_3$ | H | H | H | |
| 2.126 | OH | O | $OCH_3$ | CF | 4-$OCH_3$ | H | H | H | |
| 2.127 | OH | O | $OCH_3$ | CF | 5-$OCH_3$ | H | H | H | |
| 2.128 | OH | O | $OCH_3$ | CF | 6-$OCH_3$ | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.129 | OH | O | $OCH_3$ | CH | 2-$OCH_3$ | 4-$CH_3$ | H | H | |
| 2.130 | OH | O | $OCH_3$ | CH | 2-$OCH_3$ | 5-$CH_3$ | H | H | |
| 2.131 | OH | O | $OCH_3$ | CH | 2-$OCH_3$ | 6-$CH_3$ | H | H | |
| 2.132 | OH | O | $OCH_3$ | CH | 4-$OCH_3$ | 2-$CH_3$ | H | H | |
| 2.133 | OH | O | $OCH_3$ | CH | 4-$OCH_3$ | 5-$CH_3$ | H | H | |
| 2.134 | OH | O | $OCH_3$ | CH | 4-$OCH_3$ | 6-$CH_3$ | H | H | |
| 2.135 | OH | O | $OCH_3$ | CH | 5-$OCH_3$ | 4-$CH_3$ | H | H | |
| 2.136 | OH | O | $OCH_3$ | CH | 5-$OCH_3$ | 2-$CH_3$ | H | H | |
| 2.137 | OH | O | $OCH_3$ | CH | 5-$OCH_3$ | 6-$CH_3$ | H | H | |
| 2.138 | OH | O | $OCH_3$ | CH | 6-$OCH_3$ | 4-$CH_3$ | H | H | |
| 2.139 | OH | O | $OCH_3$ | CH | 6-$OCH_3$ | 5-$CH_3$ | H | H | |
| 2.140 | OH | O | $OCH_3$ | CH | 6-$OCH_3$ | 2-$CH_3$ | H | H | |
| 2.141 | OH | O | $OCH_3$ | CH | 2-$SCH_3$ | 4-$CH_3$ | H | H | |
| 2.142 | OH | O | $OCH_3$ | CH | 2-$SCH_3$ | 5-$CH_3$ | H | H | |
| 2.143 | OH | O | $OCH_3$ | CH | 2-$SCH_3$ | 6-$CH_3$ | H | H | |
| 2.144 | OH | O | $OCH_3$ | CH | 4-$SCH_3$ | 2-$CH_3$ | H | H | |
| 2.145 | OH | O | $OCH_3$ | CH | 4-$SCH_3$ | 5-$CH_3$ | H | H | |
| 2.146 | OH | O | $OCH_3$ | CH | 4-$SCH_3$ | 6-$CH_3$ | H | H | |
| 2.147 | OH | O | $OCH_3$ | CH | 5-$SCH_3$ | 4-$CH_3$ | H | H | |
| 2.148 | OH | O | $OCH_3$ | CH | 5-$SCH_3$ | 2-$CH_3$ | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.149 | OH | O | $OCH_3$ | CH | 5-$SCH_3$ | 6-$CH_3$ | H | H | |
| 2.150 | OH | O | $OCH_3$ | CH | 6-$SCH_3$ | 4-$CH_3$ | H | H | |
| 2.151 | OH | O | $CH_3$ | CH | 6-$SCH_3$ | 5-$CH_3$ | H | H | |
| 2.152 | OH | O | $CH_3$ | CH | 6-$SCH_3$ | 2-$CH_3$ | H | H | |
| 2.153 | OH | O | $OCH_3$ | CH | 2-Methyl | H | H | H | |
| 2.154 | OH | O | $OCH_3$ | CH | 4-Methyl | H | H | H | |
| 2.155 | OH | O | $OCH_3$ | CH | 5-Methyl | H | H | H | |
| 2.156 | OH | O | $OCH_3$ | CH | 6-Methyl | H | H | H | |
| 2.157 | OH | O | $OCH_3$ | CH | 2-Ethyl | H | H | H | |
| 2.158 | OH | O | $OCH_3$ | CH | 4-Ethyl | H | H | H | |
| 2.159 | OH | O | $OCH_3$ | CH | 5-Ethyl | H | H | H | |
| 2.160 | OH | O | $OCH_3$ | CH | 6-Ethyl | H | H | H | |
| 2.161 | OH | O | $OCH_3$ | CH | 2-$CF_3$ | H | H | H | |
| 2.162 | OH | O | $OCH_3$ | CH | 4-$CF_3$ | H | H | H | |
| 2.163 | OH | O | $OCH_3$ | CH | 5-$CF_3$ | H | H | H | |
| 2.164 | OH | O | $OCH_3$ | CH | 6-$CF_3$ | H | H | H | |
| 2.165 | OH | O | $OCH_3$ | CH | 2-Cl | H | H | H | |
| 2.166 | OH | O | $OCH_3$ | CH | 4-Cl | H | H | H | |
| 2.167 | OH | O | $OCH_3$ | CH | 5-Cl | H | H | H | |
| 2.168 | OH | O | $OCH_3$ | CH | 6-Cl | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.169 | OH | O | $OCH_3$ | CH | 2-F | H | H | H | |
| 2.170 | OH | O | $OCH_3$ | CH | 4-F | H | H | H | |
| 2.171 | OH | O | $OCH_3$ | CH | 5-F | H | H | H | |
| 2.172 | OH | O | $OCH_3$ | CH | 6-F | H | H | H | |
| 2.173 | OH | O | $OCH_3$ | CH | 2-O-i-$C_3H_7$ | H | H | H | |
| 2.174 | OH | O | $OCH_3$ | CH | 4-O-i-$C_3H_7$ | H | H | H | |
| 2.175 | OH | O | $OCH_3$ | CH | 5-O-i-$C_3H_7$ | H | H | H | |
| 2.176 | OH | O | $OCH_3$ | CH | 6-O-i-$C_3H_7$ | H | H | H | |
| 2.177 | OH | O | $OCH_3$ | CH | 2-$CF_3$ | 6-$OCH_3$ | H | H | |
| 2.178 | OH | O | $OCH_3$ | CH | 4-$CF_3$ | 6-$OCH_3$ | H | H | |
| 2.179 | OH | O | $OCH_3$ | CH | 5-$CF_3$ | 2-Cl | H | H | |
| 2.180 | OH | O | $OCH_3$ | CH | 6-$CF_3$ | 2-Cl | 4-F | H | |
| 2.181 | 2-Propaniminoxy | O | $OCH_3$ | CH | 2-Methyl | H | H | H | |
| 2.182 | 2-Propaniminoxy | O | $OCH_3$ | CH | 4-Methyl | H | H | H | |
| 2.183 | 2-Propaniminoxy | O | $OCH_3$ | CH | 5-Methyl | H | H | H | |
| 2.184 | 2-Propaniminoxy | O | $OCH_3$ | CH | 6-Methyl | H | H | H | |
| 2.185 | 2-Propaniminoxy | O | $OCH_3$ | CH | 2-Ethyl | H | H | H | |
| 2.186 | 2-Propaniminoxy | O | $OCH_3$ | CH | 4-Ethyl | H | H | H | |
| 2.187 | 2-Propaniminoxy | O | $OCH_3$ | CH | 5-Ethyl | H | H | H | |
| 2.188 | 2-Propaniminoxy | O | $OCH_3$ | CH | 6-Ethyl | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.189 | 2-Propaniminoxy | O | $OCH_3$ | CH | 2-$CF_3$ | H | H | H | |
| 2.190 | 2-Propaniminoxy | O | $OCH_3$ | CH | 4-$CF_3$ | H | H | H | |
| 2.191 | 2-Propaniminoxy | O | $OCH_3$ | CH | 5-$CF_3$ | H | H | H | |
| 2.192 | 2-Propaniminoxy | O | $OCH_3$ | CH | 6-$CF_3$ | H | H | H | |
| 2.193 | 2-Propaniminoxy | O | $OCH_3$ | CH | 2-Cl | H | H | H | |
| 2.194 | 2-Propaniminoxy | O | $OCH_3$ | CH | 4-Cl | H | H | H | |
| 2.195 | 2-Propaniminoxy | O | $OCH_3$ | CH | 5-Cl | H | H | H | |
| 2.196 | 2-Propaniminoxy | O | $OCH_3$ | CH | 6-Cl | H | H | H | |
| 2.197 | 2-Propaniminoxy | O | $OCH_3$ | CH | 2-F | H | H | H | |
| 2.198 | 2-Propaniminoxy | O | $OCH_3$ | CH | 4-F | H | H | H | |
| 2.199 | 2-Propaniminoxy | O | $OCH_3$ | CH | 5-F | H | H | H | |
| 2.200 | 2-Propaniminoxy | O | $OCH_3$ | CH | 6-F | H | H | H | |
| 2.201 | 2-Propaniminoxy | O | $OCH_3$ | CH | 2-O-i-$C_3H_7$ | H | H | H | |
| 2.202 | 2-Propaniminoxy | O | $OCH_3$ | CH | 4-O-i-$C_3H_7$ | H | H | H | |
| 2.203 | 2-Propaniminoxy | O | $OCH_3$ | CH | 5-O-i-$C_3H_7$ | H | H | H | |
| 2.204 | 2-Propaniminoxy | O | $OCH_3$ | CH | 6-O-i-$C_3H_7$ | H | H | H | |
| 2.205 | 2-Propaniminoxy | O | $OCH_3$ | CH | 2-$CF_3$ | 6-$OCH_3$ | H | H | |
| 2.206 | 2-Propaniminoxy | O | $OCH_3$ | CH | 4-$CF_3$ | 6-$OCH_3$ | H | H | |
| 2.207 | 2-Propaniminoxy | O | $OCH_3$ | CH | 5-$CF_3$ | 2-Cl | H | H | |
| 2.208 | 2-Propaniminoxy | O | $OCH_3$ | CH | 6-$CF_3$ | 2-Cl | 4-F | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.209 | $OCH_2CF_3$ | O | $OCH_3$ | CH | 2-Methyl | H | H | H | |
| 2.210 | $OCH_2CF_3$ | O | $OCH_3$ | CH | 4-Methyl | H | H | H | |
| 2.211 | $OCH_2CF_3$ | O | $OCH_3$ | CH | 5-Methyl | H | H | H | |
| 2.212 | $OCH_2CF_3$ | O | $OCH_3$ | CH | 6-Methyl | H | H | H | |
| 2.213 | $OCH_2CF_3$ | O | $OCH_3$ | CH | 2-Ethyl | H | H | H | |
| 2.214 | $OCH_2CF_3$ | O | $OCH_3$ | CH | 4-Ethyl | H | H | H | |
| 2.215 | $OCH_2CF_3$ | O | $OCH_3$ | CH | 5-Ethyl | H | H | H | |
| 2.216 | $OCH_2CF_3$ | O | $OCH_3$ | CH | 6-Ethyl | H | H | H | |
| 2.217 | $OCH_2CF_3$ | O | $OCH_3$ | CH | 2-$CF_3$ | H | H | H | |
| 2.218 | $OCH_2CF_3$ | O | $OCH_3$ | CH | 4-$CF_3$ | H | H | H | |
| 2.219 | $OCH_2CF_3$ | O | $OCH_3$ | CH | 5-$CF_3$ | H | H | H | |
| 2.220 | $OCH_2CF_3$ | O | $OCH_3$ | CH | 6-$CF_3$ | H | H | H | |
| 2.221 | $OCH_2CF_3$ | O | $OCH_3$ | CH | 2-Cl | H | H | H | |
| 2.222 | $OCH_2CF_3$ | O | $OCH_3$ | CH | 4-Cl | H | H | H | |
| 2.223 | $OCH_2CF_3$ | O | $OCH_3$ | CH | 5-Cl | H | H | H | |
| 2.224 | $OCH_2CF_3$ | O | $OCH_3$ | CH | 6-Cl | H | H | H | |
| 2.225 | $OCH_2CF_3$ | O | $OCH_3$ | CH | 2-F | H | H | H | |
| 2.226 | $OCH_2CF_3$ | O | $OCH_3$ | CH | 4-F | H | H | H | |
| 2.227 | $OCH_2CF_3$ | O | $OCH_3$ | CH | 5-F | H | H | H | |
| 2.228 | $OCH_2CF_3$ | O | $OCH_3$ | CH | 6-F | H | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.229 | $OCH_2CF_3$ | O | $OCH_3$ | CH | 2-O-i-$C_3H_7$ | H | H | H | |
| 2.230 | $OCH_2CF_3$ | O | $OCH_3$ | CH | 4-O-i-$C_3H_7$ | H | H | H | |
| 2.231 | $OCH_2CF_3$ | O | $OCH_3$ | CH | 5-O-i-$C_3H_7$ | H | H | H | |
| 2.232 | $OCH_2CF_3$ | O | $OCH_3$ | CH | 6-O-i-$C_3H_7$ | H | H | H | |
| 2.233 | $OCH_2CF_3$ | O | $OCH_3$ | CH | 2-$CF_3$ | 6-$OCH_3$ | H | H | |
| 2.234 | $OCH_2CF_3$ | O | $OCH_3$ | CH | 4-$CF_3$ | 6-$OCH_3$ | H | H | |
| 2.235 | $OCH_2CF_3$ | O | $OCH_3$ | CH | 5-$CF_3$ | 2-Cl | H | H | |
| 2.236 | $OCH_2CF_3$ | O | $OCH_3$ | CH | 6-$CF_3$ | 2-Cl | 4-F | H | |
| 2.237 | OH | O | $OCH_3$ | CH | H | H | H | H | |
| 2.238 | $OCH_2SCH_3$ | O | $OCH_3$ | CH | H | H | H | H | |
| 2.239 | $OC_2H_4OCH_3$ | O | $OCH_3$ | CH | H | H | H | H | |
| 2.240 | $OCH(CH_3)COOC_2H_5$ | O | $OCH_3$ | CH | H | H | H | H | |
| 2.241 | $OCH_2OCH_3$ | O | $OCH_3$ | CH | H | H | H | H | |
| 2.242 | Cyclohexaniminoxy | O | $OCH_3$ | CH | H | H | H | H | |
| 2.243 | Cyclopentaniminoxy | O | $OCH_3$ | CH | H | H | H | H | |
| 2.244 | Allyloxy | O | $OCH_3$ | CH | H | H | H | H | |
| 2.245 | Propargyloxy | O | $OCH_3$ | CH | H | H | H | H | |
| 2.246 | OH | O | $OCH_3$ | CH | 2-$OCH_3$ | 6-Cl | H | H | |
| 2.247 | OH | O | $OCH_3$ | CH | 4-$OCH_3$ | 6-Cl | H | H | |
| 2.248 | OH | O | $OCH_3$ | CH | 5-$OCH_3$ | 6-Cl | H | H | |

| Nr. | $R^1$ | Y | $R^3$ | X | $R^{14}$ | $R^{15}$ | $R^{16}$ | $R^{17}$ | Phys. Daten $^1H$-NMR ($CDCl_3$) |
|---|---|---|---|---|---|---|---|---|---|
| 2.249 | OH | O | $OCH_3$ | CH | 2-$OCH_3$ | 4-Cl | H | H | |
| 2.250 | OH | O | $OCH_3$ | CH | 6-$OCH_3$ | 4-Cl | H | H | |
| 2.251 | OH | O | $OCH_3$ | CH | 5-$OCH_3$ | 4-Cl | H | H | |
| 2.252 | OH | O | $OCH_3$ | CH | 2-Methyl | 6-Cl | H | H | |
| 2.253 | OH | O | $OCH_3$ | CH | 4-Methyl | 6-Cl | H | H | |
| 2.254 | OH | O | $OCH_3$ | CH | 5-Methyl | 6-Cl | H | H | |
| 2.255 | OH | O | $OCH_3$ | CH | 2-Methyl | 4-Cl | H | H | |
| 2.256 | OH | O | $OCH_3$ | CH | 6-Methyl | 4-Cl | H | H | |
| 2.257 | OH | O | $OCH_3$ | CH | 5-Methyl | 4-Cl | H | H | |
| 2.258 | OH | O | $OCH_3$ | CH | 2-$OCH_3$ | 6-$OCH_3$ | H | H | |
| 2.259 | OH | O | $OCH_3$ | CH | 4-$OCH_3$ | 6-$OCH_3$ | H | H | |
| 2.260 | OH | O | $OCH_3$ | CH | 5-$OCH_3$ | 6-$OCH_3$ | H | H | |
| 2.261 | 2-Propaniminoxy | O | $OCH_3$ | CH | H | H | H | H | |
| 2.262 | 2-Propaniminoxy | O | $OCH_3$ | N | H | H | H | H | δ [ppm]: 1,75(s); 1,95(s); 4,00(s); 7,30(m); 7,62(t); 8,23(m), 8,37(s) |

Tabelle 3

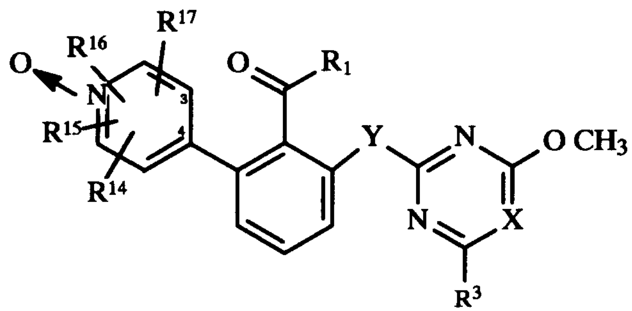

| Nr. | $R^1$ | Y | $R^3$ | X | $R^{14}$ | $R^{15}$ | $R^{16}$ | $R^{17}$ |
|---|---|---|---|---|---|---|---|---|
| 3.001 | OH | O | $OCH_3$ | CH | 3-$OCH_3$ | H | H | H |
| 3.002 | OH | O | $OCH_3$ | CH | 2-$OCH_3$ | H | H | H |
| 3.003 | 1-Imidazolyl | O | $OCH_3$ | CH | 3-$OCH_3$ | H | H | H |
| 3.004 | 1-Imidazolyl | O | $OCH_3$ | CH | 2-$OCH_3$ | H | H | H |
| 3.005 | 2-Propaniminoxy | O | $OCH_3$ | CH | 3-$OCH_3$ | H | H | H |
| 3.006 | 2-Propaniminoxy | O | $OCH_3$ | CH | 2-$OCH_3$ | H | H | H |
| 3.007 | Methylthiomethyl | O | $OCH_3$ | CH | 3-$OCH_3$ | H | H | H |
| 3.008 | Methylthiomethyl | O | $OCH_3$ | CH | 2-$OCH_3$ | H | H | H |
| 3.009 | Benzyloxy | O | $OCH_3$ | CH | 3-$OCH_3$ | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.010 | Benzyloxy | O | $OCH_3$ | CH | 2-$OCH_3$ | H | H | H |
| 3.011 | Cyclohexaniminoxy | O | $OCH_3$ | CH | 3-$OCH_3$ | H | H | H |
| 3.012 | Cyclohexaniminoxy | O | $OCH_3$ | CH | 2-$OCH_3$ | H | H | H |
| 3.013 | OH | O | $OCH_3$ | CH | 3-$OC_2H_5$ | H | H | H |
| 3.014 | OH | O | $OCH_3$ | CH | 2-$OC_2H_5$ | H | H | H |
| 3.015 | 2-Propaniminoxy | O | $OCH_3$ | CH | 3-$OC_2H_5$ | H | H | H |
| 3.016 | 2-Propaniminoxy | O | $OCH_3$ | CH | 2-$OC_2H_5$ | H | H | H |
| 3.017 | Benzyloxy | O | $OCH_3$ | CH | 3-$OC_2H_5$ | H | H | H |
| 3.018 | Benzyloxy | O | $OCH_3$ | CH | 2-$OC_2H_5$ | H | H | H |
| 3.019 | OH | O | $OCH_3$ | CH | 3-n-$C_3H_7$ | H | H | H |
| 3.020 | OH | O | $OCH_3$ | CH | 2-n-$C_3H_7$ | H | H | H |
| 3.021 | 2-Propaniminoxy | O | $OCH_3$ | CH | 3-n-$C_3H_7$ | H | H | H |
| 3.022 | 2-Propaniminoxy | O | $OCH_3$ | CH | 2-n-$C_3H_7$ | H | H | H |
| 3.023 | Benzyloxy | O | $OCH_3$ | CH | 3-n-$C_3H_7$ | H | H | H |
| 3.024 | Benzyloxy | O | $OCH_3$ | CH | 2-n-$C_3H_7$ | H | H | H |
| 3.025 | OH | O | $OCH_3$ | CH | 3-i-$C_3H_7$ | H | H | H |
| 3.026 | OH | O | $OCH_3$ | CH | 2-i-$C_3H_7$ | H | H | H |
| 3.027 | 2-Propaniminoxy | O | $OCH_3$ | CH | 3-i-$C_3H_7$ | H | H | H |
| 3.028 | 2-Propaniminoxy | O | $OCH_3$ | CH | 2-i-$C_3H_7$ | H | H | H |
| 3.029 | Benzyloxy | O | $OCH_3$ | CH | 3-i-$C_3H_7$ | H | H | H |
| 3.030 | Benzyloxy | O | $OCH_3$ | CH | 2-i-$C_3H_7$ | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.031 | OH | O | $OCH_3$ | CH | $3-SCH_3$ | H | H | H |
| 3.032 | OH | O | $OCH_3$ | CH | $2-SCH_3$ | H | H | H |
| 3.033 | 2-Propaniminoxy | O | $OCH_3$ | CH | $3-SCH_3$ | H | H | H |
| 3.034 | 2-Propaniminoxy | O | $OCH_3$ | CH | $2-SCH_3$ | H | H | H |
| 3.035 | Benzyloxy | O | $OCH_3$ | CH | $3-SCH_3$ | H | H | H |
| 3.036 | Benzyloxy | O | $OCH_3$ | CH | $2-SCH_3$ | H | H | H |
| 3.037 | OH | O | $OCH_3$ | CH | $3-SC_2H_5$ | H | H | H |
| 3.038 | OH | O | $OCH_3$ | CH | $2-SC_2H_5$ | H | H | H |
| 3.039 | 2-Propaniminoxy | O | $OCH_3$ | CH | $3-SC_2H_5$ | H | H | H |
| 3.040 | 2-Propaniminoxy | O | $OCH_3$ | CH | $2-SC_2H_5$ | H | H | H |
| 3.041 | Benzyloxy | O | $OCH_3$ | CH | $3-SC_2H_5$ | H | H | H |
| 3.042 | Benzyloxy | O | $OCH_3$ | CH | $2-SC_2H_5$ | H | H | H |
| 3.043 | OH | O | $OCH_3$ | CH | $3-O-n-C_3H_7$ | H | H | H |
| 3.044 | OH | O | $OCH_3$ | CH | $2-O-n-C_3H_7$ | H | H | H |
| 3.045 | 2-Propaniminoxy | O | $OCH_3$ | CH | $3-O-n-C_3H_7$ | H | H | H |
| 3.046 | 2-Propaniminoxy | O | $OCH_3$ | CH | $2-O-n-C_3H_7$ | H | H | H |
| 3.047 | Benzyloxy | O | $OCH_3$ | CH | $3-O-n-C_3H_7$ | H | H | H |
| 3.048 | Benzyloxy | O | $OCH_3$ | CH | $2-O-n-C_3H_7$ | H | H | H |
| 3.049 | OH | O | $OCH_3$ | CH | $3-O-i-C_3H_7$ | H | H | H |
| 3.050 | OH | O | $OCH_3$ | CH | $2-O-i-C_3H_7$ | H | H | H |
| 3.051 | 2-Propaniminoxy | O | $OCH_3$ | CH | $3-O-i-C_3H_7$ | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.052 | 2-Propaniminoxy | O | $OCH_3$ | CH | 2-O-i-$C_3H_7$ | H | H | H |
| 3.053 | Benzyloxy | O | $OCH_3$ | CH | 3-O-i-$C_3H_7$ | H | H | H |
| 3.054 | Benzyloxy | O | $OCH_3$ | CH | 2-O-i-$C_3H_7$ | H | H | H |
| 3.055 | OH | O | $OCH_3$ | CH | 6-$OCH_2CF_3$ | H | H | H |
| 3.056 | 2-Propaniminoxy | O | $OCH_3$ | CH | 6-$OCH_2CF_3$ | H | H | H |
| 3.057 | Benzyloxy | O | $OCH_3$ | CH | 6-$OCH_2CF_3$ | H | H | H |
| 3.058 | OH | O | $OCH_3$ | CH | 6-$OCH_2CH_2OCH_3$ | H | H | H |
| 3.059 | 2-Propaniminoxy | O | $OCH_3$ | CH | 6-$OCH_2CH_2OCH_3$ | H | H | H |
| 3.060 | Benzyloxy | O | $OCH_3$ | CH | 6-$OCH_2CH_2OCH_3$ | H | H | H |
| 3.061 | OH | O | $OCH_3$ | CH | 6-$OCH_2CH_2N(CH_3)_2$ | H | H | H |
| 3.062 | 2-Propaniminoxy | O | $OCH_3$ | CH | 6-$OCH_2CH_2N(CH_3)_2$ | H | H | H |
| 3.063 | Benzyloxy | O | $OCH_3$ | CH | 6-$OCH_2CH_2N(CH_3)_2$ | H | H | H |
| 3.064 | OH | O | $OCH_3$ | CH | 6-$ON(CH_3)_2$ | H | H | H |
| 3.065 | 2-Propaniminoxy | O | $OCH_3$ | CH | 6-$ON(CH_3)_2$ | H | H | H |
| 3.066 | Benzyloxy | O | $OCH_3$ | CH | 6-$ON(CH_3)_2$ | H | H | H |
| 3.067 | $OCH_2CF_3$ | O | $OCH_3$ | CH | 3-$OCH_3$ | H | H | H |
| 3.068 | $OCH_2CF_3$ | O | $OCH_3$ | CH | 2-$OCH_3$ | H | H | H |
| 3.069 | Propargyloxy | O | $OCH_3$ | CH | 3-$OCH_3$ | H | H | H |
| 3.070 | Propargyloxy | O | $OCH_3$ | CH | 2-$OCH_3$ | H | H | H |
| 3.071 | Allyloxy | O | $OCH_3$ | CH | 3-$OCH_3$ | H | H | H |
| 3.072 | Allyloxy | O | $OCH_3$ | CH | 2-$OCH_3$ | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.073 | OH | O | $OCH_2CH_2$ | | 3-$OCH_3$ | H | H | H |
| 3.074 | OH | O | $OCH_2CH_2$ | | 2-$OCH_3$ | H | H | H |
| 3.075 | OH | O | $OCH_3$ | CF | 3-$OCH_3$ | H | H | H |
| 3.076 | OH | O | $OCH_3$ | CF | 2-$OCH_3$ | H | H | H |
| 3.077 | OH | O | $OCH_3$ | CH | 3-$OCH_3$ | 2-$CH_3$ | H | H |
| 3.078 | OH | O | $OCH_3$ | CH | 3-$OCH_3$ | 5-$CH_3$ | H | H |
| 3.079 | OH | O | $OCH_3$ | CH | 3-$OCH_3$ | 6-$CH_3$ | H | H |
| 3.080 | OH | O | $OCH_3$ | CH | 2-$OCH_3$ | 3-$CH_3$ | H | H |
| 3.081 | OH | O | $OCH_3$ | CH | 2-$OCH_3$ | 5-$CH_3$ | H | H |
| 3.082 | OH | O | $OCH_3$ | CH | 2-$OCH_3$ | 6-$CH_3$ | H | H |
| 3.083 | 2-Propaniminoxy | O | $OCH_3$ | CH | 3-$OCH_3$ | 2-$CH_3$ | H | H |
| 3.084 | 2-Propaniminoxy | O | $OCH_3$ | CH | 3-$OCH_3$ | 5-$CH_3$ | H | H |
| 3.085 | 2-Propaniminoxy | O | $OCH_3$ | CH | 3-$OCH_3$ | 6-$CH_3$ | H | H |
| 3.086 | 2-Propaniminoxy | O | $OCH_3$ | CH | 2-$OCH_3$ | 3-$CH_3$ | H | H |
| 3.087 | 2-Propaniminoxy | O | $OCH_3$ | CH | 2-$OCH_3$ | 5-$CH_3$ | H | H |
| 3.088 | 2-Propaniminoxy | O | $OCH_3$ | CH | 2-$OCH_3$ | 6-$CH_3$ | H | H |
| 3.089 | OH | O | $OCH_3$ | CH | 2-Methyl | H | H | H |
| 3.090 | OH | O | $OCH_3$ | CH | 3-Methyl | H | H | H |
| 3.091 | OH | O | $OCH_3$ | CH | 2-Ethyl | H | H | H |
| 3.092 | OH | O | $OCH_3$ | CH | 3-Ethyl | H | H | H |
| 3.093 | OH | O | $OCH_3$ | CH | 2-$CF_3$ | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.094 | OH | O | $OCH_3$ | CH | 3-$CF_3$ | H | H | H |
| 3.095 | OH | O | $OCH_3$ | CH | 2-Cl | H | H | H |
| 3.096 | OH | O | $OCH_3$ | CH | 3-Cl | H | H | H |
| 3.097 | OH | O | $OCH_3$ | CH | 2-F | H | H | H |
| 3.098 | OH | O | $OCH_3$ | CH | 3-F | H | H | H |
| 3.099 | OH | O | $OCH_3$ | CH | 2-S-i-$C_3H_7$ | H | H | H |
| 3.100 | OH | O | $OCH_3$ | CH | 3-S-i-$C_3H_7$ | H | H | H |
| 3.101 | OH | O | $OCH_3$ | CH | 2-$CF_3$ | 6-$OCH_3$ | H | H |
| 3.102 | OH | O | $OCH_3$ | CH | 3-$CF_3$ | 6-$OCH_3$ | H | H |
| 3.103 | OH | O | $OCH_3$ | CH | 5-$CF_3$ | 2-Cl | H | H |
| 3.104 | OH | O | $OCH_3$ | CH | 6-$CF_3$ | 2-Cl | 4-F | H |
| 3.105 | 2-Propaniminoxy | O | $OCH_3$ | CH | 2-Methyl | H | H | H |
| 3.106 | 2-Propaniminoxy | O | $OCH_3$ | CH | 3-Methyl | H | H | H |
| 3.107 | 2-Propaniminoxy | O | $OCH_3$ | CH | 2-Ethyl | H | H | H |
| 3.108 | 2-Propaniminoxy | O | $OCH_3$ | CH | 3-Ethyl | H | H | H |
| 3.109 | 2-Propaniminoxy | O | $OCH_3$ | CH | 2-$CF_3$ | H | H | H |
| 3.110 | 2-Propaniminoxy | O | $OCH_3$ | CH | 3-$CF_3$ | H | H | H |
| 3.111 | 2-Propaniminoxy | O | $OCH_3$ | CH | 2-Cl | H | H | H |
| 3.112 | 2-Propaniminoxy | O | $OCH_3$ | CH | 3-Cl | H | H | H |
| 3.113 | 2-Propaniminoxy | O | $OCH_3$ | CH | 2-F | H | H | H |
| 3.114 | 2-Propaniminoxy | O | $OCH_3$ | CH | 3-F | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.115 | 2-Propaniminoxy | O | $OCH_3$ | CH | 2-O-i-$C_3H_7$ | H | H | H |
| 3.116 | 2-Propaniminoxy | O | $OCH_3$ | CH | 3-O-i-$C_3H_7$ | H | H | H |
| 3.117 | $OCH_2CF_3$ | O | $OCH_3$ | CH | 2-Methyl | H | H | H |
| 3.118 | $OCH_2CF_3$ | O | $OCH_3$ | CH | 3-Methyl | H | H | H |
| 3.119 | $OCH_2CF_3$ | O | $OCH_3$ | CH | 2-Ethyl | H | H | H |
| 3.120 | $OCH_2CF_3$ | O | $OCH_3$ | CH | 3-Ethyl | H | H | H |
| 3.121 | $OCH_2CF_3$ | O | $OCH_3$ | CH | 2-$CF_3$ | H | H | H |
| 3.122 | $OCH_2CF_3$ | O | $OCH_3$ | CH | 3-$CF_3$ | H | H | H |
| 3.123 | $OCH_2CF_3$ | O | $OCH_3$ | CH | 3-Cl | H | H | H |
| 3.124 | $OCH_2CF_3$ | O | $OCH_3$ | CH | 2-Cl | H | H | H |
| 3.125 | $OCH_2CF_3$ | O | $OCH_3$ | CH | 2-F | H | H | H |
| 3.126 | $OCH_2CF_3$ | O | $OCH_3$ | CH | 3-F | H | H | H |
| 3.127 | $OCH_2CF_3$ | O | $OCH_3$ | CH | 2-O-i-$C_3H_7$ | H | H | H |
| 3.128 | $OCH_2CF_3$ | O | $OCH_3$ | CH | 3-O-i-$C_3H_7$ | H | H | H |
| 3.129 | OH | O | $OCH_3$ | CH | H | H | H | H |
| 3.130 | $OCH_2SCH_3$ | O | $OCH_3$ | CH | H | H | H | H |
| 3.131 | $OC_2H_4OCH_3$ | O | $OCH_3$ | CH | H | H | H | H |
| 3.132 | $OCH(CH_3)COOC_2H_5$ | O | $OCH_3$ | CH | H | H | H | H |
| 3.133 | $OCH_2OCH_3$ | O | $OCH_3$ | CH | H | H | H | H |
| 3.134 | Cyclohexaniminoxy | O | $OCH_3$ | CH | H | H | H | H |
| 3.135 | Cyclopentaniminoxy | O | $OCH_3$ | CH | H | H | H | H |

| Nr. | $R^1$ | Y | $R^3$ | X | $R^{14}$ | $R^{15}$ | $R^{16}$ | $R^{17}$ |
|---|---|---|---|---|---|---|---|---|
| 3.136 | Allyloxy | O | $OCH_3$ | CH | H | H | H | H |
| 3.137 | Propargyloxy | O | $OCH_3$ | CH | H | H | H | H |
| 3.138 | OH | O | $OCH_3$ | CH | 2-$OCH_3$ | 6-Cl | H | H |
| 3.139 | OH | O | $OCH_3$ | CH | 3-$OCH_3$ | 6-Cl | H | H |
| 3.140 | OH | O | $OCH_3$ | CH | 5-$OCH_3$ | 6-Cl | H | H |
| 3.141 | OH | O | $OCH_3$ | CH | 2-$OCH_3$ | 3-Cl | H | H |
| 3.142 | OH | O | $OCH_3$ | CH | 6-$OCH_3$ | 3-Cl | H | H |
| 3.143 | OH | O | $OCH_3$ | CH | 5-$OCH_3$ | 3-Cl | H | H |
| 3.144 | OH | O | $OCH_3$ | CH | 2-Methyl | 6-Cl | H | H |
| 3.145 | OH | O | $OCH_3$ | CH | 3-Methyl | 6-Cl | H | H |
| 3.146 | OH | O | $OCH_3$ | CH | 5-Methyl | 6-Cl | H | H |
| 3.147 | OH | O | $OCH_3$ | CH | 2-Methyl | 3-Cl | H | H |
| 3.148 | OH | O | $OCH_3$ | CH | 6-Methyl | 3-Cl | H | H |
| 3.149 | OH | O | $OCH_3$ | CH | 5-Methyl | 3-Cl | H | H |
| 3.150 | OH | O | $OCH_3$ | CH | 2-$OCH_3$ | 6-$OCH_3$ | H | H |
| 3.151 | OH | O | $OCH_3$ | CH | 3-$OCH_3$ | 6-$OCH_3$ | H | H |
| 3.152 | OH | O | $OCH_3$ | CH | 5-$OCH_3$ | 6-$OCH_3$ | H | H |
| 3.153 | 2-Propaniminoxy | $OCH_3$ | CH | H | H | H | H | H |

Tabelle 4

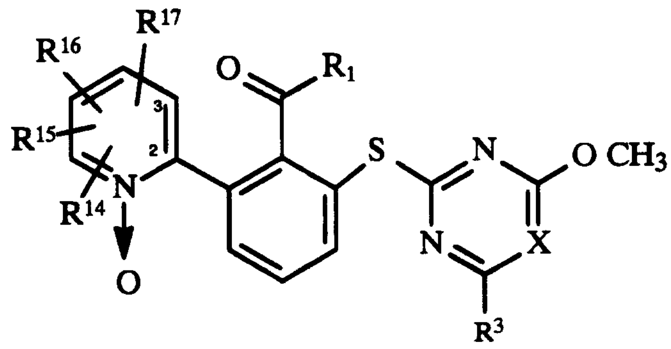

Verbindungen der o.g. Formel, in denen die Kombination der Substituenten $R^1$, $R^3$, X und $R^{14}$-$R^{17}$ für eine Verbindung jeweils eine Zeile der Tabelle 1 entspricht. D.h. Gegenstand der Tabelle 4 sind Verbindungen der Nummern 4.001 bis 4.261, wobei Nr. 4.001 der Nr. 1.001 aus Tabelle 1, jedoch mit Y=S statt O entspricht.

Tabelle 5

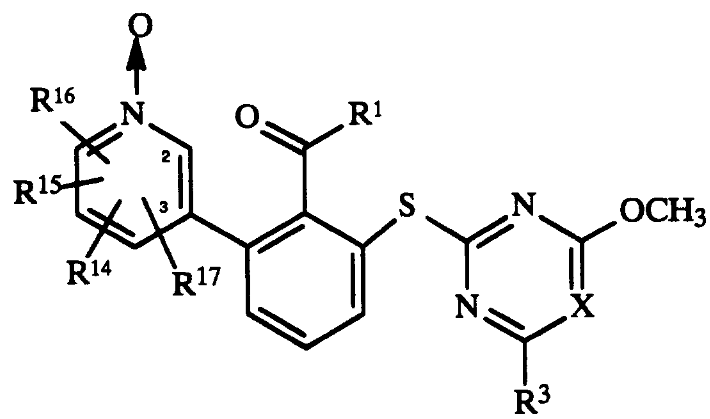

Verbindungen der o.g. Formel, in denen die Kombination der Substituenten $R^1$, $R^3$, X und $R^{14}$-$R^{17}$ für eine Verbindung jeweils eine Zeile der Tabelle 2 entspricht. D.h. Gegenstand der Tabelle 5 sind Verbindungen der Nummern 5.001 bis 5.261, wobei Nr. 5.001 der Nr. 2.001 aus Tabelle 2, jedoch mit Y=S statt O entspricht.

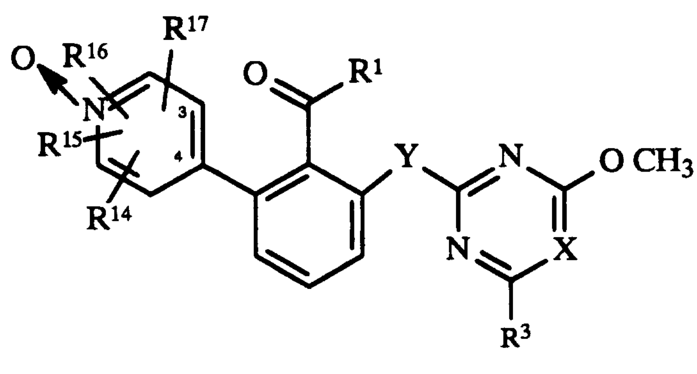

Verbindungen der o.g. Formel, in denen die Kombination der Substituenten $R^1$, $R^3$, X und $R^{14}$-$R^{17}$ für eine Verbindung jeweils eine Zeile der Tabelle 3 entspricht. D.h. Gegenstand der Tabelle 6 sind Verbindungen

der Nummern 6.001 bis 6.153, wobei Nr. 6.001 der Nr. 3.001 aus Tabelle 3, jedoch mit Y=S statt O entspricht.

Zwischenprodukte der Formel IV sind in nachfolgender Tabelle 7 zusammengestellt.

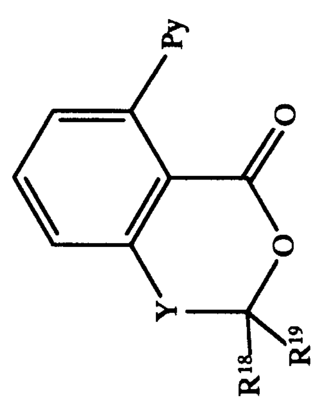

IV

Tabelle 7

| Nr. | Py | $R^{18}$ | $R^{19}$ | Y | phys. Daten |
|---|---|---|---|---|---|
| 7.001 | 1-Oxopyridin-2-yl | $CH_3$ | $CH_3$ | O | |
| 7.002 | 1-Oxopyridin-2-yl | $CH_3$ | $CH_3$ | S | |
| 7.003 | 1-Oxopyridin-3-yl | $CH_3$ | $CH_3$ | O | |
| 7.004 | 1-Oxopyridin-3-yl | $CH_3$ | $CH_3$ | S | |
| 7.005 | 1-Oxopyridin-4-yl | $CH_3$ | $CH_3$ | O | |
| 7.006 | 1-Oxopyridin-4-yl | $CH_3$ | $CH_3$ | S | |
| 7.007 | 3-Methoxy-1-oxopyridin-2-yl | $CH_3$ | $CH_3$ | O | |
| 7.008 | 3-Methoxy-1-oxopyridin-2-yl | $CH_3$ | $CH_3$ | S | |
| 7.009 | 4-Methoxy-1-oxopyridin-2-yl | $CH_3$ | $CH_3$ | O | |

| Nr. | Py | $R^{18}$ | $R^{19}$ | Y | phys. Daten |
|---|---|---|---|---|---|
| 7.010 | 4-Methoxy-1-oxopyridin-2-yl | $CH_3$ | $CH_3$ | S | |
| 7.011 | 5-Methoxy-1-oxopyridin-2-yl | $CH_3$ | $CH_3$ | O | |
| 7.012 | 5-Methoxy-1-oxopyridin-2-yl | $CH_3$ | $CH_3$ | S | |
| 7.013 | 6-Methoxy-1-oxopyridin-2-yl | $CH_3$ | $CH_3$ | O | |
| 7.014 | 6-Methoxy-1-oxopyridin-2-yl | $CH_3$ | $CH_3$ | S | |
| 7.015 | 2-Methoxy-1-oxopyridin-3-yl | $CH_3$ | $CH_3$ | O | |
| 7.016 | 2-Methoxy-1-oxopyridin-3-yl | $CH_3$ | $CH_3$ | S | |
| 7.017 | 4-Methoxy-1-oxopyridin-3-yl | $CH_3$ | $CH_3$ | O | |
| 7.018 | 4-Methoxy-1-oxopyridin-3-yl | $CH_3$ | $CH_3$ | S | |
| 7.019 | 5-Methoxy-1-oxopyridin-3-yl | $CH_3$ | $CH_3$ | O | |
| 7.020 | 5-Methoxy-1-oxopyridin-3-yl | $CH_3$ | $CH_3$ | S | |
| 7.021 | 6-Methoxy-1-oxopyridin-3-yl | $CH_3$ | $CH_3$ | O | |
| 7.022 | 6-Methoxy-1-oxopyridin-3-yl | $CH_3$ | $CH_3$ | S | |
| 7.023 | 2-Methoxy-1-oxopyridin-4-yl | $CH_3$ | $CH_3$ | O | |
| 7.024 | 2-Methoxy-1-oxopyridin-4-yl | $CH_3$ | $CH_3$ | S | |
| 7.025 | 3-Methoxy-1-oxopyridin-4-yl | $CH_3$ | $CH_3$ | O | |
| 7.026 | 3-Methoxy-1-oxopyridin-4-yl | $CH_3$ | $CH_3$ | S | |
| 7.027 | 3-Ethoxy-1-oxopyridin-2-yl | $CH_3$ | $CH_3$ | O | |
| 7.028 | 3-Ethoxy-1-oxopyridin-2-yl | $CH_3$ | $CH_3$ | S | |
| 7.029 | 4-Ethoxy-1-oxopyridin-2-yl | $CH_3$ | $CH_3$ | O | |
| 7.030 | 4-Ethoxy-1-oxopyridin-2-yl | $CH_3$ | $CH_3$ | S | |

| Nr. | Py | $R^{18}$ | $R^{19}$ | Y | phys. Daten |
|---|---|---|---|---|---|
| 7.031 | 5-Ethoxy-1-oxopyridin-2-yl | $CH_3$ | $CH_3$ | O | |
| 7.032 | 5-Ethoxy-1-oxopyridin-2-yl | $CH_3$ | $CH_3$ | S | |
| 7.033 | 6-Ethoxy-1-oxopyridin-2-yl | $CH_3$ | $CH_3$ | O | |
| 7.034 | 6-Ethoxy-1-oxopyridin-2-yl | $CH_3$ | $CH_3$ | S | |
| 7.035 | 2-Ethoxy-1-oxopyridin-3-yl | $CH_3$ | $CH_3$ | O | |
| 7.036 | 2-Ethoxy-1-oxopyridin-3-yl | $CH_3$ | $CH_3$ | S | |
| 7.037 | 4-Ethoxy-1-oxopyridin-3-yl | $CH_3$ | $CH_3$ | O | |
| 7.038 | 4-Ethoxy-1-oxopyridin-3-yl | $CH_3$ | $CH_3$ | S | |
| 7.039 | 5-Ethoxy-1-oxopyridin-3-yl | $CH_3$ | $CH_3$ | O | |
| 7.040 | 5-Ethoxy-1-oxopyridin-3-yl | $CH_3$ | $CH_3$ | S | |
| 7.041 | 6-Ethoxy-1-oxopyridin-3-yl | $CH_3$ | $CH_3$ | O | |
| 7.042 | 6-Ethoxy-1-oxopyridin-3-yl | $CH_3$ | $CH_3$ | S | |
| 7.043 | 2-Ethoxy-1-oxopyridin-4-yl | $CH_3$ | $CH_3$ | O | |
| 7.044 | 2-Ethoxy-1-oxopyridin-4-yl | $CH_3$ | $CH_3$ | S | |
| 7.045 | 3-Ethoxy-1-oxopyridin-4-yl | $CH_3$ | $CH_3$ | O | |
| 7.046 | 3-Ethoxy-1-oxopyridin-4-yl | $CH_3$ | $CH_3$ | S | |
| 7.047 | 3-Isopropoxy-1-oxopyridin-2-yl | $CH_3$ | $CH_3$ | O | |
| 7.048 | 3-Isopropoxy-1-oxopyridin-2-yl | $CH_3$ | $CH_3$ | S | |
| 7.049 | 4-Isopropoxy-1-oxopyridin-2-yl | $CH_3$ | $CH_3$ | O | |
| 7.050 | 4-Isopropoxy-1-oxopyridin-2-yl | $CH_3$ | $CH_3$ | S | |
| 7.051 | 5-Isopropoxy-1-oxopyridin-2-yl | $CH_3$ | $CH_3$ | O | |

| Nr. | Py | $R^{18}$ | $R^{19}$ | Y | phys. Daten |
|---|---|---|---|---|---|
| 7.052 | 5-Isopropoxy-1-oxopyridin-2-yl | $CH_3$ | $CH_3$ | S | |
| 7.053 | 6-Isopropoxy-1-oxopyridin-2-yl | $CH_3$ | $CH_3$ | O | |
| 7.054 | 6-Isopropoxy-1-oxopyridin-2-yl | $CH_3$ | $CH_3$ | S | |
| 7.055 | 2-Isopropoxy-1-oxopyridin-3-yl | $CH_3$ | $CH_3$ | O | |
| 7.056 | 2-Isopropoxy-1-oxopyridin-3-yl | $CH_3$ | $CH_3$ | S | |
| 7.057 | 4-Isopropoxy-1-oxopyridin-3-yl | $CH_3$ | $CH_3$ | O | |
| 7.058 | 4-Isopropoxy-1-oxopyridin-3-yl | $CH_3$ | $CH_3$ | S | |
| 7.059 | 5-Isopropoxy-1-oxopyridin-3-yl | $CH_3$ | $CH_3$ | O | |
| 7.060 | 5-Isopropoxy-1-oxopyridin-3-yl | $CH_3$ | $CH_3$ | S | |
| 7.061 | 6-Isopropoxy-1-oxopyridin-3-yl | $CH_3$ | $CH_3$ | O | |
| 7.062 | 6-Isopropoxy-1-oxopyridin-3-yl | $CH_3$ | $CH_3$ | S | |
| 7.063 | 2-Isopropoxy-1-oxopyridin-4-yl | $CH_3$ | $CH_3$ | O | |
| 7.064 | 2-Isopropoxy-1-oxopyridin-4-yl | $CH_3$ | $CH_3$ | S | |
| 7.065 | 3-Isopropoxy-1-oxopyridin-4-yl | $CH_3$ | $CH_3$ | O | |
| 7.066 | 3-Isopropoxy-1-oxopyridin-4-yl | $CH_3$ | $CH_3$ | S | |
| 7.067 | 3-Methylthio-1-oxopyridin-2-yl | $CH_3$ | $CH_3$ | O | |
| 7.068 | 3-Methylthio-1-oxopyridin-2-yl | $CH_3$ | $CH_3$ | S | |
| 7.069 | 4-Methylthio-1-oxopyridin-2-yl | $CH_3$ | $CH_3$ | O | |
| 7.070 | 4-Methylthio-1-oxopyridin-2-yl | $CH_3$ | $CH_3$ | S | |
| 7.071 | 5-Methylthio-1-oxopyridin-2-yl | $CH_3$ | $CH_3$ | O | |
| 7.072 | 5-Methylthio-1-oxopyridin-2-yl | $CH_3$ | $CH_3$ | S | |

| Nr. | Py | $R^{18}$ | $R^{19}$ | Y | phys. Daten |
|---|---|---|---|---|---|
| 7.073 | 6-Methylthio-1-oxopyridin-2-yl | $CH_3$ | $CH_3$ | O | |
| 7.074 | 6-Methylthio-1-oxopyridin-2-yl | $CH_3$ | $CH_3$ | S | |
| 7.075 | 2-Methylthio-1-oxopyridin-3-yl | $CH_3$ | $CH_3$ | O | |
| 7.076 | 2-Methylthio-1-oxopyridin-3-yl | $CH_3$ | $CH_3$ | S | |
| 7.077 | 4-Methylthio-1-oxopyridin-3-yl | $CH_3$ | $CH_3$ | O | |
| 7.078 | 4-Methylthio-1-oxopyridin-3-yl | $CH_3$ | $CH_3$ | S | |
| 7.079 | 5-Methylthio-1-oxopyridin-3-yl | $CH_3$ | $CH_3$ | O | |
| 7.080 | 5-Methylthio-1-oxopyridin-3-yl | $CH_3$ | $CH_3$ | S | |
| 7.081 | 6-Methylthio-1-oxopyridin-3-yl | $CH_3$ | $CH_3$ | O | |
| 7.082 | 6-Methylthio-1-oxopyridin-3-yl | $CH_3$ | $CH_3$ | S | |
| 7.083 | 2-Methylthio-1-oxopyridin-4-yl | $CH_3$ | $CH_3$ | O | |
| 7.084 | 2-Methylthio-1-oxopyridin-4-yl | $CH_3$ | $CH_3$ | S | |
| 7.085 | 3-Methylthio-1-oxopyridin-4-yl | $CH_3$ | $CH_3$ | O | |
| 7.086 | 3-Methylthio-1-oxopyridin-4-yl | $CH_3$ | $CH_3$ | S | |
| 7.087 | 3-Propoxy-1-oxopyridin-2-yl | $CH_3$ | $CH_3$ | O | |
| 7.088 | 3-Propoxy-1-oxopyridin-2-yl | $CH_3$ | $CH_3$ | S | |
| 7.089 | 4-Propoxy-1-oxopyridin-2-yl | $CH_3$ | $CH_3$ | O | |
| 7.090 | 4-Propoxy-1-oxopyridin-2-yl | $CH_3$ | $CH_3$ | S | |
| 7.091 | 5-Propoxy-1-oxopyridin-2-yl | $CH_3$ | $CH_3$ | O | |
| 7.092 | 5-Propoxy-1-oxopyridin-2-yl | $CH_3$ | $CH_3$ | S | |
| 7.093 | 6-Propoxy-1-oxopyridin-2-yl | $CH_3$ | $CH_3$ | O | |

| Nr. | Py | $R^{18}$ | $R^{19}$ | Y | phys. Daten |
|---|---|---|---|---|---|
| 7.094 | 6-Propoxy-1-oxopyridin-2-yl | $CH_3$ | $CH_3$ | S | |
| 7.095 | 2-Propoxy-1-oxopyridin-3-yl | $CH_3$ | $CH_3$ | O | |
| 7.096 | 2-Propoxy-1-oxopyridin-3-yl | $CH_3$ | $CH_3$ | S | |
| 7.097 | 4-Propoxy-1-oxopyridin-3-yl | $CH_3$ | $CH_3$ | O | |
| 7.098 | 4-Propoxy-1-oxopyridin-3-yl | $CH_3$ | $CH_3$ | S | |
| 7.099 | 5-Propoxy-1-oxopyridin-3-yl | $CH_3$ | $CH_3$ | O | |
| 7.100 | 5-Propoxy-1-oxopyridin-3-yl | $CH_3$ | $CH_3$ | S | |
| 7.101 | 6-Propoxy-1-oxopyridin-3-yl | $CH_3$ | $CH_3$ | O | |
| 7.102 | 6-Propoxy-1-oxopyridin-3-yl | $CH_3$ | $CH_3$ | S | |
| 7.103 | 2-Propoxy-1-oxopyridin-4-yl | $CH_3$ | $CH_3$ | O | |
| 7.104 | 2-Propoxy-1-oxopyridin-4-yl | $CH_3$ | $CH_3$ | S | |
| 7.105 | 3-Propoxy-1-oxopyridin-4-yl | $CH_3$ | $CH_3$ | O | |
| 7.106 | 3-Propoxy-1-oxopyridin-4-yl | $CH_3$ | $CH_3$ | S | |
| 7.107 | 3-Trifluormethyl-1-oxopyridin-2-yl | $CH_3$ | $CH_3$ | O | |
| 7.108 | 3-Trifluormethyl-1-oxopyridin-2-yl | $CH_3$ | $CH_3$ | S | |
| 7.109 | 4-Trifluormethyl-1-oxopyridin-2-yl | $CH_3$ | $CH_3$ | O | |
| 7.110 | 4-Trifluormethyl-1-oxopyridin-2-yl | $CH_3$ | $CH_3$ | S | |
| 7.111 | 5-Trifluormethyl-1-oxopyridin-2-yl | $CH_3$ | $CH_3$ | O | |
| 7.112 | 5-Trifluormethyl-1-oxopyridin-2-yl | $CH_3$ | $CH_3$ | S | |
| 7.113 | 6-Trifluormethyl-1-oxopyridin-2-yl | $CH_3$ | $CH_3$ | O | |
| 7.114 | 6-Trifluormethyl-1-oxopyridin-2-yl | $CH_3$ | $CH_3$ | S | |

| Nr. | Py | $R^{18}$ | $R^{19}$ | Y | phys. Daten |
|---|---|---|---|---|---|
| 7.115 | 2-Trifluormethyl-1-oxopyridin-3-yl | $CH_3$ | $CH_3$ | O | |
| 7.116 | 2-Trifluormethyl-1-oxopyridin-3-yl | $CH_3$ | $CH_3$ | S | |
| 7.117 | 4-Trifluormethyl-1-oxopyridin-3-yl | $CH_3$ | $CH_3$ | O | |
| 7.118 | 4-Trifluormethyl-1-oxopyridin-3-yl | $CH_3$ | $CH_3$ | S | |
| 7.119 | 5-Trifluormethyl-1-oxopyridin-3-yl | $CH_3$ | $CH_3$ | O | |
| 7.120 | 5-Trifluormethyl-1-oxopyridin-3-yl | $CH_3$ | $CH_3$ | S | |
| 7.121 | 6-Trifluormethyl-1-oxopyridin-3-yl | $CH_3$ | $CH_3$ | O | |
| 7.122 | 6-Trifluormethyl-1-oxopyridin-3-yl | $CH_3$ | $CH_3$ | S | |
| 7.123 | 2-Trifluormethyl-1-oxopyridin-4-yl | $CH_3$ | $CH_3$ | O | |
| 7.124 | 2-Trifluormethyl-1-oxopyridin-4-yl | $CH_3$ | $CH_3$ | S | |
| 7.125 | 3-Trifluormethyl-1-oxopyridin-4-yl | $CH_3$ | $CH_3$ | O | |
| 7.126 | 3-Trifluormethyl-1-oxopyridin-4-yl | $CH_3$ | $CH_3$ | S | |
| 7.127 | 3-Fluor-1-oxopyridin-2-yl | $CH_3$ | $CH_3$ | O | |
| 7.128 | 3-Fluor-1-oxopyridin-2-yl | $CH_3$ | $CH_3$ | S | |
| 7.129 | 4-Fluor-1-oxopyridin-2-yl | $CH_3$ | $CH_3$ | O | |
| 7.130 | 4-Fluor-1-oxopyridin-2-yl | $CH_3$ | $CH_3$ | S | |
| 7.131 | 5-Fluor-1-oxopyridin-2-yl | $CH_3$ | $CH_3$ | O | |
| 7.132 | 5-Fluor-1-oxopyridin-2-yl | $CH_3$ | $CH_3$ | S | |
| 7.133 | 6-Fluor-1-oxopyridin-2-yl | $CH_3$ | $CH_3$ | O | |
| 7.134 | 6-Fluor-1-oxopyridin-2-yl | $CH_3$ | $CH_3$ | S | |
| 7.135 | 2-Fluor-1-oxopyridin-3-yl | $CH_3$ | $CH_3$ | O | |

| Nr. | Py | $R^{18}$ | $R^{19}$ | Y | phys. Daten |
|---|---|---|---|---|---|
| 7.136 | 2-Fluor-1-oxopyridin-3-yl | $CH_3$ | $CH_3$ | S | |
| 7.137 | 4-Fluor-1-oxopyridin-3-yl | $CH_3$ | $CH_3$ | O | |
| 7.138 | 4-Fluor-1-oxopyridin-3-yl | $CH_3$ | $CH_3$ | S | |
| 7.139 | 5-Fluor-1-oxopyridin-3-yl | $CH_3$ | $CH_3$ | O | |
| 7.140 | 5-Fluor-1-oxopyridin-3-yl | $CH_3$ | $CH_3$ | S | |
| 7.141 | 6-Fluor-1-oxopyridin-3-yl | $CH_3$ | $CH_3$ | O | |
| 7.142 | 6-Fluor-1-oxopyridin-3-yl | $CH_3$ | $CH_3$ | S | |
| 7.143 | 2-Fluor-1-oxopyridin-4-yl | $CH_3$ | $CH_3$ | O | |
| 7.144 | 2-Fluor-1-oxopyridin-4-yl | $CH_3$ | $CH_3$ | S | |
| 7.145 | 3-Fluor-1-oxopyridin-4-yl | $CH_3$ | $CH_3$ | O | |
| 7.146 | 3-Fluor-1-oxopyridin-4-yl | $CH_3$ | $CH_3$ | S | |
| 7.147 | 3-Chlor-1-oxopyridin-2-yl | $CH_3$ | $CH_3$ | O | |
| 7.148 | 3-Chlor-1-oxopyridin-2-yl | $CH_3$ | $CH_3$ | S | |
| 7.149 | 4-Chlor-1-oxopyridin-2-yl | $CH_3$ | $CH_3$ | O | |
| 7.150 | 4-Chlor-1-oxopyridin-2-yl | $CH_3$ | $CH_3$ | S | |
| 7.151 | 5-Chlor-1-oxopyridin-2-yl | $CH_3$ | $CH_3$ | O | |
| 7.152 | 5-Chlor-1-oxopyridin-2-yl | $CH_3$ | $CH_3$ | S | |
| 7.153 | 6-Chlor-1-oxopyridin-2-yl | $CH_3$ | $CH_3$ | O | |
| 7.154 | 6-Chlor-1-oxopyridin-2-yl | $CH_3$ | $CH_3$ | S | |
| 7.155 | 2-Chlor-1-oxopyridin-3-yl | $CH_3$ | $CH_3$ | O | |
| 7.156 | 2-Chlor-1-oxopyridin-3-yl | $CH_3$ | $CH_3$ | S | |

| Nr. | Py | $R^{18}$ | $R^{19}$ | Y | phys. Daten |
|---|---|---|---|---|---|
| 7.157 | 4-Chlor-1-oxopyridin-3-yl | $CH_3$ | $CH_3$ | O | |
| 7.158 | 4-Chlor-1-oxopyridin-3-yl | $CH_3$ | $CH_3$ | S | |
| 7.159 | 5-Chlor-1-oxopyridin-3-yl | $CH_3$ | $CH_3$ | O | |
| 7.160 | 5-Chlor-1-oxopyridin-3-yl | $CH_3$ | $CH_3$ | S | |
| 7.161 | 6-Chlor-1-oxopyridin-3-yl | $CH_3$ | $CH_3$ | O | |
| 7.162 | 6-Chlor-1-oxopyridin-3-yl | $CH_3$ | $CH_3$ | S | |
| 7.163 | 2-Chlor-1-oxopyridin-4-yl | $CH_3$ | $CH_3$ | O | |
| 7.164 | 2-Chlor-1-oxopyridin-4-yl | $CH_3$ | $CH_3$ | S | |
| 7.165 | 3-Chlor-1-oxopyridin-4-yl | $CH_3$ | $CH_3$ | O | |
| 7.166 | 3-Chlor-1-oxopyridin-4-yl | $CH_3$ | $CH_3$ | S | |
| 7.167 | 3-Methyl-1-oxopyridin-2-yl | $CH_3$ | $CH_3$ | O | |
| 7.168 | 3-Methyl-1-oxopyridin-2-yl | $CH_3$ | $CH_3$ | S | |
| 7.169 | 4-Methyl-1-oxopyridin-2-yl | $CH_3$ | $CH_3$ | O | |
| 7.170 | 4-Methyl-1-oxopyridin-2-yl | $CH_3$ | $CH_3$ | S | |
| 7.171 | 5-Methyl-1-oxopyridin-2-yl | $CH_3$ | $CH_3$ | O | |
| 7.172 | 5-Methyl-1-oxopyridin-2-yl | $CH_3$ | $CH_3$ | S | |
| 7.173 | 6-Methyl-1-oxopyridin-2-yl | $CH_3$ | $CH_3$ | O | |
| 7.174 | 6-Methyl-1-oxopyridin-2-yl | $CH_3$ | $CH_3$ | S | |
| 7.175 | 2-Methyl-1-oxopyridin-3-yl | $CH_3$ | $CH_3$ | O | |
| 7.176 | 2-Methyl-1-oxopyridin-3-yl | $CH_3$ | $CH_3$ | S | |
| 7.177 | 4-Methyl-1-oxopyridin-3-yl | $CH_3$ | $CH_3$ | O | |

| Nr. | Py | $R^{18}$ | $R^{19}$ | Y | phys. Daten |
|---|---|---|---|---|---|
| 7.178 | 4-Methyl-1-oxopyridin-3-yl | $CH_3$ | $CH_3$ | S | |
| 7.179 | 5-Methyl-1-oxopyridin-3-yl | $CH_3$ | $CH_3$ | O | |
| 7.180 | 5-Methyl-1-oxopyridin-3-yl | $CH_3$ | $CH_3$ | S | |
| 7.181 | 6-Methyl-1-oxopyridin-3-yl | $CH_3$ | $CH_3$ | O | |
| 7.182 | 6-Methyl-1-oxopyridin-3-yl | $CH_3$ | $CH_3$ | S | |
| 7.183 | 2-Methyl-1-oxopyridin-4-yl | $CH_3$ | $CH_3$ | O | |
| 7.184 | 2-Methyl-1-oxopyridin-4-yl | $CH_3$ | $CH_3$ | S | |
| 7.185 | 3-Methyl-1-oxopyridin-4-yl | $CH_3$ | $CH_3$ | O | |
| 7.186 | 3-Methyl-1-oxopyridin-4-yl | $CH_3$ | $CH_3$ | S | |
| 7.187 | 3-Ethyl-1-oxopyridin-2-yl | $CH_3$ | $CH_3$ | O | |
| 7.188 | 3-Ethyl-1-oxopyridin-2-yl | $CH_3$ | $CH_3$ | S | |
| 7.189 | 4-Ethyl-1-oxopyridin-2-yl | $CH_3$ | $CH_3$ | O | |
| 7.190 | 4-Ethyl-1-oxopyridin-2-yl | $CH_3$ | $CH_3$ | S | |
| 7.191 | 5-Ethyl-1-oxopyridin-2-yl | $CH_3$ | $CH_3$ | O | |
| 7.192 | 5-Ethyl-1-oxopyridin-2-yl | $CH_3$ | $CH_3$ | S | |
| 7.193 | 6-Ethyl-1-oxopyridin-2-yl | $CH_3$ | $CH_3$ | O | |
| 7.194 | 6-Ethyl-1-oxopyridin-2-yl | $CH_3$ | $CH_3$ | S | |
| 7.195 | 2-Ethyl-1-oxopyridin-3-yl | $CH_3$ | $CH_3$ | O | |
| 7.196 | 2-Ethyl-1-oxopyridin-3-yl | $CH_3$ | $CH_3$ | S | |
| 7.197 | 4-Ethyl-1-oxopyridin-3-yl | $CH_3$ | $CH_3$ | O | |
| 7.198 | 4-Ethyl-1-oxopyridin-3-yl | $CH_3$ | $CH_3$ | S | |

| Nr. | Py | $R^{18}$ | $R^{19}$ | Y | phys. Daten |
|---|---|---|---|---|---|
| 7.199 | 5-Ethyl-1-oxopyridin-3-yl | $CH_3$ | $CH_3$ | O | |
| 7.200 | 5-Ethyl-1-oxopyridin-3-yl | $CH_3$ | $CH_3$ | S | |
| 7.201 | 6-Ethyl-1-oxopyridin-3-yl | $CH_3$ | $CH_3$ | O | |
| 7.202 | 6-Ethyl-1-oxopyridin-3-yl | $CH_3$ | $CH_3$ | S | |
| 7.203 | 2-Ethyl-1-oxopyridin-4-yl | $CH_3$ | $CH_3$ | O | |
| 7.204 | 2-Ethyl-1-oxopyridin-4-yl | $CH_3$ | $CH_3$ | S | |
| 7.205 | 3-Ethyl-1-oxopyridin-4-yl | $CH_3$ | $CH_3$ | O | |
| 7.206 | 3-Ethyl-1-oxopyridin-4-yl | $CH_3$ | $CH_3$ | S | |
| 7.207 | 3-(2-Methoxyethoxy)-1-oxopyridin-2-yl | $CH_3$ | $CH_3$ | O | |
| 7.208 | 3-(2-Methoxyethoxy)-1-oxopyridin-2-yl | $CH_3$ | $CH_3$ | S | |
| 7.209 | 4-(2-Methoxyethoxy)-1-oxopyridin-2-yl | $CH_3$ | $CH_3$ | O | |
| 7.210 | 4-(2-Methoxyethoxy)-1-oxopyridin-2-yl | $CH_3$ | $CH_3$ | S | |
| 7.211 | 5-(2-Methoxyethoxy)-1-oxopyridin-2-yl | $CH_3$ | $CH_3$ | O | |
| 7.212 | 5-(2-Methoxyethoxy)-1-oxopyridin-2-yl | $CH_3$ | $CH_3$ | S | |
| 7.213 | 6-(2-Methoxyethoxy)-1-oxopyridin-2-yl | $CH_3$ | $CH_3$ | O | |
| 7.214 | 6-(2-Methoxyethoxy)-1-oxopyridin-2-yl | $CH_3$ | $CH_3$ | S | |
| 7.215 | 2-(2-Methoxyethoxy)-1-oxopyridin-3-yl | $CH_3$ | $CH_3$ | O | |
| 7.216 | 2-(2-Methoxyethoxy)-1-oxopyridin-3-yl | $CH_3$ | $CH_3$ | S | |
| 7.217 | 4-(2-Methoxyethoxy)-1-oxopyridin-3-yl | $CH_3$ | $CH_3$ | O | |
| 7.218 | 4-(2-Methoxyethoxy)-1-oxopyridin-3-yl | $CH_3$ | $CH_3$ | S | |
| 7.219 | 5-(2-Methoxyethoxy)-1-oxopyridin-3-yl | $CH_3$ | $CH_3$ | O | |

| Nr. | Py | $R^{18}$ | $R^{19}$ | Y | phys. Daten |
|---|---|---|---|---|---|
| 7.220 | 5-(2-Methoxyethoxy)-1-oxopyridin-3-yl | $CH_3$ | $CH_3$ | S | |
| 7.221 | 6-(2-Methoxyethoxy)-1-oxopyridin-3-yl | $CH_3$ | $CH_3$ | O | |
| 7.222 | 6-(2-Methoxyethoxy)-1-oxopyridin-3-yl | $CH_3$ | $CH_3$ | S | |
| 7.223 | 2-(2-Methoxyethoxy)-1-oxopyridin-4-yl | $CH_3$ | $CH_3$ | O | |
| 7.224 | 2-(2-Methoxyethoxy)-1-oxopyridin-4-yl | $CH_3$ | $CH_3$ | S | |
| 7.225 | 3-(2-Methoxyethoxy)-1-oxopyridin-4-yl | $CH_3$ | $CH_3$ | O | |
| 7.226 | 3-(2-Methoxyethoxy)-1-oxopyridin-4-yl | $CH_3$ | $CH_3$ | S | |
| 7.227 | 3,5-Dimethyl-1-oxopyridin-2-yl | $CH_3$ | $CH_3$ | O | |
| 7.228 | 3,5-Dimethyl-1-oxopyridin-2-yl | $CH_3$ | $CH_3$ | S | |
| 7.229 | 6-Methoxy-4-methyl-1-oxopyridin-2-yl | $CH_3$ | $CH_3$ | O | |
| 7.230 | 6-Methoxy-4-methyl-1-oxopyridin-2-yl | $CH_3$ | $CH_3$ | S | |
| 7.231 | 5-(2,2,2-Trifluorethoxy)-1-oxopyridin-2-yl | $CH_3$ | $CH_3$ | O | |
| 7.232 | 5-(2,2,2-Trifluorethoxy)-1-oxopyridin-2-yl | $CH_3$ | $CH_3$ | S | |
| 7.233 | 6-Dimethylamino-1-oxopyridin-2-yl | $CH_3$ | $CH_3$ | O | |
| 7.234 | 6-Dimethylamino-1-oxopyridin-2-yl | $CH_3$ | $CH_3$ | S | |
| 7.235 | 2-Methoxy-5-trifluormethyl-1-oxopyridin-3-yl | $CH_3$ | $CH_3$ | O | |
| 7.236 | 2-Methoxy-5-trifluormethyl-1-oxopyridin-3-yl | $CH_3$ | $CH_3$ | S | |
| 7.237 | 6-Dimethylaminoxy-1-oxopyridin-2-yl | $CH_3$ | $CH_3$ | O | |
| 7.238 | 6-Dimethylaminoxy-1-oxopyridin-2-yl | $CH_3$ | $CH_3$ | S | |
| 7.239 | 6-Isopropyl-1-oxopyridin-2-yl | $CH_3$ | $CH_3$ | O | |
| 7.240 | 6-Isopropyl-1-oxopyridin-4-yl | $CH_3$ | $CH_3$ | S | |

| Nr. | Py | $R^{18}$ | $R^{19}$ | Y | phys. Daten |
|---|---|---|---|---|---|
| 7.241 | 1-Oxopyridin-2-yl | $C_6H_5$ | H | O | |
| 7.242 | 1-Oxopyridin-2-yl | t-$C_4H_9$ | H | O | |
| 7.243 | 1-Oxopyridin-4-yl | $C_6H_5$ | $CH_3$ | O | |
| 7.244 | 1-Oxopyridin-4-yl | $C_2H_5$ | $CH_3$ | S | |

Zwischenprodukte der Formel III sind in nachfolgender Tabelle 8 zusammengestellt.

Tabelle 8

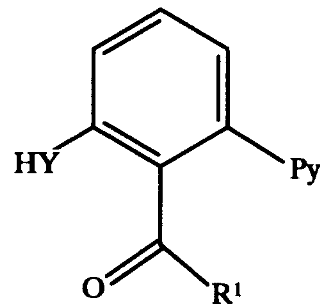

III

| Nr. | Py | $R^1$ | Y | phys. Dat. |
|---|---|---|---|---|
| 8.001 | 1-Oxopyridin-2-yl | 2-Propaniminoxy | O | |
| 8.002 | 1-Oxopyridin-2-yl | 2-Propaniminoxy | S | |
| 8.003 | 1-Oxopyridin-3-yl | 2-Propaniminoxy | O | |
| 8.004 | 1-Oxopyridin-3-yl | 2-Propaniminoxy | S | |
| 8.005 | 1-Oxopyridin-4-yl | 2-Propaniminoxy | O | |
| 8.006 | 1-Oxopyridin-4-yl | 2-Propaniminoxy | S | |
| 8.007 | 3-Methoxy-1-oxopyridin-2-yl | 2-Propaniminoxy | O | |
| 8.008 | 3-Methoxy-1-oxopyridin-2-yl | 2-Propaniminoxy | S | |
| 8.009 | 4-Methoxy-1-oxopyridin-2-yl | 2-Propaniminoxy | O | |

| Nr. | Py | $R^1$ | Y | phys. Dat. |
|---|---|---|---|---|
| 8.010 | 4-Methoxy-1-oxopyridin-2-yl | 2-Propaniminoxy | S | |
| 8.011 | 5-Methoxy-1-oxopyridin-2-yl | 2-Propaniminoxy | O | |
| 8.012 | 5-Methoxy-1-oxopyridin-2-yl | 2-Propaniminoxy | S | |
| 8.013 | 6-Methoxy-1-oxopyridin-2-yl | 2-Propaniminoxy | O | |
| 8.014 | 6-Methoxy-1-oxopyridin-2-yl | 2-Propaniminoxy | S | |
| 8.015 | 2-Methoxy-1-oxopyridin-3-yl | 2-Propaniminoxy | O | |
| 8.016 | 2-Methoxy-1-oxopyridin-3-yl | 2-Propaniminoxy | S | |
| 8.017 | 4-Methoxy-1-oxopyridin-3-yl | 2-Propaniminoxy | O | |
| 8.018 | 4-Methoxy-1-oxopyridin-3-yl | 2-Propaniminoxy | S | |
| 8.019 | 5-Methoxy-1-oxopyridin-3-yl | 2-Propaniminoxy | O | |
| 8.020 | 5-Methoxy-1-oxopyridin-3-yl | 2-Propaniminoxy | S | |
| 8.021 | 6-Methoxy-1-oxopyridin-3-yl | 2-Propaniminoxy | O | |
| 8.022 | 6-Methoxy-1-oxopyridin-3-yl | 2-Propaniminoxy | S | |
| 8.023 | 2-Methoxy-1-oxopyridin-4-yl | 2-Propaniminoxy | O | |
| 8.024 | 2-Methoxy-1-oxopyridin-4-yl | 2-Propaniminoxy | S | |
| 8.025 | 3-Methoxy-1-oxopyridin-4-yl | 2-Propaniminoxy | O | |
| 8.026 | 3-Methoxy-1-oxopyridin-4-yl | 2-Propaniminoxy | S | |
| 8.027 | 3-Ethoxy-1-oxopyridin-2-yl | 2-Propaniminoxy | O | |
| 8.028 | 3-Ethoxy-1-oxopyridin-2-yl | 2-Propaniminoxy | S | |
| 8.029 | 4-Ethoxy-1-oxopyridin-2-yl | 2-Propaniminoxy | O | |
| 8.030 | 4-Ethoxy-1-oxopyridin-2-yl | 2-Propaniminoxy | S | |

| Nr. | Py | $R^1$ | Y | phys. Dat. |
|---|---|---|---|---|
| 8.031 | 5-Ethoxy-1-oxopyridin-2-yl | 2-Propaniminoxy | O | |
| 8.032 | 5-Ethoxy-1-oxopyridin-2-yl | 2-Propaniminoxy | S | |
| 8.033 | 6-Ethoxy-1-oxopyridin-2-yl | 2-Propaniminoxy | O | |
| 8.034 | 6-Ethoxy-1-oxopyridin-2-yl | 2-Propaniminoxy | S | |
| 8.035 | 2-Ethoxy-1-oxopyridin-3-yl | 2-Propaniminoxy | O | |
| 8.036 | 2-Ethoxy-1-oxopyridin-3-yl | 2-Propaniminoxy | S | |
| 8.037 | 4-Ethoxy-1-oxopyridin-3-yl | 2-Propaniminoxy | O | |
| 8.038 | 4-Ethoxy-1-oxopyridin-3-yl | 2-Propaniminoxy | S | |
| 8.039 | 5-Ethoxy-1-oxopyridin-3-yl | 2-Propaniminoxy | O | |
| 8.040 | 5-Ethoxy-1-oxopyridin-3-yl | 2-Propaniminoxy | S | |
| 8.041 | 6-Ethoxy-1-oxopyridin-3-yl | 2-Propaniminoxy | O | |
| 8.042 | 6-Ethoxy-1-oxopyridin-3-yl | 2-Propaniminoxy | S | |
| 8.043 | 2-Ethoxy-1-oxopyridin-4-yl | 2-Propaniminoxy | O | |
| 8.044 | 2-Ethoxy-1-oxopyridin-4-yl | 2-Propaniminoxy | S | |
| 8.045 | 3-Ethoxy-1-oxopyridin-4-yl | 2-Propaniminoxy | O | |
| 8.046 | 3-Ethoxy-1-oxopyridin-4-yl | 2-Propaniminoxy | S | |
| 8.047 | 3-Isopropoxy-1-oxopyridin-2-yl | 2-Propaniminoxy | O | |
| 8.048 | 3-Isopropoxy-1-oxopyridin-2-yl | 2-Propaniminoxy | S | |
| 8.049 | 4-Isopropoxy-1-oxopyridin-2-yl | 2-Propaniminoxy | O | |
| 8.050 | 4-Isopropoxy-1-oxopyridin-2-yl | 2-Propaniminoxy | S | |
| 8.051 | 5-Isopropoxy-1-oxopyridin-2-yl | 2-Propaniminoxy | O | |

| Nr. | Py | $R^1$ | Y | phys. Dat. |
|---|---|---|---|---|
| 8.052 | 5-Isopropoxy-1-oxopyridin-2-yl | 2-Propaniminoxy | S | |
| 8.053 | 6-Isopropoxy-1-oxopyridin-2-yl | 2-Propaniminoxy | O | |
| 8.054 | 6-Isopropoxy-1-oxopyridin-2-yl | 2-Propaniminoxy | S | |
| 8.055 | 2-Isopropoxy-1-oxopyridin-3-yl | 2-Propaniminoxy | O | |
| 8.056 | 2-Isopropoxy-1-oxopyridin-3-yl | 2-Propaniminoxy | S | |
| 8.057 | 4-Isopropoxy-1-oxopyridin-3-yl | 2-Propaniminoxy | O | |
| 8.058 | 4-Isopropoxy-1-oxopyridin-3-yl | 2-Propaniminoxy | S | |
| 8.059 | 5-Isopropoxy-1-oxopyridin-3-yl | 2-Propaniminoxy | O | |
| 8.060 | 5-Isopropoxy-1-oxopyridin-3-yl | 2-Propaniminoxy | S | |
| 8.061 | 6-Isopropoxy-1-oxopyridin-3-yl | 2-Propaniminoxy | O | |
| 8.062 | 6-Isopropoxy-1-oxopyridin-3-yl | 2-Propaniminoxy | S | |
| 8.063 | 2-Isopropoxy-1-oxopyridin-4-yl | 2-Propaniminoxy | O | |
| 8.064 | 2-Isopropoxy-1-oxopyridin-4-yl | 2-Propaniminoxy | S | |
| 8.065 | 3-Isopropoxy-1-oxopyridin-4-yl | 2-Propaniminoxy | O | |
| 8.066 | 3-Isopropoxy-1-oxopyridin-4-yl | 2-Propaniminoxy | S | |
| 8.067 | 3-Methylthio-1-oxopyridin-2-yl | 2-Propaniminoxy | O | |
| 8.068 | 3-Methylthio-1-oxopyridin-2-yl | 2-Propaniminoxy | S | |
| 8.069 | 4-Methylthio-1-oxopyridin-2-yl | 2-Propaniminoxy | O | |
| 8.070 | 4-Methylthio-1-oxopyridin-2-yl | 2-Propaniminoxy | S | |
| 8.071 | 5-Methylthio-1-oxopyridin-2-yl | 2-Propaniminoxy | O | |
| 8.072 | 5-Methylthio-1-oxopyridin-2-yl | 2-Propaniminoxy | S | |

| Nr. | Py | $R^1$ | Y | phys. Dat. |
|---|---|---|---|---|
| 8.073 | 6-Methylthio-1-oxopyridin-2-yl | 2-Propaniminoxy | O | |
| 8.074 | 6-Methylthio-1-oxopyridin-2-yl | 2-Propaniminoxy | S | |
| 8.075 | 2-Methylthio-1-oxopyridin-3-yl | 2-Propaniminoxy | O | |
| 8.076 | 2-Methylthio-1-oxopyridin-3-yl | 2-Propaniminoxy | S | |
| 8.077 | 4-Methylthio-1-oxopyridin-3-yl | 2-Propaniminoxy | O | |
| 8.078 | 4-Methylthio-1-oxopyridin-3-yl | 2-Propaniminoxy | S | |
| 8.079 | 5-Methylthio-1-oxopyridin-3-yl | 2-Propaniminoxy | O | |
| 8.080 | 5-Methylthio-1-oxopyridin-3-yl | 2-Propaniminoxy | S | |
| 8.081 | 6-Methylthio-1-oxopyridin-3-yl | 2-Propaniminoxy | O | |
| 8.082 | 6-Methylthio-1-oxopyridin-3-yl | 2-Propaniminoxy | S | |
| 8.083 | 2-Methylthio-1-oxopyridin-4-yl | 2-Propaniminoxy | O | |
| 8.084 | 2-Methylthio-1-oxopyridin-4-yl | 2-Propaniminoxy | S | |
| 8.085 | 3-Methylthio-1-oxopyridin-4-yl | 2-Propaniminoxy | O | |
| 8.086 | 3-Methylthio-1-oxopyridin-4-yl | 2-Propaniminoxy | S | |
| 8.087 | 3-Propoxy-1-oxopyridin-2-yl | 2-Propaniminoxy | O | |
| 8.088 | 3-Propoxy-1-oxopyridin-2-yl | 2-Propaniminoxy | S | |
| 8.089 | 4-Propoxy-1-oxopyridin-2-yl | 2-Propaniminoxy | O | |
| 8.090 | 4-Propoxy-1-oxopyridin-2-yl | 2-Propaniminoxy | S | |
| 8.091 | 5-Propoxy-1-oxopyridin-2-yl | 2-Propaniminoxy | O | |
| 8.092 | 5-Propoxy-1-oxopyridin-2-yl | 2-Propaniminoxy | S | |
| 8.093 | 6-Propoxy-1-oxopyridin-2-yl | 2-Propaniminoxy | O | |

| Nr. | Py | $R^1$ | Y | phys. Dat. |
|---|---|---|---|---|
| 8.094 | 6-Propoxy-1-oxopyridin-2-yl | 2-Propaniminoxy | S | |
| 8.095 | 2-Propoxy-1-oxopyridin-3-yl | 2-Propaniminoxy | O | |
| 8.096 | 2-Propoxy-1-oxopyridin-3-yl | 2-Propaniminoxy | S | |
| 8.097 | 4-Propoxy-1-oxopyridin-3-yl | 2-Propaniminoxy | O | |
| 8.098 | 4-Propoxy-1-oxopyridin-3-yl | 2-Propaniminoxy | S | |
| 8.099 | 5-Propoxy-1-oxopyridin-3-yl | 2-Propaniminoxy | O | |
| 8.100 | 5-Propoxy-1-oxopyridin-3-yl | 2-Propaniminoxy | S | |
| 8.101 | 6-Propoxy-1-oxopyridin-3-yl | 2-Propaniminoxy | O | |
| 8.102 | 6-Propoxy-1-oxopyridin-3-yl | 2-Propaniminoxy | S | |
| 8.103 | 2-Propoxy-1-oxopyridin-4-yl | 2-Propaniminoxy | O | |
| 8.104 | 2-Propoxy-1-oxopyridin-4-yl | 2-Propaniminoxy | S | |
| 8.105 | 3-Propoxy-1-oxopyridin-4-yl | 2-Propaniminoxy | O | |
| 8.106 | 3-Propoxy-1-oxopyridin-4-yl | 2-Propaniminoxy | S | |
| 8.107 | 3-Trifluormethyl-1-oxopyridin-2-yl | 2-Propaniminoxy | O | |
| 8.108 | 3-Trifluormethyl-1-oxopyridin-2-yl | 2-Propaniminoxy | S | |
| 8.109 | 4-Trifluormethyl-1-oxopyridin-2-yl | 2-Propaniminoxy | O | |
| 8.110 | 4-Trifluormethyl-1-oxopyridin-2-yl | 2-Propaniminoxy | S | |
| 8.111 | 5-Trifluormethyl-1-oxopyridin-2-yl | 2-Propaniminoxy | O | |
| 8.112 | 5-Trifluormethyl-1-oxopyridin-2-yl | 2-Propaniminoxy | S | |
| 8.113 | 6-Trifluormethyl-1-oxopyridin-2-yl | 2-Propaniminoxy | O | |
| 8.114 | 6-Trifluormethyl-1-oxopyridin-2-yl | 2-Propaniminoxy | S | |

| Nr. | Py | $R^1$ | Y | phys. Dat. |
|---|---|---|---|---|
| 8.115 | 2-Trifluormethyl-1-oxopyridin-3-yl | 2-Propaniminoxy | O | |
| 8.116 | 2-Trifluormethyl-1-oxopyridin-3-yl | 2-Propaniminoxy | S | |
| 8.117 | 4-Trifluormethyl-1-oxopyridin-3-yl | 2-Propaniminoxy | O | |
| 8.118 | 4-Trifluormethyl-1-oxopyridin-3-yl | 2-Propaniminoxy | S | |
| 8.119 | 5-Trifluormethyl-1-oxopyridin-3-yl | 2-Propaniminoxy | O | |
| 8.120 | 5-Trifluormethyl-1-oxopyridin-3-yl | 2-Propaniminoxy | S | |
| 8.121 | 6-Trifluormethyl-1-oxopyridin-3-yl | 2-Propaniminoxy | O | |
| 8.122 | 6-Trifluormethyl-1-oxopyridin-3-yl | 2-Propaniminoxy | S | |
| 8.123 | 2-Trifluormethyl-1-oxopyridin-4-yl | 2-Propaniminoxy | O | |
| 8.124 | 2-Trifluormethyl-1-oxopyridin-4-yl | 2-Propaniminoxy | S | |
| 8.125 | 3-Trifluormethyl-1-oxopyridin-4-yl | 2-Propaniminoxy | O | |
| 8.126 | 3-Trifluormethyl-1-oxopyridin-4-yl | 2-Propaniminoxy | S | |
| 8.127 | 3-Fluor-1-oxopyridin-2-yl | 2-Propaniminoxy | O | |
| 8.128 | 3-Fluor-1-oxopyridin-2-yl | 2-Propaniminoxy | S | |
| 8.129 | 4-Fluor-1-oxopyridin-2-yl | 2-Propaniminoxy | O | |
| 8.130 | 4-Fluor-1-oxopyridin-2-yl | 2-Propaniminoxy | S | |
| 8.131 | 5-Fluor-1-oxopyridin-2-yl | 2-Propaniminoxy | O | |
| 8.132 | 5-Fluor-1-oxopyridin-2-yl | 2-Propaniminoxy | S | |
| 8.133 | 6-Fluor-1-oxopyridin-2-yl | 2-Propaniminoxy | O | |
| 8.134 | 6-Fluor-1-oxopyridin-2-yl | 2-Propaniminoxy | S | |
| 8.135 | 2-Fluor-1-oxopyridin-3-yl | 2-Propaniminoxy | O | |

| Nr. | Py | $R^1$ | Y | phys. Dat. |
|---|---|---|---|---|
| 8.136 | 2-Fluor-1-oxopyridin-3-yl | 2-Propaniminoxy | S | |
| 8.137 | 4-Fluor-1-oxopyridin-3-yl | 2-Propaniminoxy | O | |
| 8.138 | 4-Fluor-1-oxopyridin-3-yl | 2-Propaniminoxy | S | |
| 8.139 | 5-Fluor-1-oxopyridin-3-yl | 2-Propaniminoxy | O | |
| 8.140 | 5-Fluor-1-oxopyridin-3-yl | 2-Propaniminoxy | S | |
| 8.141 | 6-Fluor-1-oxopyridin-3-yl | 2-Propaniminoxy | O | |
| 8.142 | 6-Fluor-1-oxopyridin-3-yl | 2-Propaniminoxy | S | |
| 8.143 | 2-Fluor-1-oxopyridin-4-yl | 2-Propaniminoxy | O | |
| 8.144 | 2-Fluor-1-oxopyridin-4-yl | 2-Propaniminoxy | S | |
| 8.145 | 3-Fluor-1-oxopyridin-4-yl | 2-Propaniminoxy | O | |
| 8.146 | 3-Fluor-1-oxopyridin-4-yl | 2-Propaniminoxy | S | |
| 8.147 | 3-Chlor-1-oxopyridin-2-yl | 2-Propaniminoxy | O | |
| 8.148 | 3-Chlor-1-oxopyridin-2-yl | 2-Propaniminoxy | S | |
| 8.149 | 4-Chlor-1-oxopyridin-2-yl | 2-Propaniminoxy | O | |
| 8.150 | 4-Chlor-1-oxopyridin-2-yl | 2-Propaniminoxy | S | |
| 8.151 | 5-Chlor-1-oxopyridin-2-yl | 2-Propaniminoxy | O | |
| 8.152 | 5-Chlor-1-oxopyridin-2-yl | 2-Propaniminoxy | S | |
| 8.153 | 6-Chlor-1-oxopyridin-2-yl | 2-Propaniminoxy | O | |
| 8.154 | 6-Chlor-1-oxopyridin-2-yl | 2-Propaniminoxy | S | |
| 8.155 | 2-Chlor-1-oxopyridin-3-yl | 2-Propaniminoxy | O | |
| 8.156 | 2-Chlor-1-oxopyridin-3-yl | 2-Propaniminoxy | S | |

| Nr. | Py | $R^1$ | Y | phys. Dat. |
|---|---|---|---|---|
| 8.157 | 4-Chlor-1-oxopyridin-3-yl | 2-Propaniminoxy | O | |
| 8.158 | 4-Chlor-1-oxopyridin-3-yl | 2-Propaniminoxy | S | |
| 8.159 | 5-Chlor-1-oxopyridin-3-yl | 2-Propaniminoxy | O | |
| 8.160 | 5-Chlor-1-oxopyridin-3-yl | 2-Propaniminoxy | S | |
| 8.161 | 6-Chlor-1-oxopyridin-3-yl | 2-Propaniminoxy | O | |
| 8.162 | 6-Chlor-1-oxopyridin-3-yl | 2-Propaniminoxy | S | |
| 8.163 | 2-Chlor-1-oxopyridin-4-yl | 2-Propaniminoxy | O | |
| 8.164 | 2-Chlor-1-oxopyridin-4-yl | 2-Propaniminoxy | S | |
| 8.165 | 3-Chlor-1-oxopyridin-4-yl | 2-Propaniminoxy | O | |
| 8.166 | 3-Chlor-1-oxopyridin-4-yl | 2-Propaniminoxy | S | |
| 8.167 | 3-Methyl-1-oxopyridin-2-yl | 2-Propaniminoxy | O | |
| 8.168 | 3-Methyl-1-oxopyridin-2-yl | 2-Propaniminoxy | S | |
| 8.169 | 4-Methyl-1-oxopyridin-2-yl | 2-Propaniminoxy | O | |
| 8.170 | 4-Methyl-1-oxopyridin-2-yl | 2-Propaniminoxy | S | |
| 8.171 | 5-Methyl-1-oxopyridin-2-yl | 2-Propaniminoxy | O | |
| 8.172 | 5-Methyl-1-oxopyridin-2-yl | 2-Propaniminoxy | S | |
| 8.173 | 6-Methyl-1-oxopyridin-2-yl | 2-Propaniminoxy | O | |
| 8.174 | 6-Methyl-1-oxopyridin-2-yl | 2-Propaniminoxy | S | |
| 8.175 | 2-Methyl-1-oxopyridin-3-yl | 2-Propaniminoxy | O | |
| 8.176 | 2-Methyl-1-oxopyridin-3-yl | 2-Propaniminoxy | S | |
| 8.177 | 4-Methyl-1-oxopyridin-3-yl | 2-Propaniminoxy | O | |

| Nr. | Py | $R^1$ | Y | phys. Dat. |
|---|---|---|---|---|
| 8.178 | 4-Methyl-1-oxopyridin-3-yl | 2-Propaniminoxy | S | |
| 8.179 | 5-Methyl-1-oxopyridin-3-yl | 2-Propaniminoxy | O | |
| 8.180 | 5-Methyl-1-oxopyridin-3-yl | 2-Propaniminoxy | S | |
| 8.181 | 6-Methyl-1-oxopyridin-3-yl | 2-Propaniminoxy | O | |
| 8.182 | 6-Methyl-1-oxopyridin-3-yl | 2-Propaniminoxy | S | |
| 8.183 | 2-Methyl-1-oxopyridin-4-yl | 2-Propaniminoxy | O | |
| 8.184 | 2-Methyl-1-oxopyridin-4-yl | 2-Propaniminoxy | S | |
| 8.185 | 3-Methyl-1-oxopyridin-4-yl | 2-Propaniminoxy | O | |
| 8.186 | 3-Methyl-1-oxopyridin-4-yl | 2-Propaniminoxy | S | |
| 8.187 | 3-Ethyl-1-oxopyridin-2-yl | 2-Propaniminoxy | O | |
| 8.188 | 3-Ethyl-1-oxopyridin-2-yl | 2-Propaniminoxy | S | |
| 8.189 | 4-Ethyl-1-oxopyridin-2-yl | 2-Propaniminoxy | O | |
| 8.190 | 4-Ethyl-1-oxopyridin-2-yl | 2-Propaniminoxy | S | |
| 8.191 | 5-Ethyl-1-oxopyridin-2-yl | 2-Propaniminoxy | O | |
| 8.192 | 5-Ethyl-1-oxopyridin-2-yl | 2-Propaniminoxy | S | |
| 8.193 | 6-Ethyl-1-oxopyridin-2-yl | 2-Propaniminoxy | O | |
| 8.194 | 6-Ethyl-1-oxopyridin-2-yl | 2-Propaniminoxy | S | |
| 8.195 | 2-Ethyl-1-oxopyridin-3-yl | 2-Propaniminoxy | O | |
| 8.196 | 2-Ethyl-1-oxopyridin-3-yl | 2-Propaniminoxy | S | |
| 8.197 | 4-Ethyl-1-oxopyridin-3-yl | 2-Propaniminoxy | O | |
| 8.198 | 4-Ethyl-1-oxopyridin-3-yl | 2-Propaniminoxy | S | |

| Nr. | Py | $R^1$ | Y | phys. Dat. |
|---|---|---|---|---|
| 8.199 | 5-Ethyl-1-oxopyridin-3-yl | 2-Propaniminoxy | O | |
| 8.200 | 5-Ethyl-1-oxopyridin-3-yl | 2-Propaniminoxy | S | |
| 8.201 | 6-Ethyl-1-oxopyridin-3-yl | 2-Propaniminoxy | O | |
| 8.202 | 6-Ethyl-1-oxopyridin-3-yl | 2-Propaniminoxy | S | |
| 8.203 | 2-Ethyl-1-oxopyridin-4-yl | 2-Propaniminoxy | O | |
| 8.204 | 2-Ethyl-1-oxopyridin-4-yl | 2-Propaniminoxy | S | |
| 8.205 | 3-Ethyl-1-oxopyridin-4-yl | 2-Propaniminoxy | O | |
| 8.206 | 3-Ethyl-1-oxopyridin-4-yl | 2-Propaniminoxy | S | |
| 8.207 | 3-(2-Methoxyethoxy)-1-oxopyridin-2-yl | 2-Propaniminoxy | O | |
| 8.208 | 3-(2-Methoxyethoxy)-1-oxopyridin-2-yl | 2-Propaniminoxy | S | |
| 8.209 | 4-(2-Methoxyethoxy)-1-oxopyridin-2-yl | 2-Propaniminoxy | O | |
| 8.210 | 4-(2-Methoxyethoxy)-1-oxopyridin-2-yl | 2-Propaniminoxy | S | |
| 8.211 | 5-(2-Methoxyethoxy)-1-oxopyridin-2-yl | 2-Propaniminoxy | O | |
| 8.212 | 5-(2-Methoxyethoxy)-1-oxopyridin-2-yl | 2-Propaniminoxy | S | |
| 8.213 | 6-(2-Methoxyethoxy)-1-oxopyridin-2-yl | 2-Propaniminoxy | O | |
| 8.214 | 6-(2-Methoxyethoxy)-1-oxopyridin-2-yl | 2-Propaniminoxy | S | |
| 8.215 | 2-(2-Methoxyethoxy)-1-oxopyridin-3-yl | 2-Propaniminoxy | O | |
| 8.216 | 2-(2-Methoxyethoxy)-1-oxopyridin-3-yl | 2-Propaniminoxy | S | |
| 8.217 | 4-(2-Methoxyethoxy)-1-oxopyridin-3-yl | 2-Propaniminoxy | O | |
| 8.218 | 4-(2-Methoxyethoxy)-1-oxopyridin-3-yl | 2-Propaniminoxy | S | |
| 8.219 | 5-(2-Methoxyethoxy)-1-oxopyridin-3-yl | 2-Propaniminoxy | O | |

| Nr. | Py | $R^1$ | Y | phys. Dat. |
|---|---|---|---|---|
| 8.220 | 5-(2-Methoxyethoxy)-1-oxopyridin-3-yl | 2-Propaniminoxy | S | |
| 8.221 | 6-(2-Methoxyethoxy)-1-oxopyridin-3-yl | 2-Propaniminoxy | O | |
| 8.222 | 6-(2-Methoxyethoxy)-1-oxopyridin-3-yl | 2-Propaniminoxy | S | |
| 8.223 | 2-(2-Methoxyethoxy)-1-oxopyridin-4-yl | 2-Propaniminoxy | O | |
| 8.224 | 2-(2-Methoxyethoxy)-1-oxopyridin-4-yl | 2-Propaniminoxy | S | |
| 8.225 | 3-(2-Methoxyethoxy)-1-oxopyridin-4-yl | 2-Propaniminoxy | O | |
| 8.226 | 3-(2-Methoxyethoxy)-1-oxopyridin-4-yl | 2-Propaniminoxy | S | |
| 8.227 | 3,5-Dimethyl-1-oxopyridin-2-yl | 2-Propaniminoxy | O | |
| 8.228 | 3,5-Dimethyl-1-oxopyridin-2-yl | 2-Propaniminoxy | S | |
| 8.229 | 6-Methoxy-4-methyl-1-oxopyridin-2-yl | 2-Propaniminoxy | O | |
| 8.230 | 6-Methoxy-4-methyl-1-oxopyridin-2-yl | 2-Propaniminoxy | S | |
| 8.231 | 5-(2,2,2-Trifluorethoxy)-1-oxopyridin-2-yl | 2-Propaniminoxy | O | |
| 8.232 | 5-(2,2,2-Trifluorethoxy)-1-oxopyridin-2-yl | 2-Propaniminoxy | S | |
| 8.233 | 6-Dimethylamino-1-oxopyridin-2-yl | 2-Propaniminoxy | O | |
| 8.234 | 6-Dimethylamino-1-oxopyridin-2-yl | 2-Propaniminoxy | S | |
| 8.235 | 2-Methoxy-5-trifluormethyl-1-oxopyridin-3-yl | 2-Propaniminoxy | O | |
| 8.236 | 2-Methoxy-5-trifluormethyl-1-oxopyridin-3-yl | 2-Propaniminoxy | S | |
| 8.237 | 6-Dimethylaminoxy-1-oxopyridin-2-yl | 2-Propaniminoxy | O | |
| 8.238 | 6-Dimethylaminoxy-1-oxopyridin-2-yl | 2-Propaniminoxy | S | |
| 8.239 | 6-Isopropyl-1-oxopyridin-2-yl | 2-Propaniminoxy | O | |
| 8.240 | 6-Isopropyl-1-oxopyridin-4-yl | 2-Propaniminoxy | S | |

| Nr. | Py | $R^1$ | Y | phys. Dat. |
|---|---|---|---|---|
| 8.241 | 1-Oxopyridin-2-yl | Benzyloxy | O | |
| 8.242 | 1-Oxopyridin-2-yl | Benzyloxy | S | |
| 8.243 | 1-Oxopyridin-3-yl | Benzyloxy | O | |
| 8.244 | 1-Oxopyridin-3-yl | Benzyloxy | S | |
| 8.245 | 1-Oxopyridin-4-yl | Benzyloxy | O | |
| 8.246 | 1-Oxopyridin-4-yl | Benzyloxy | S | |
| 8.247 | 1-Oxopyridin-2-yl | Methoxy | O | |
| 8.248 | 1-Oxopyridin-2-yl | Methoxy | S | |
| 8.249 | 1-Oxopyridin-3-yl | Methoxy | O | |
| 8.259 | 1-Oxopyridin-3-yl | Methoxy | S | |
| 8.260 | 1-Oxopyridin-4-yl | Methoxy | O | |
| 8.261 | 1-Oxopyridin-4-yl | Methoxy | S | |
| 8.262 | 1-Oxopyridin-2-yl | Hydroxy | O | |
| 8.263 | 1-Oxopyridin-2-yl | Hydroxy | S | |
| 8.264 | 1-Oxopyridin-3-yl | Hydroxy | O | |
| 8.265 | 1-Oxopyridin-3-yl | Hydroxy | S | |
| 8.266 | 1-Oxopyridin-4-yl | Hydroxy | O | |
| 8.267 | 1-Oxopyridin-4-yl | Hydroxy | S | |
| 8.268 | 1-Oxopyridin-2-yl | Propargyloxy | O | |
| 8.269 | 1-Oxopyridin-2-yl | Propargyloxy | S | |
| 8.270 | 1-Oxopyridin-3-yl | Propargyloxy | O | |

| Nr. | Py | $R^1$ | Y | phys. Dat. |
|---|---|---|---|---|
| 8.271 | 1-Oxopyridin-3-yl | Propargyloxy | S | |
| 8.272 | 1-Oxopyridin-4-yl | Propargyloxy | O | |
| 8.273 | 1-Oxopyridin-4-yl | Propargyloxy | S | |
| 8.274 | 1-Oxopyridin-2-yl | $OCH_2CF_3$ | O | |
| 8.275 | 1-Oxopyridin-2-yl | $OCH_2CF_3$ | S | |
| 8.276 | 1-Oxopyridin-3-yl | $OCH_2CF_3$ | O | |
| 8.277 | 1-Oxopyridin-3-yl | $OCH_2CF_3$ | S | |
| 8.278 | 1-Oxopyridin-4-yl | $OCH_2CF_3$ | O | |
| 8.279 | 1-Oxopyridin-4-yl | $OCH_2CF_3$ | S | |

Anwendungsbeispiele

Die herbizide und wachstumsregulatorische Wirkung der Verbindungen der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die erfindungsgemäßen Verbindungen Nr. 2.262 und 1.156 zeigten eine gute herbizide Wirkung.

## Patentansprüche

**1.** Pyridin-N-oxid-substituierte Salicylaldehyd- und Salicylsäurederivate der allgemeinen Formel I,

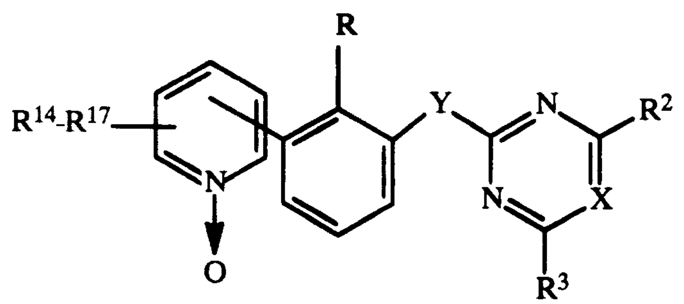

I

in der R eine Formylgruppe, eine Gruppe $CO_2H$ oder einen zu $CO_2H$ hydrolysierbaren Rest bedeutet und die übrigen Substituenten folgende Bedeutung haben:

$R^2$ Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;

X Stickstoff oder $CR^{13}$, wobei $R^{13}$ Wasserstoff oder Halogen bedeutet oder zusammen mit $R^3$ eine 3- bis 4-gliedrige Alkylen- oder Alkenylenkette bildet, in der jeweils eine Methylengruppe durch Sauerstoff ersetzt ist;

$R^3$ Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, oder $R^3$ ist mit $R^{13}$ wie oben angegeben zu einem 5- oder 6-gliedrigen Ring verknüpft;

Y Sauerstoff oder Schwefel;

$R^{14}$-$R^{17}$

a) eine $C_3$-$C_8$-Cycloalkylgruppe, welche ein bis drei $C_1$-$C_4$-Alkylreste tragen kann;

b) eine $C_1$-$C_8$-Alkylgruppe, eine $C_1$-$C_8$-Alkoxygruppe, eine $C_1$-$C_4$-Alkylthiogruppe, wobei die genannten Gruppen jeweils ein bis fünf Halogenatome und/oder einen der folgenden Reste tragen können:

$C_1$-$C_4$-Alkoxy, $C_1$-$C_4$-Halogenalkoxy, $C_1$-$C_4$-Alkylthio, Cyano, $C_3$-$C_8$-Cycloalkyl oder Di-$C_1$-$C_4$-Alkylamino;

c) eine Di-$C_1$-$C_4$-Alkylamino- oder eine Di-$C_1$-$C_4$-Alkylaminoxygruppe;

d) eine $C_2$-$C_6$-Alkenyl- oder eine $C_2$-$C_6$-Alkinylgruppe, welche ein bis fünf Halogenatome und/oder einen der folgenden Reste tragen kann:
$C_1$-$C_4$-Alkoxy, $C_1$-$C_4$-Halogenalkoxy oder $C_1$-$C_4$-Alkylthio;
e) Wasserstoff, Halogen, Nitro oder Cyano.

**2.** Pyridin-N-oxid-substituierte Salicylaldehyd- und Salicylsäurederivate der Formel I gemäß Anspruch 1, in der R für eine Gruppe

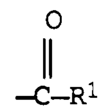

steht, wobei $R^1$ die folgende Bedeutung hat:

a) Wasserstoff;
b) eine Succinylimidoxygruppe;
c) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
$C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
d) ein Rest $-(O)_m-NR^6R^7$, in dem m für O oder 1 steht und $R^6$ und $R^7$, die gleich oder unterschiedlich sein können, die folgende Bedeutung haben:
Wasserstoff;
$C_1$-$C_8$-Alkyl, $C_3$-$C_6$-Alkenyl, $C_3$-$C_6$-Alkinyl, $C_3$-$C_8$-Cycloalkyl, wobei diese Reste jeweils ein bis fünf Halogenatome und/oder ein bis zwei der folgenden Gruppen tragen können: $C_1$-$C_4$-Alkyl, $C_1$-$C_4$-Alkoxy, $C_3$-$C_6$-Alkenyloxy, $C_3$-$C_6$-Alkinyloxy, $C_1$-$C_4$-Alkylthio, $C_3$-$C_6$-Alkenylthio, $C_3$-$C_6$-Alkinylthio, $C_1$-$C_4$-Halogenalkoxy, $C_1$-$C_4$-Alkylcarbonyl, $C_3$-$C_6$-Alkenylcarbonyl, $C_3$-$C_6$-Alkinylcarbonyl, $C_1$-$C_4$-Alkoxycarbonyl, $C_3$-$C_6$-Alkenyloxycarbonyl, $C_3$-$C_6$-Alkinyloxycarbonyl, Di-($C_1$-$C_4$-alkyl)amino, $C_3$-$C_8$-Cycloalkyl, Phenyl, ein oder mehrfach durch Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio substituiertes Phenyl;
Phenyl, das durch einen oder mehrere der folgenden Reste substituiert sein kann: Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;
$R^6$ und $R^7$ gemeinsam eine zu einem Ring geschlossene, optionell substituierte $C_4$-$C_7$-Alkylenkette oder gemeinsam eine zu einem Ring geschlossene, optionell substituierte $C_3$-$C_6$-Alkylenkette mit einem Heteroatom, ausgewählt aus der Gruppe Sauerstoff, Schwefel oder Stickstoff;
e) $R^1$ ferner eine Gruppe

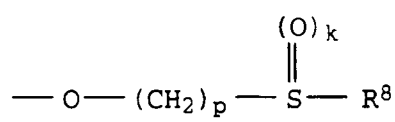

in der $R^8$ für $C_1$-$C_4$-Alkyl, Phenyl, ein- oder mehrfach durch Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio substituiertes Phenyl, $C_1$-$C_4$-Halogenalkyl, $C_3$-$C_6$-Alkenyl oder $C_3$-$C_6$-Alkinyl steht, p die Werte 1, 2, 3 oder 4 und k die Werte 0, 1 oder 2 annehmen;
f) einen Rest $OR^9$, worin $R^9$ bedeutet:
i) Wasserstoff, ein Alkalimetallkation, das Äquivalent eines Erdalkalimetallkations, das Ammoniumkation oder ein organisches Ammoniumion;
ii) eine $C_3$-$C_8$-Cycloalkylgruppe, welche ein bis drei $C_1$-$C_4$-Alkylreste tragen kann;
iii) eine $C_1$-$C_8$-Alkylgruppe, welche ein bis fünf Halogenatome und/oder einen der folgenden Reste tragen kann:
$C_1$-$C_4$-Alkoxy, $C_1$-$C_4$-Alkylthio, Cyano, $C_1$-$C_4$-Alkylcarbonyl, $C_3$-$C_8$-Cycloalkyl, $C_1$-$C_4$-Alkoxycarbonyl, Phenyl, ein- oder mehrfach durch Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiertes Phenyl oder Phenoxy;
iv) eine $C_1$-$C_8$-Alkylgruppe, welche ein bis fünf Halogenatome tragen kann und einen der folgenden Reste trägt: ein 5-gliedriger Heteroaromat, enthaltend ein bis drei Stickstoffatome, oder

ein 5-gliedriger Heteroaromat, enthaltend ein Stickstoffatom und ein Sauerstoff- oder Schwefelatom, welche ein bis vier Halogenatome und/oder ein bis zwei der folgenden Reste tragen können: Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

v) eine $C_2$-$C_6$-Alkylgruppe, welche in der 2-Position einen der folgenden Reste trägt: $C_1$-$C_4$-Alkoxyimino, $C_3$-$C_6$-Alkenyloxyimino, $C_3$-$C_6$-Halogenalkenyloxyimino oder Benzyloxyimino;

vi) eine $C_3$-$C_6$-Alkenyl- oder eine $C_3$-$C_6$-Alkinylgruppe, wobei diese Gruppen ihrerseits ein bis fünf Halogenatome tragen können;

vii) ein Phenylrest, welcher ein bis fünf Halogenatome und/oder ein bis drei der folgenden Reste tragen kann: Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

viii) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

ix) $R^9$ ferner eine Gruppe $-N=C-R^{10}R^{11}$, worin $R^{10}$ und $R^{11}$, die gleich oder verschieden sein können, bedeuten:

$C_1$-$C_{12}$-Alkyl, $C_3$-$C_6$-Alkenyl, $C_3$-$C_6$-Alkinyl, $C_3$-$C_8$-Cycloalkyl, wobei diese Reste einen $C_1$-$C_4$-Alkoxy, $C_1$-$C_4$-Alkylthio- und/oder einen Phenylrest tragen können;

Phenyl, das durch einen oder mehrere der folgenden Reste substituiert sein kann:

Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;

oder $R^{10}$ und $R^{11}$ bilden gemeinsam eine $C_3$-$C_{12}$-Alkylenkette, welche ein bis drei $C_1$-$C_4$-Alkylgruppen tragen kann;

g) oder $R^1$ bildet einen Rest $-NH-SO_2-R^{12}$, in dem $R^{12}$ bedeutet:

$C_1$-$C_4$-Alkyl, $C_3$-$C_6$-Alkenyl, $C_3$-$C_6$-Alkinyl, $C_3$-$C_8$-Cycloalkyl, wobei diese Reste einen $C_1$-$C_4$-Alkoxy-, $C_1$-$C_4$-Alkylthio- und/oder einen Phenylrest tragen können;

Phenyl, das durch einen oder mehrere der folgenden Reste substituiert sein kann: Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio.

**3.** Pyridin-N-oxid-substituierte Salicylaldehyd- und Salicylsäurederivate der Formel I gemäß Anspruch 1, in der mindestens zwei der Reste $R^{15}$ bis $R^{17}$ Wasserstoff bedeuten.

**4.** Pyridin-N-oxid-substituierte Salicylaldehyd- und Salicylsäurederivate der Formel I gemäß Anspruch 1, in der $R^3$ für Methoxy und X für CH, CF stehen oder $R^3$ mit X zu einer $-OCH_2CH_2-$ Kette verknüpft sind.

**5.** Pyridin-N-oxid-substituierte Salicylaldehyd- und Salicylsäurederivate der Formel I gemäß Anspruch 1, in der zwei der Reste $R^{14}$ bis $R^{17}$ Wasserstoff und der verbleibende Rest eine $C_1$-$C_4$-Alkylgruppe darstellt.

**6.** Zwischenprodukte der Formel IV

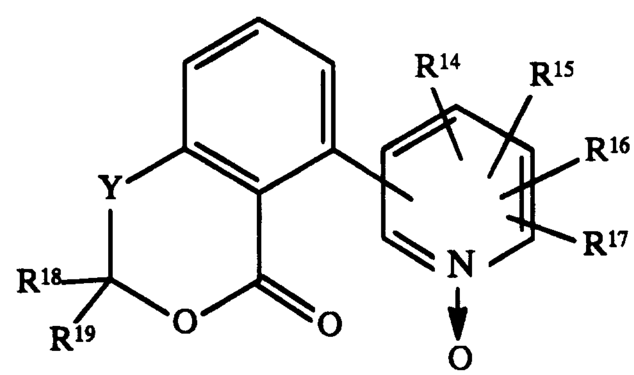

IV

in der die Reste folgende Bedeutungen haben:

$R^{18}$, $R^{19}$ Wasserstoff;

$C_1$-$C_4$-Alkyl, wobei dieser Rest jeweils ein bis fünf Halogenatome und/oder ein bis zwei $C_1$-$C_4$-Alkoxy-Gruppen tragen kann;
Phenyl, wobei dieser Rest jeweils ein bis fünf Halogenatome und/oder ein bis zwei der folgenden Gruppen tragen kann: $C_1$-$C_4$-Alkyl, $C_1$-$C_4$-Alkoxy, $C_1$-$C_4$-Halogenalkyl oder Nitro;
ferner die beiden Reste gemeinsam eine $C_2$-$C_6$-Alkylenkette, die durch ein bis fünf Halogenatome und/oder $C_1$-$C_4$-Alkylreste substituiert sein kann;

Y ein Sauerstoff- oder Schwefelatom;

$R^{14}$-$R^{17}$ die unter Anspruch 1 genannten Gruppen.

**7.** Zwischenprodukte der Formel III

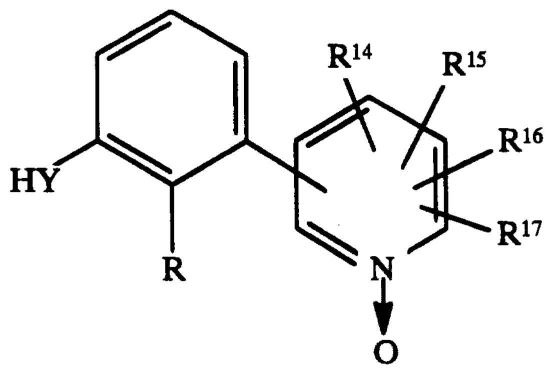

III

in der die Reste die in Anspruch 1 angegebenen Bedeutungen haben.

**8.** Zwischenprodukte der Formel III nach Anspruch 7, bei denen R für $COR^1$ steht und $R^1$ die unter Anspruch 2 angegebenen Bedeutungen hat.

**9.** Verfahren zur Herstellung von Pflanzenschutzwirkstoffen der Formel I'

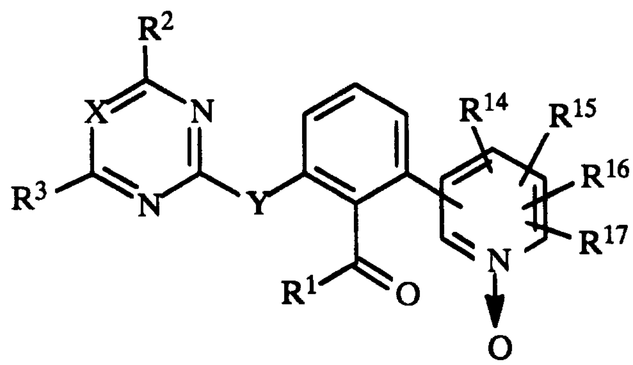

I'

in der $R^2$, $R^3$, $R^{14}$-$R^{17}$, X und Y die unter Anspruch 1 angegebenen Bedeutungen haben und $R^1$ die unter Anspruch 2 genannten Bedeutungen hat mit Ausnahme von Wasserstoff, der Succinylimidoxygruppe sowie von denjenigen Resten $OR^9$, bei denen $R^9$ für Wasserstoff, ein Alkalimetallkation, das Äquivalent eines Erdalkalimetallkations, das Ammoniumkation oder ein organisches Ammoniumion steht, dadurch gekennzeichnet, daß man Verbindungen der Formel IV, gemäß Anspruch 6

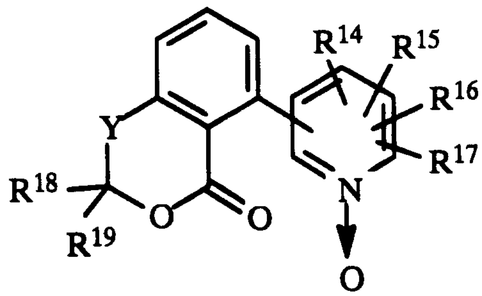

IV

zunächst mit einem Salz $R^1$-M, wobei M für ein Alkalimetallkation oder ein Äquivalent eines Erdalkalimetallkations steht, und dann mit einem Heterocyclus der Formel V,

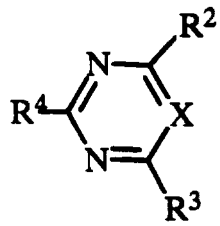

V

in der $R^4$ für Halogen, Alkylsulfonyl, Halogenalkylsulfonyl oder eine äquivalente Abgangsgruppe steht, in einem inerten Lösungsmittel umsetzt.

**10.** Verfahren zur Herstellung von Pyridin-N-oxid-substituierten Salicylaldehyd- und Salicylsäurederivaten gemäß Anspruch 1, dadurch gekennzeichnet, daß man entsprechende aromatische ortho-Mercapto- oder ortho-Hydroxycarbonsäurederivate der Formel III

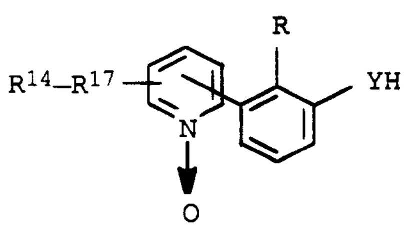

III

mit einem Heterocyclus der Formel II,

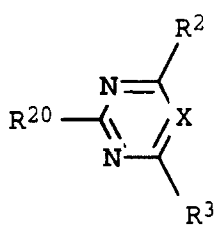

II

worin $R^{20}$ für Halogen, Alkylsulfonyloxy, Arylsulfonyloxy oder eine andere äquivalente Abgangsgruppe steht, in Gegenwart einer anorganischen oder organischen Base umsetzt.

**11.** Verfahren zur Herstellung von Pyridin-N-oxid substituierten salicylaldehyd- bzw. -säurederivaten gemäß Anspruch 1, dadurch gekennzeichnet, daß man entsprechende Verbindungen der Formel I', wobei $R^1$ für den Hydroxylrest oder ein Salz des Hydroxylrestes steht, zunächst in das Halogenid oder eine andere aktivierte Form der Carbonsäure überführt und dieses dann gegebenenfalls in Gegenwart einer anorganischen oder organischen Base mit einem gegebenenfalls substituierten Alkohol, einem Azol,

einem Oxim oder N-Hydroxysuccinylimin umsetzt.

**12.** Herbizides Mittel sowie Mittel zur Beeinflussung des Pflanzenwachstums, gekennzeichnet durch einen Gehalt an mindestens einem Pyridin-N-oxid substituierten Salicylaldehyd- bzw. -säurederivat der Formel I, gemäß Anspruch 1 und üblichen inerten Trägerstoffen.

**13.** Verfahren zur Bekämpfung unerwünschten Pflanzenwuchses sowie zur Beeinflussung des Pflanzenwachstums, dadurch gekennzeichnet, daß man eine herbizid wirksame Menge eines Pyridin-N-oxid substituierten Salicylaldehyd- bzw. Säurederivates der Formel I gemäß Anspruch 1 auf die Pflanzen und/oder deren Lebensraum einwirken läßt.

**14.** Verfahren zur Herstellung von Pyridin-N-oxid-substituierten Verbindungen gemäß Anspruch 1, dadurch gekennzeichnet, daß man entsprechende Pyridin-substituierte Verbindungen oxidiert.

Europäisches Patentamt

# EUROPÄISCHER RECHERCHENBERICHT

Nummer der Anmeldung
EP 94 11 6757

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X,D | EP-A-0 402 751 (BASF AG) 19. Dezember 1990<br>--- | 1-14 | C07D401/12<br>A01N43/54<br>C07D407/14<br>A01N43/66<br>C07D405/04<br>//(C07D401/12, 239:00, 213:00), (C07D401/12, 215:00,213:00) |
| X,D | WO-A-91 13065 (FMC CORPORATION) 5. September 1991<br>* siehe insbesondere Tabelle 1B auf Seite 69 *<br>--- | 1-14 | |
| X | US-A-5 149 357 (FMC CORPORATION) 22. September 1992<br>--- | 1-14 | |
| Y | CHEMICAL ABSTRACTS, vol. 119, no. 15, 11. Oktober 1993, Columbus, Ohio, US; abstract no. 160312g, WADA,N. ET AL. 'Preparation of (carboxyphenoxy)pyrimidines as herbicides' Seite 939 ;<br>* Zusammenfassung * | 1-14 | |
| Y | & JP-A-05 032 639 (...)<br>--- | 1-14 | |
| Y | EP-A-0 435 104 (BASF AG) 3. Juli 1991<br>--- | 1-14 | **RECHERCHIERTE SACHGEBIETE (Int.Cl.6)** |
| Y | EP-A-0 527 380 (BASF AG) 17. Februar 1993<br>--- | 1-14 | C07D<br>A01N |
| Y | EP-A-0 061 913 (E.I. DU PONT DE NEMOURS AND COMPANY) 6. Oktober 1982<br>--- | 1-14 | |
| Y | EP-A-0 477 637 (BAYER AG)<br>--- | 1-14 | |
| X,P | WO-A-94 22310 (BASF AG) 13. Oktober 1994<br>----- | 1-14 | |

Der vorliegende Recherchenbericht wurde für alle Patentansprüche erstellt

| Recherchenort | Abschlußdatum der Recherche | Prüfer |
|---|---|---|
| MÜNCHEN | 6. März 1995 | Stellmach, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)